# EUROPEAN PATENT SPECIFICATION

(11) **EP 2 006 391 B1**
(45) Date of publication and mention of the grant of the patent: **22.11.2017**
(21) Application number: 07740960.5
(22) Date of filing: 28.03.2007
(51) Int. Cl.: C12P 13/08, C12N 15/09, C12R 1/01, C12R 1/025, C12R 1/07, C12R 1/38

(54) **METHOD FOR PRODUCTION OF CARBOXYLIC ACID USING METHANOL-UTILIZING BACTERIUM**
VERFAHREN ZUR HERSTELLUNG EINER CARBONSÄURE UNTER VERWENDUNG EINES METHANOL NUTZENDEN BAKTERIUMS
PROCEDE DE PRODUCTION D'UN ACIDE CARBOXYLIQUE UTILISANT UNE BACTERIE AVEC DU METHANOL

(30) Priority: 30.03.2006 JP 2006095207
(43) Date of publication of application: 24.12.2008
(73) Proprietor: Ajinomoto Co., Inc., Tokyo 104-8315 (JP)
(72) Inventor: MURAKOSHI, Yuriko c/o AJINOMOTO CO., INC., Kawasaki-shi, Kanagawa 210-8681 (JP); ISHIKAWA, Kohei c/o AJINOMOTO CO., INC., Kawasaki-shi, Kanagawa 210-8681 (JP); KONDO, Kazuya c/o AJINOMOTO CO., INC., Kawasaki-shi, Kanagawa 210-8681 (JP)
(74) Representative: Strehl Schübel-Hopf & Partner
(86) International application number: PCT/JP2007/057524
(87) International publication number: WO 2007/114465

(56) References cited:
- WO-A1-00/61723
- WO-A2-90/12105
- JP-A- 10 215 883
- JP-A- 2004 166 594
- US-A1- 2004 146 974
- GROTHE E ET AL: "Fermentation optimization for the production of poly([beta]-hydroxybutyric acid) microbial thermoplastic", ENZYME AND MICROBIAL TECHNOLOGY 19990715 US LNKD- DOI:10.1016/S0141-0229(99)00023-X, vol. 25, no. 1-2, 15 July 1999 (1999-07-15), pages 132-141, XP002661967, ISSN: 0141-0229
- WEUSTER-BOTZ D ET AL: "Substrate controlled fed-batch production of L-lysine with Corynebacterium glutamicum", BIOTECHNOLOGY PROGRESS, vol. 13, no. 4, 1997, pages 387-393, XP002661968, ISSN: 8756-7938
- BAEV M V ET AL: "Regulation of ammonia assimilation in an obligate methylotroph Methylobacillus flagellatum under steady-state and transient growth conditions", ANTONIE VAN LEEUWENHOEK, INTERNATIONAL JOURNAL OF GENERAL AND MOLECULAR MICROBIOLOGY 1997 NL LNKD- DOI:10.1023/A:1000293619500, vol. 71, no. 4, 1997, pages 353-361, XP002661969, ISSN: 0003-6072
- GUNJI Y ET AL: "Enhancement of l-lysine production in methylotroph Methylophilus methylotrophus by introducing a mutant LysE exporter", JOURNAL OF BIOTECHNOLOGY, ELSEVIER SCIENCE PUBLISHERS, AMSTERDAM, NL, vol. 127, no. 1, 15 December 2006 (2006-12-15), pages 1-13, XP024956556, ISSN: 0168-1656, DOI: 10.1016/J.JBIOTEC.2006.06.003 [retrieved on 2006-12-15]
- WENDLANDT K.-D. ET AL.: 'Einfluss ausgewählter Nährsalzionen auf die Wachstumskinetik des methanotrophen Bakteriums GB 25' ACTA BIOTECHNOL. vol. 7, no. 6, 1987, pages 521 - 527, XP003017180
- MOLLER T. ET AL.: 'Ammonium Toxicity in Bacteria' CURRENT MICROBIOLOGY vol. 52, no. 5, 2006, pages 400 - 406, XP019365732
- TAKAHIRO SUZUKI ET AL: "Mass production of poly-Î-hydroxybutyric acid by fully automatic fed-batch culture of methylotroph", APPLIED MICROBIOLOGY AND BIOTECHNOLOGY,, vol. 23, 1 January 1986 (1986-01-01), pages 322-329, XP001320309,
- IZUMI Y ET AL: "L-serine production by a methylotroph and its related enzymes", APPLIED MICROBIOLOGY AND BIOTECHNOLOGY 1993 DE, vol. 39, no. 4-5, 1993, pages 427-432, ISSN: 0175-7598
- CHAN T ET AL: "The interaction of methanol dehydrogenase and cytCytochrome CL in the acidophilic methylotroph Acetobacter methanolicus", Biochem. J, 1 January 1991 (1991-01-01), pages 139-146, XP055103737, Retrieved from the Internet: URL:http://www.biochemj.org/bj/280/0139/28 00139.pdf [retrieved on 2014-02-20]

## Description

### Technical Field

The present invention relates to the microbiology industries, and more particularly, the present invention relates to a method of producing L-amino acid by fermentation ,and microorganism used for the production method

### Background Art

L-amino acids such as L-lysine, L-glutamic acid, L-threonine, L-leucine, L-isoleucine, L-valine, and L-phenylalanine are industrially produced by fermentation using microorganism belonging to the genus *Brevibacterium, Corynebacterium, Bacillus, Escherichia, Streptomyces, Pseudomonas, Arthrobacter, Serratia, Penicillium, Candida,* and the like. To enhance productivity, these microorganisms are employed in the form of strains that have been isolated from nature and artificial variants thereof. Moreover, various technologies have been disclosed for increasing the L-amino acid producing abilities, such as a recombinant DNA technology to enhance L-amino acid productivity.

Conventionally known methods of producing carboxylic acid by fermentation from methanol, which is an economical and commercial available fermentation raw material, include methods employing microorganisms belonging to the genus Achromobacter and genus *Pseudomonas* (Patent Document 1), genus *Protaminobacter* (Patent Document 2), genus *Protaminobacter* and genus *Methanomonas* (Patent Document 3), genus *Microcyclus* (Patent Document 4), genus Methylobacillus (Patent Document 5), and genus Bacillus (Patent Document 6).

Thus far, a method of producing L-amino acids with bacteria of the genus *Methylophilus* by increasing enzymatic activities dihydrodipicolinate synthase and/or aspartokinase by breeding based on artificial mutation or the use of recombinant DNA techniques has been developed (Patent Document 7). The amino acid export process has been found to be a major obstacle to producing amino acids by fermentation processes from methanol using methanol-assimilating bacteria. As one means of overcoming this problem, a mutant of the LysE protein -- a protein that participates in the export of L-lysine -- that exhibits L-lysine export activity in methanol-assimilating bacteria has been isolated from bacteria of the genus *Corynebacterium.* We have found that by breeding based on artificial mutation, employing recombinant DNA techniques, and employing this modified L-lysine export carrier, it is possible to cause L-lysine to be efficiently produced, and have developed a method for producing L-amino acid with bacteria of the genus Methylophilus (Patent Document 7 and 8).

Through the enhanced breeding of microorganisms such as the above, the ability to produce substances using methanol-assimilating bacteria has been greatly enhanced. However, there is need for the development of inexpensive and efficient methods of producing carboxylic acids from methanol.
- Patent Document 1:: Japanese Patent Application Publication No. Showa 45-25273
- Patent Document 2:: Japanese Patent Publication No. Showa 49-125590
- Patent Document 3:: Japanese Patent Application Publication No. Showa 50-25790
- Patent Document 4:: Japanese Patent Application Publication No. Showa 52-18886
- Patent Document 5:: Japanese Patent Application Publication No. Heisei 4-91793
- Patent Document 6:: Japanese Patent Application Publication No. Heisei 3-505284
- Patent Document 7:: International Publication No. 00/61723
- Patent Document 8:: Japanese Patent Publication No. 2004-166594

### Disclosure of the Invention

It is an object the present invention is to provide a method for producing L-amino acids with good efficiency from inexpensive and abundantly available methanol.

The present inventors conducted extensive research into solving the above-stated problem, resulting in the discovery that, in the course of conducting fermentation production with methanol-assimilating bacteria using methanol as a carbon source, a high ionic strength inhibited the proliferation of the methanol-assimilating bacteria. By conducting feeding while keeping the rate of increase in ionic strength in the culture medium at or below a certain level, the present inventors succeeded in maintaining high fermentation production capability without inhibiting the growth of the bacteria. That is, the present invention provides the following:
[1] A method for producing an L-amino acid by a fermentation process, comprising culturing a methanol-assimilating bacterium that has an ability to produce the L-amino acid in a liquid medium containing methanol and a substance comprising a counter ion and causing the L-amino acid to be produced and accumulated in the medium,
   wherein the feeding of a substance comprising methanol and a substance comprising a counter ion to the medium is carried out by fed-batch culturing to control the total ionic strength in fermentation medium,
   wherein the ionic strength is controlled to achieve a rate of increase in total ionic strength in the medium of 0.02 mol/m³/hour or less,
   wherein said substance comprising a counter ion is one or more substances selected from the group consisting of ammonium sulfate, ammonium chloride, ammonium glutamate, ammonium succinate, ammonium fumarate, and ammonium aspartate, and
   wherein said methanol-assimilating bacterium is a microorganism selected from the group consisting of bacteria of the genus Acromobacter, Pseudomonas, Protaminobacter, Methanomonas, Microcyclus, Methylobacillus, Bacillus, and Methylophilus.
[2] The method according to [1], wherein the ionic strength is controlled during the proliferation period of the methanol-assimilating bacterium.
[3] The method according to [1], wherein said L-amino acid is L-lysine.
[4] The method according to [1] wherein said methanol- assimilating bacterium has been modified to increase the activity of one or more enzymes selected from the group consisting of diaminopimelate dehydrogenase, diaminopimelate decarboxylase, and aspartate semialdehyde dehydrogenase.
[5] The method according to [4], wherein said methanol- assimilating bacterium comprises DNA encoding dihydrodipicolinate synthase and/or aspartokinase that has been modified so as not to subject to feedback inhibition by L-lysine.
[6] The method according to [4], wherein said methanol- assimilating bacterium comprises DNA encoding a mutant lysE protein promoting the export of L-lysine to the outside of the cell when introduced into a methanol-assimilating bacterium.

### Brief Description of Drawings

Fig. 1 is an illustration showing the construction of plasmid pM12.
Fig. 2 is an illustration showing the construction of plasmid pMIV5.
Fig. 3 is an illustration showing the construction of plasmid pMIV-FRTmFRT.
Fig. 4 is an illustration showing the construction of plasmid pAET7.

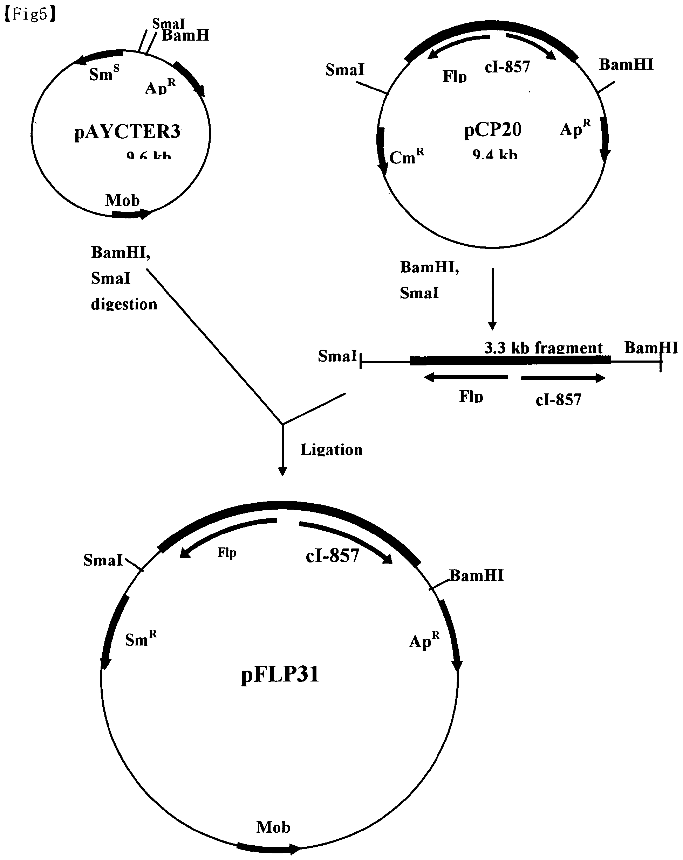

Fig. 5 is an illustration showing the construcion of plasmid pFLP31.

### Best Modes of Carrying Out the Invention

### <1> The production method of the present invention

The method of the present invention is a method of producing an L-amino acid by culturing a methanol-assimilating bacterium having the ability to produce an L-amino acid in a liquid medium containing methanol and a substance comprising a counter ion and causing the carboxylic acid to be produced and accumulate in the medium, wherein methanol and a substance comprising counter ion are added to the medium by fed-batch culturing to control the total ionic strength at a predetermined level. Here, the term "total ionic strength" means the strength of all ions contained in the medium. The term "all ions contained in the medium" means the cations and anions in a medium containing carboxylic acid, carboxylic acid counter ions, other organic acids, and the like.

In the present invention, there is no particular limitation on the type of L-amino acid. Examples are: basic amino acids such as L-lysine, L-ornithine, L-arginine, L-histidine, and L-citrulline; aliphatic amino acids such as L-isoleucine, L-alanine, L-valine, L-leucine, and L-glycine; amino acids in the form of hydroxymonoaminocarboxylic acids, such as L-threonine and L-serine; cyclic amino acids such as L-proline; aromatic amino acids such as L-phenylalanine, L-tyrosine, and L-tryptophan; sulfur-containing amino acids such as L-cysteine, L-cystine, and Lmethionine; and acidic amino acids such as L-glutamic acid, L-aspartic acid, L-glutamine, and L-asparagine. Of these, the basic L-amine acids, L-lysine, L-asparagine, L-ornithine, and L-histidine are desirable.

The medium employed in the present invention contains methanol as a carbon source and is adjusted so as to contain counter ions of L-amino acid Here, the substance serving as the source of the counter ions is desirably a substance containing ammonium ions as a nitrogen source.

The substance serving as the source of the counter ions is a substance capable of maintaining a low total ionic strength in the medium. The substance comprising a counter ion is one or more substances selected from the group consisting of ammonium sulfate, ammonium Chloride, ammonium glutamate, ammonium succinate, ammonium fumarate, and ammonium aspartate.

In the present invention, the substance containing methanol and a substance comprising a counter ion is desirably supplied to the medium by feeding a feed medium. However, the substance containing methanol and a substance comprising counter ions may also be contained in the initial medium. In the present invention, the term "initial medium" means the medium employed in batch culturing prior to feeding, or the fermentation medium that is added by feeding. The term "feed medium" means a medium that is fed into a fermentation vessel during the course of fed-batch culturing. In the present invention, the term "fermentation medium" means the medium in the fermentation vessel; the L-amino acid is collected from the fermentation medium. In the present invention, "fermentation vessel" means the apparatus in which L-amino acid fermentation is conducted; a fermentation tank or jar fermenter may be employed. The capacity thereof need only be adequate for the production and collection of the L-amino acid.

In the present invention, culturing is desirably conducted while controlling the ionic strength of the counter ions in the fermentation medium, with the rate of rise in ionic strength of the total counter ions in the medium being desirably kept at or below a predetermined level. Feeding of the substance containing the counter ions is conducted while controlling the rate of rise in ionic strength to 0.02 mol/m²/hour or less, desirably 0.015 mol/m²/hour or less, and preferably, 0.01 mol/m²/hour or less.

The ionic strength is desirably limited to or less a predetermined level over the entire course of culturing, but may be so limited only during certain steps. For example, when the method of the present invention comprises a period of proliferating the microorganism having the ability to produce a carboxylic acid (proliferation period) and a period of producing L-amino acid (production period), the ionic concentration is desirably limited to or less a predetermined concentration during the proliferation period. The ionic strength need not be kept within the above-stated range at all times during culturing; it is possible for the content of counter ions present at a certain period to exceed the above-stated range, and then be reduced during culturing. A substance containing counter ions can also be intermittently added when counter ion run short in fermentation. In the present invention, the term "proliferation period" means a period beginning at the start of culturing during which the carbon source is primarily used for bacterial growth that lasts 10 hours, desirably 18 hours, and preferably 24 hours; that is, the period during which the microorganism undergoes logarithmic growth. In the present invention, the "production period" means a period beginning 24 hours after the start of culturing that is used primarily used for the production of carboxylic acid.

It is sufficient that the feed medium contain a minimum of counter ions of carboxylic acid, and however,the amount of sulfur may temporarily run short. The term "temporarily," for example, means the counter ions may run short for a period corresponding to about 20 percent, 40 percent, or a maximum of 60 percent of the total duration of fermentation. During the period when the counter ions runs short ,although the level of counter ion may temporary be 0; it is desirable present for the rate of the rise in ionic strength to be 0.001 mol/m²/hour or higher.

The rate of rise in ionic strength in the fermentation medium can be calculated by measuring the quantity of sulfate ions, chloride ion, carbonate ion, and carboxylic acid. For example, the sulfate ions ions and chloride ion can be measured by ion chromatography, and the carbonate ion can be measured with a carbonic acid gas measuring apparatus.

In addition to methanol, the carbon source contained in the medium employed in the present invention may simultaneously comprise glucose, glycerol, fructose, sucrose, maltose, mannose, galactose, starch hydrolysis products, molasses, and other sugars. Additionally, acetic acid, citric acid, and other organic acids may be employed in combination with methanol. Cane molasses, beet molasses, high test molasses, and citrus molasses, as well as the hydrolysis products of natural raw materials such as cellulose, starch, corn, cereals, and tapioca may be employed as raw materials for the carbon source. Carbon dioxide dissolved into the culture liquid can also be employed as a carbon source. These carbon sources can be employed in the initial medium as well as in the feed medium. They may be mixed with methanol in both the initial medium and feed medium, or culturing may be conducted using methanol as the carbon source in the feed medium and some other carbon source initially.

Methanol is desirably fed by feeding so as to be contained in the fermentation medium in a proportion of 2 percent or less, desirably 0.5 percent or less, and preferably, 0.2 percent or less.

Substances supplying counter ions can be employed as nitrogen sources contained in the medium in the present invention. Ammonia gas and ammonia water that are employed for pH adjustment can also be used as nitrogen sources. Peptones, yeast extracts, meat extracts, wheat germ extracts, corn steep liquor, soybean hydrolysis products, and the like may also be employed. These nitrogen sources may be employed in both the initial medium and feed medium. These nitrogen sources may be blended into both the initial medium and the feed medium, or the nitrogen source employed in the feed medium may be different from that employed in the initial medium.

In addition to a carbon source, nitrogen source, and sulfur source, a phosphorus source is also desirably contained in the medium in the present invention. Phosphorus sources that are suitable for use include potassium dihydrogen phosphate, dipotassium hydrogen phosphate, pyrolinic acid, and other phosphoric acid polymers.

In addition to a carbon source, nitrogen source, and sulfur source, a growth-promoting factor may also be contained in the medium in the present invention. Growth-promoting factors which can be used include trace metal, amino acids, vitamins, fatty acids, nucleic acids, peptones containing the same, casamino acids, yeast extracts, and soy protein degradation products. Examples of trace metals include iron, manganese, magnesium, and calcium. Examples of vitamins include vitamin B1, vitamin B2, vitamin B6, nicotinic acid, nicotinamide, and vitamin B12. These growth-promoting factors may be incorporated into the initial medium, or may be incorporated into the feed medium.

Furthemore, when auxotrophic mutant that requires amino acids or the like for growth, the nutrients that are required are desirably supplemented in the medium in the present invention. The addition of L-methionine and L-threonine for the L-lysine producing bacteria that can be employed in the present invention is desirable because an L-methionine or L-threonine auxotroph and/or the deletion to degradate L-threonine are often imparted to L-Lysine producing bacteria (U.S. Patent Application Publication 2004-0214296).

Culturing is desirably conducted at a fermentation temperature of 20 to 45°C, preferably 33 to 42°C, with aeration. Here, the oxygen concentration is adjusted to 5 to 50 percent, desirably about 10 percent. The pH is controlled to 5 to 9 and culturing is conducted with aeration. When the pH is lowered during culturing, for example,calcium carbonate or an alkali such as ammonia gas and aqueous ammonia may be added to neutralize the culture. Culturing under such conditions, desirably for about 10 to 120 hours, causes a large amount of L-amino acid to accumulate in the culture solution. The concentration of the carboxylic acid that accumulates is higher than with wild strains; the concentration of L-lysine is not limited as long as L-lysine can be isolated and collected from the medium. The concentration is 10 g/L or greater, desirably 30 g/L or greater, and preferably, 50 g/L or greater.

The L-amino acid can be collected from the culture medium at the end of culturing by any known recovery method. For example, after removing the bacterial mass from the culture solution by centrifugal separation or the like, concentration precipitation can be used for collection. L-lysine can be collected by combining ordinary ion-exchange resin methods, precipitation methods, and other known methods.

Any one from among batch culture, fed-batch culture, and continuous culture can be employed as the culturing method in the present invention. To maintain the L-amino acid accumulation at or above a prescribed level in the present invention, seed culture and main culture can be conducted separately. Seed culture can be conducted as a shaking culture in a flask or the like, or as a batch culture. The main culture can be conducted as fed-batch culture or continuous culture. Both seed culture and main culturing can be conducted as batch culture.

In the course of fed-batch cultureor continuous culture in the present invention, the feeding of methanol and nutrient sources can be temporary suspended in culture. During maximum feeding, feeding is stopped 30 percent of the time or less, desirably 20 percent of the time or less, and preferably, 10 percent of the time or less. When the feed medium is intermittently added, the feed medium may be initially added over a predetermined level, and the second and following additions may be controlled so that is is started when an increase in the pH or the dissoloved oxygen concentration is detected by a computer. (U.S. Patent 5,912,113).

The present invention is a method of producing a L-amino acid by a fermentation process in which a methanol-assimilating bacterium having the ability to produce a L-amino acid is cultured in a liquid medium containing methanol and a substance comprising counter ions, the L-amino acid is produced and accumulated in the same medium, and a monovalent ion is employed as the counter ion. Here, ammonium chloride, ammonium glutamate, ammonium succinate, ammonium fumarate, and ammonium aspartate can be employed as the monovalent ion.

### <2> Methanol-assimilating bacteria that can be employed in the present invention

In the present invention, the term "methanol-assimilating bacterium" is a bacterium that is capable of growing using methanol as its main carbon source and is selected from the bacteria recited in claim 1. Specific examples include: bacteria of the genus *Methylophilus,* such as *Methylophilus methylotrophus*; bacteria of the genus *Methylobacillus,* such as *Methylobacillus glycogenes* and *Methylobacillus flagellatus*; bacteria of thegenus genera *Acromobacter* and *Pseudomonas,* (JP45-25273A); bacteria of the genus *Protaminobacter* (JP49-125590A); bacteria of the genus *Protaminobacter* and *Methanomonas* (JP50-25790A), and bacteria of the genus *Microcyclus* (JP52-18886A). The bacterium may also include of the genus *Methylobacterium.*

Examples of *Methylophilus methylotrophus* include the AS1 strain (NCIMB10515) and W3A1 (NCIMB 11348 strain). *Methylophilus methylotrophus* AS1 strain (NCIMB10515) and W3A1 (NCIMB 11348 strain) are available from the National Collections of Industrial and Marine Bacteria, NCIMB Lts., Torry Research Station 135, Abbey Road, Aberdeen AB9 8DG, United Kingdom.

Examples of *Methylobacillus glycogenes* include the T-11 strain (NCIMP 11375), ATCC 21276 strain, ATCC 21371 strain, ATR80 strain (Appl. Microbiol. Biotechnol., (1994), Vol. 42, pp. 67-72), and A513 strain (Appl. Microbiol. Biotechnol., (1994), Vol. 42, pp. 67-72). The *Methylobacillus glycogenes* NCIMB 11375 strain is available from the National Collections of Industrial and Marine Bacteria, NCIMB Lts., Torry Research Station 135, Abbey Road, Aberdeen AB9 8DG, United Kingdom.

Examples of *Methylobacillus flagellatus* are the ATTC 51484 strain, KT strain (N. 1. Govorukhina et al., Microbiology (Russia) 56 (1987), pp. 849-854), and VKM B-1610 strain. *Methylobacillus flagellatus* VKM B-1610 strain is available from the All-Russian Collection of Microorganisms (Russia, 142290, Moscow Region, Pushchino, pr. Nauki, 5, IBPM).

The *Methylobacillus glycogenes* ATCC 21276 and ATCC 21371 strains, and the *Methylobacillus flagellatus* ATTC 51484 strain may be obtained from the American Type Culture Collection (ATCC) (ATCC, Address: P.O. Box 1549, Manassas, VA 20108, 1, USA).

In the present invention, the term "capable of producing a L-amino acid" means the ability to produce and accumulate free L-amino acid in the medium, that is, outside the cell, when cultured in a medium, particularly the ability to accumulate more L-amino acid than the wild strain (parent strain). Examples of "wild strains" are the AS1 strain (NCIMB 10515) for bacteria of the genus *Methylophilus* and the T-11 strain (NCIMB 11375) for bacteria of the genus *Methylobacillus.*

To obtain the ability to produce a L-amino acid,method can be used which have conventionally adopted for breeding Escherichia bacteria, such a acquisition of an auxotrophic mutant, analog resistant strain or metabolism control mutant strain as well as construction of recombinant strain in which an activity of L-amino acid biosynthesis enzyme is enhanced. (see Amino Acid Fermentation, Gakkai Shuppan Center, 1st ed. May 30, 1986, pp. 77-100). Here, in breeding L-amino acid-producing bacteria, there may be one or more properties such as an imparted auxotrophic mutant, analog resistance, or metabolically controlled mutation. There may be one or more L-amino acid biosynthesis-related enzymes the expression of which has been increased. It is also possible to combine the imparting of a property such as a auxotrophic mutant, analog resistance, or metabolically controlled mutation with the strengthening of a L.amino acid biosynthesis-related enzyme.

Strains with auxotrophic mutant, L-amino acid analog-resistant strains, and variants with controlled metabolisms that are capable of producing L-amino acid can be obtained by subjecting a parent strain or wild strain to the usual mutagenic treatments, such as irradiation with X-rays or ultraviolet radiation or treatment with a mutagenic agent such as N-methyl-N'-nitro-N-nitrosoguanidine (NTG) or ethyl methane sulfonate (EMS), and selecting those strains that both exhibit a auxotrophic mutant, analog resistance, or metabolically controlled mutation and the ability to produce a L-amino acid from among the variants obtained.

Examples of gene recombination are the method of increasing the expression of a gene encoding an enzyme involved in the biosynthesis of the target L-amino acid and the method of reducing the activity of a gene encoding an enzyme involved in degradating the targeted L-amino acid.

Specific examples of microorganisms with the ability to produce L-amino acids will be given below.

### <2-1> Imparting the ability to produce L-lysine

Methanol-assimilating bacteria having the ability to produce L-lysine, such as Methylophilus methylotrophus strains, can be obtained by subjecting a strain with no ability to produce L-lysine or a strain with little ability to produce L-lysine to a mutagenic treatment to impart resistance to a lysine analog such as S-(2-aminoethyl)-L-cysteine (abbreviated to "AEC" hereinafter). Methods of mutagenic treatment include subjecting the bacterial strain to a physical stimulus such as UV radiation, X-ray, or γ-rays, or treating it with a chemical mutagenic agent such as NTG. *Methylophilus methylotrophus* AJ13608 is a example of a bacterial strain of the genus *Methylophilus* having the ability to produce L-lysine that was obtained in this manner.

The original bacterial strain was bred by imparting AEC-resistance to the *Methylophilus methylotrophus* AS1 strain. *Methylophilus methylotrophus* AJ13608 was deposited as repository number FERM P-17416 with the National Institute of Bioscience and Human-Technology, Agency of Industrial Science and Technology (now the International Patent Organism Depositary (Chuo No. 6, 1-banchi, 1-chome, Tsukuba-shi Higashi, Ibaraki-ken, Japan, Postal Code 305-8566) of the National Institute of Advanced Industrial Science and Technology, an Independent Administrative Institution) on June 10, 1999, and transferred to international depositary authority under the Budapest Treaty on March 31, 2000, with the designation FERM BP-7112.

Methanol-assimilating bacteria having the ability to produce L-lysine can also be bred using a gene recombination technique to introduce and increase DNA encoding genetic information contributing to the biosynthesis of L-lysine. The genes that are introduced are those encoding enzymes of the L-lysine biosynthesis pathway, such as dihydrodipicolinate synthase, diaminopimelate decarboxylase, and aspartate semialdehyde dehydrogenase.

For the genes of enzymes that undergo feedback inhibition by L-lysine, such as dihydropicolinate acid synthase and aspartokinase, it is desirable to removed feedback inhibition by L-Lysine.

Further, the ability to produce L-amino acid can also be enhanced by increasing the activity of proteins encoding export of L-amino acids to the outside of the bacterium. For example, the LysE protein encoded by the lysE gene is known as a protein that contributes to the export of L-lysine to the outside of the cell (Vrljic, M., Sahm, H. and Eggeling, L. (1996) Mol. Microbiol. 22, 815-826, International Publication No. 97/23597). The wild type of the lysE gene derived from bacteria of the genus *Brevibacterium* does not function at all in bacteria of the genera *Methylophilus* and *Methylobacillus,* but that mutant lysE permitting functioning in methylotrophes was possible. The mutantlysE24 protein described in the embodiments below is an example of such a modification product of the lysE protein. The methanol-assimilating bacterium AJ110196, which has the gene lysE24 and a gene encoding dihydrodipicolinate synthase modified so as not to subject feedback inhibition by L-lysine, was internationally deposited with the designation FERM BP-10434 under the Budapest Treaty at the International Patent Organism Depositary (Chuo No. 6, 1-banchi, 1-chome, Tsukuba-shi Higashi, Ibaraki-ken, Japan, Postal Code 305-8566) of the National Institute of Advanced Industrial Science and Technology, an Independent Administrative Institution, on October 12, 2005.

The genes that can be employed in microorganisms in the present invention will be specifically described below.

### <The dapA* gene>

The DNA encoding dihydrodipicolinate synthase which is desensitized to feedback inhibition by L-lysine (hereinafter, also referred to as the dapA* gene) is not particularly limited, however, it is preferably, for example, a DNA encoding a dihydrodipicolinate synthase derived from, or native to, a bacterium belonging to the genus *Escherichia* and having a mutation to desensitize to feedback inhibition by L-lysine.

Examples of the DNA encoding wild-type dihydrodipicolinate synthase derived from a bacterium belonging to the genus *Escherichia* include a DNA encoding the amino acid sequence of SEQ ID NO: 41. Examples of the mutation to desensitize the feedback inhibition by L-lysine include a mutation that replaces the histidine residue at position 118 in the amino acid sequence of SEQ ID NO: 41 with a tyrosine residue (H118Y mutation). Therefore, examples of the dapA* gene include a DNA encoding the amino acid sequence of SEQ ID NO: 41 in which the histidine residue at position 118 is replaced with a tyrosine residue.

The dapA* gene may be a DNA encoding a protein having at least 80%, preferably at least 90%, more preferably at least 95%, particularly preferably at least 98% homology to the entire amino acid sequence of SEQ ID NO: 41, having the H118Y mutation, and having dihydrodipicolinate synthase activity.

Moreover, the dapA* gene may be a DNA encoding a protein having the amino acid sequence of SEQ ID NO: 41, but which includes substitution, deletion, insertion, addition, or the like of one or several amino acids so long as the protein has the H118Y mutation and the dihydrodipicolinate synthase activity is not impaired.

Although the number of amino acids which constitutes "several" may differ depending on their relative positions in the three-dimensional structure of the protein, or the types of amino acid residues being altered, it is specifically 1 to 20, preferably 1 to 10, and more preferably 1 to 5. The above-mentioned substitution of amino acids is preferably a conservative substitution. Examples of conservative substitutions include: substitution of ser or thr for ala; substitution of gln, his, or lys for arg; substitution of glu, gln, lys, his, or asp for asn; substitution of asn, glu, or gln for asp; substitution of ser or ala for cys; substitution of asn, glu, lys, his, asp, or arg for gln; substitution of gly, asn, gln, lys, or asp for glu; substitution of pro for gly; substitution of asn, lys, gln, arg, or tyr for his; substitution of leu, met, val, or phe for ile; substitution of ile, met, val, or phe for leu; substitution of asn, glu, gln, his, or arg for lys; substitution of ile, leu, val or phe for met; substitution of trp, tyr, met, ile, or leu for phe; substitution of thr or ala for ser; substitution of ser or ala for thr; substitution of phe or tyr for trp; substitution of his, phe, or trp for tyr; and substitution of met, ile, or leu for val. The above-mentioned amino acid substitutions, deletions, insertions, additions, inversions, or the like may be the result of a naturally-occurring mutation (mutant or variant) due to an individual difference, or a difference of bacterial species harboring the dihydrodipicolinate synthase gene.

The dapA* gene may be a DNA which is able to hybridize with a complementary strand of the nucleotide sequence of SEQ ID NO: 40 or a probe that can be prepared from the sequence under stringent conditions so long as the gene encodes a protein having the H118Y mutation and having dihydrodipicolinate synthase activity. The term "stringent conditions" refers to conditions where a so-called specific hybrid is formed and a nonspecific hybrid is not formed. It is difficult to clearly define the conditions with a numerical value, but examples thereof include conditions corresponding to a salt concentration and temperature of washing which are typical for a standard Southern hybridization, e.g., washing at 60°C with 1×SSC and 0.1% SDS, preferably at 60°C with 0.1×SSC and 0.1% SDS, and more preferably at 68°C with 0.0×SSC and 0.1% SDS, once or preferably twice or three times.

The dapA* gene may be obtained by site-specific mutagenesis, or from the RSFD80 plasmid as described below.

It is known that the wild-type dihydrodipicolinate synthase derived from coryneform bacteria is not subject to feedback inhibition by L-lysine (J Gen Microbiol. 1988 Dec; 134 (12) :3221-9.). Therefore, it is not always necessary to use a DNA encoding a dihydrodipicolinate synthase which is desensitized to feedback inhibition by L-lysine.

### < DNA encoding mutant LysE that promotes export of L-lysine to the outside of the bacterium when the DNA is introduced into methanol-assimilating bacterium >

Examples of the DNA encoding mutant LysE that promotes export of L-lysine to the outside of a bacterium when the DNA is introduced into a methanol-assimilating bacterium include the LysE24 gene (US 2003-0124687), lysE56 gene (US 2004-0146974), and lysE24m5 (WO 2006/059715).

The expression "promoting export of L-lysine to the outside of a bacterium" means that when a methanol-assimilating bacterium containing the DNA is cultured in a medium, the amount of L-lysine exported into the medium increases as compared with the methanol-assimilating bacterium not containing the DNA. An increase in the export of the L-lysine to the outside of the cell occurs when there is an increase in L-lysine accumulation in the medium during the culture of the methanol-assimilating bacterium containing the DNA as compared with the accumulation when the methanol-assimilating bacterium not containing the DNA is cultured.

The DNA encoding mutant LysE that promotes export of L-lysine to the outside of a bacterium when the DNA is introduced into the methanol-assimilating bacterium is preferably, but is not limited to, a DNA encoding the LysE protein derived from a bacterium belonging to the genus *Brevibacterium* and having a mutation for promoting export of L-lysine to the outside of a bacterium when the DNA is introduced into the methanol-assimilating bacterium, and examples thereof include the LysE24 gene (US 2003-0124687), lysE56 gene (US 2004-0146974), and lysE24m5 (WO 2006/059715).

### (1) lysE24 gene

The lysE24 gene is a DNA encoding a mutant of a protein having a loop region and six hydrophobic helixes that is involved in the export of L-lysine to the outside of a bacterium (wild-type lysE protein: SEQ ID NO: 55), wherein the mutant lysE protein does not have the above-mentioned loop region and promotes export of L-lysine, L-arginine, or both to the outside of a bacterium when the DNA is introduced into the methanol-assimilating bacterium. Examples of the LysE24 gene include the LysE24 gene described in JP 2004-166594 A (US 2005-003495).

An example includes a DNA encoding the protein of SEQ ID NO: 51. In addition, the protein may be a protein having not less than 80%, preferably not less than 90%, more preferably not less than 95%, particularly preferably not less than 98% homology to the entire amino acid sequence of SEQ ID NO: 51 so long as the gene can promote export of L-lysine to the outside of a bacterium when it is introduced into a methanol-assimilating bacterium.

Moreover, the gene may be a DNA encoding a protein having the sequence of SEQ ID NO: 51, but which includes substitutions, deletions, insertions, additions, or the like of one or several amino acids so long as the activity for promoting export of L-lysine to the outside of a cell is not impaired.

Moreover, the lysE24 gene may be a DNA which is able to hybridize with a complementary strand of the nucleotide sequence of SEQ ID NO: 50 or a probe that can be prepared from the nucleotide sequence under stringent conditions so long as the gene encodes a protein having the activity for promoting export of L-lysine to the outside of a cell.

The definitions of the terms "several" and "stringent conditions" and preferable amino acid substitutions are the same as described above.

The lysE24 gene can be obtained from, for example, the plasmid pRSlysE24 described in JP 2004-166594 A (US 2005-003495). *E.coli* JM109 strain transformed with pRSlysE24 was designated as AJ13830, and deposited at National Institute of Advanced Industrial Science and Technology, International Patent Organism Depository on June 4, 2001 and given an accession number of FERM P-18369. Then, the deposit was converted to an international deposit under the provisions of the Budapest Treaty on May 13, 2002, and given the accession number FERM BP-8040.

### (2) lysE56 gene

An example of the lysE56 gene includes a gene encoding a protein having the amino acid sequence of SEQ ID NO: 55 in which at least the glycine residue at position 56 is replaced with another amino acid residue (US 2004-0146974). The gene may also encode a protein having the amino acid sequence of SEQ ID NO: 55 having not less than 80%, preferably not less than 90%, more preferably not less than 95%, particularly preferably not less than 98% homology to SEQ ID NO: 55, in which the glycine residue at position 56 is replaced with another amino acid residue, so long as the gene can promote export of L-lysine to the outside of a methanol-assimilating bacterium.

### (3) lysE24m5 gene

This gene includes a DNA having the nucleotide sequence of the lysE24 gene, which has been modified so that each reading frame includes a stop codon, and promotes export of L-lysine, L-arginine, or both to the outside of a methanol-assimilating bacterium when the DNA is introduced into the bacterium. Specific examples thereof include the DNA having the nucleotide sequence of SEQ ID NO: 56 (WO 2006/059715). The gene may be a DNA which is able to hybridize with a complementary strand of the nucleotide sequence of SEQ ID NO: 56 or a probe that can be prepared from the nucleotide sequence under stringent conditions so long as the gene encodes a protein having activity for promoting export of L-lysine to the outside of a cell.

### <The ddh gene>

The diaminopimelate dehydrogenase activity can be enhanced using a gene encoding diaminopimelate dehydrogenase (hereinafter, also referred to as the ddh gene).

An example of the ddh gene includes, but is not limited to, a DNA encoding diaminopimelate dehydrogenase derived from, or native to, a coryneform bacterium (SEQ ID NO: 53).

The ddh gene may be a DNA encoding a protein having not less than 80%, preferably not less than 90%, more preferably not less than 95%, particularly preferably not less than 98% homology to the entire amino acid sequence of SEQ ID NO: 53 and having the diaminopimelate dehydrogenase activity.

The ddh gene may also be a DNA encoding the protein having a sequence of SEQ ID NO: 53, but which includes one or several amino acid substitutions, deletions, insertions, additions, or the like so long as diaminopimelate dehydrogenase activity is not impaired.

Moreover, the ddh gene may be a DNA which is able to hybridize with a complementary strand of the nucleotide sequence of SEQ ID NO: 52 or a probe that can be prepared from the nucleotide sequence under stringent conditions so long as the gene encodes a protein having the diaminopimelate dehydrogenase activity.

The definitions of the terms "several" and "stringent conditions" and preferable amino acid substitution are the same as described above.

The ddh gene from coryneform bacterium can be obtained by amplification through PCR using two oligonucleotide primers (for example, SEQ ID NOS: 11 and 12 described in WO/9516042, US6,040,160), which are prepared based on the known nucleotide sequence of ddh from *Corynebacterium glutamicum* (Ishino, S. et al., Nucleic Acid Res., 15, 3917 (1987)) and using the chromosomal DNA of *Brevibacterium lactofermentum* or *Corynebacterium glutamicum* as the template.

### <The lysA gene>

The diaminopimelate decarboxylase activity can be enhanced using the diaminopimelate decarboxylase gene (hereinafter, also referred to as the lysA gene). An example of the lysA gene includes, but is not limited to, a DNA encoding diaminopimelate decarboxylase derived from, or native to, a bacterium belonging to the genus *Methylophilus* (SEQ ID NO: 49).

The lysA gene may be a DNA encoding a protein having not less than 80%, preferably not less than 90%, more preferably not less than 95%, particularly preferably not less than 98% homology to the entire amino acid sequence of SEQ ID NO: 49 and having the diaminopimelate decarboxylase activity.

The lysA gene may also be a DNA encoding a protein having the sequence of SEQ ID NO: 49, but which includes one or several amino acid substitutions, deletions, insertions, additions, or the like so long as diaminopimelate decarboxylase activity is not impaired.

Moreover, the lysA gene may be a DNA which is able to hybridize with a complementary strand of the nucleotide sequence of SEQ ID NO: 48 or a probe that can be prepared from the nucleotide sequence under stringent conditions so long as the gene encodes a protein having diaminopimelate decarboxylase activity.

The definitions of the terms "several" and "stringent conditions" and preferable amino acid substitutions are the same as described above.

The lysA gene of *Methylophilus methylotrophus* can be obtained by PCR using two oligonucleotide primers prepared based on the known sequence and using the chromosomal DNA of *Methylophilus methylotrophus* as the template.

### <The dapB gene>

The dihydrodipicolinate reductase activity can be enhanced using a gene encoding dihydrodipicolinate reductase (hereinafter, also referred to as the dapB gene). An example of the dapB gene includes, but is not limited to, a DNA encoding dihydrodipicolinate reductase derived from, or native to, a bacterium belonging to the genus *Escherichia* (SEQ ID NO: 43).

The dapB gene may be a DNA encoding a protein having not less than 80%, preferably not less than 90%, more preferably not less than 95%, particularly preferably not less than 98% homology to the entire amino acid sequence of SEQ ID NO: 43 and having dihydrodipicolinate reductase activity.

The dapB gene may also be a DNA encoding the protein having a sequence of SEQ ID NO: 43, but which includes one or several amino acid substitutions, deletions, insertions, additions, so long as the dihydrodipicolinate reductase activity is not impaired.

Moreover, the dapB gene may be a DNA which is able to hybridize with a complementary strand of the nucleotide sequence of SEQ ID NO: 42 or a probe that can be prepared from the nucleotide sequence under stringent conditions so long as the gene encodes a protein having the dihydrodipicolinate reductase activity.

The definitions of the terms "several" and "stringent conditions" and preferable amino acid substitutions are the same as described above.

The dihydrodipicolinate reductase gene (dapB) can be amplified by PCR using two oligonucleotide primers prepared based on the known nucleotide sequence and using the chromosomal DNA of *E.coli* as a template.

### <The asd gene>

The aspartate-semialdehyde dehydrogenase activity can be enhanced using the gene encoding aspartate-semialdehyde dehydrogenase (hereinafter, also referred to as the asd gene). An example of the asd gene includes, but is not limited to, a DNA encoding aspartate-semialdehyde dehydrogenase derived from, or native to, a bacterium belonging to the genus *Escherichia* (SEQ ID NO: 45).

The asd gene may be a DNA encoding a protein having not less than 80%, preferably not less than 90%, more preferably not less than 95%, particularly preferably not less than 98% homology to the entire amino acid sequence of SEQ ID NO: 45 and having the aspartate-semialdehyde dehydrogenase activity.

The asd gene may also be a DNA encoding a protein having the sequence of SEQ ID NO: 45, but which includes one or several amino acid substitutions, deletions, insertions, additions, or the like so long as the aspartate-semialdehyde dehydrogenase activity is not impaired.

Moreover, the asd gene may be a DNA which is able to hybridize with a complementary strand of the nucleotide sequence of SEQ ID NO: 44 or a probe that can be prepared from the nucleotide sequence under stringent conditions so long as the gene encodes a protein having aspartate-semialdehyde dehydrogenase activity.

The definitions of the terms "several" and "stringent conditions" and preferable amino acid substitutions are the same as described above.

The aspartate-semialdehyde dehydrogenase gene (asd) can be amplified by PCR using two oligonucleotide primers prepared based on the known nucleotide sequence and using the chromosomal DNA of *E.coli* as the template.

### <The lysC* gene>

The methanol-assimilating bacterium of the present invention may further include a DNA encoding aspartokinase that is desensitized to feedback inhibition by L-lysine.

Examples of a DNA encoding aspartokinase that is desensitized to feedback inhibition by L-lysine (hereinafter, also referred to as lysC* gene) preferably include, but are not limited to, a DNA encoding aspartokinase derived from, or native to, a bacterium belonging to the genus *Escherichia* and having a mutation to desensitize to feedback inhibition by L-lysine.

An example of the DNA encoding the wild-type aspartokinase derived from a bacterium belonging to the genus *Escherichia* includes the DNA encoding the amino acid sequence of SEQ ID NO: 47. An example of the mutation to desensitize feedback inhibition by L-lysine includes a mutation that replaces the threonine residue at position 352 with an isoleucine residue in the amino acid sequence of SEQ ID NO: 47 (T352I mutation). Therefore, an example of the lysC* gene includes a DNA encoding the amino acid sequence of SEQ ID NO: 47 in which the threonine residue at position 352 is replaced with an isoleucine residue.

The lysC* gene may be a DNA encoding a protein having not less than 80%, preferably not less than 90%, more preferably not less than 95%, particularly preferably not less than 98% homology to the entire amino acid sequence of SEQ ID NO: 47, having the T352I mutation, and having the aspartokinase activity.

The lysC*gene may also be a DNA encoding the protein having the sequence of SEQ ID NO: 47, but which includes one or more amino acid substitutions, deletions, insertions, additions, or the like so long as it has the T352I mutation and aspartokinase activity is not impaired.

Moreover, the lysC* gene may be a DNA which is able to hybridize with a complementary strand of the nucleotide sequence of SEQ ID NO: 46 or a probe that can be prepared from the nucleotide sequence under stringent conditions so long as the gene has the T352I mutation and encodes a protein having aspartokinase activity.

The definitions of the terms "several" and "stringent conditions" and preferable amino acid substitutions are the same as described above.

The lysC* gene may be obtained by site-specific mutagenesis, or from the RSFD80 plasmid as described below.

The DNA encoding aspartokinase from which feedback inhibition by L-lysine has been removed does not necessarily have to be DNA encoding variant aspartokinase. That is, the wild form can be employed so long as the wild form of the protein is not subject to L-lysine feedback inhibition.

In the present invention, the phrase "the activity of increase the activity of enzyme" means that the enzymatic activity in the cell is increased relative to what it is in the wild strain (for example, M. methylotrophus AS1 strain) or the parent strain (the strain in which none of the activity in the cell is increased by the specific combination of enzymes in the present invention),and also means that a bacterium has an enzymatic activity that is not possessed by a wild type strain or a parent strain including the strain where enzymatic activity having present that is not present in the wild strain or the parent strain. The methods of measuring the activity of the above-described enzymes are known, and increasing of activity in the cell is readily confirmed by those of ordinary skill in the art.

Example of a procedure for enhancing an intracellular activity include,but are not limited to, the following procedure and a combination thereof The following means, and combinations thereof, are examples of means of strengthening activity within the cell. However, there is no limitation thereto.
(1) Transformation with a plasmid carrying a DNA encoding each proteins.
(2) Incorporation into the chromosome of DNA encoding the each proteins.
(3) Modifying the promoter sequences of the genes encoding the each proteins.

The methanol-assimilating bacterium that can be used in the present invention has the ability to produce L-lysine due to changes such as those mentioned above (strengthening of the expression of the each genes: lysE24, dapA*, lysA, ddh, dapB, asd, (lysC*)). Here, the phrase "the ability to produce L-lysine" means the ability to accumulate a recoverable quantity of L-lysine in the medium when the methanol-assimilating bacterium of the present invention is cultivated in medium.

The methanol-assimilating bacterium of the present invention can be a bacterium in which changes such as those set forth above have been made to a variant having a mutation such as a auxotrophic mutant,, an analog-resistant mutation, a metabolically controlled mutation, or the like. Examples are bacteria that have been changed as set forth above, such as variants requiring L-homoserine, or L-threonine and L-methionine (JP48-28078A and 56-6499A); mutant auxotroph inositol or acetic acid (JP55-9784A and 56-8692A); and mutant having resistance to oxalidine, lysine hydroxamate, S-(2-aminoethyl)cysteine, γ-methyl lysine, α-chlorocaprolactam, DL-α-amino-ε-caprolactam, α-aminolauryllactam, aspartic acid-analog, sulfa agents, quinoids, or N-lauroyl leucine.

After imparting the above-described modification(gene amplification), mutations such as auxotrophic mutant, analog resistance, metabolism controls, and the like can be introduced.

Examples of methods of increasing expression of the aspartokinase gene (lysC* gene) and dihydrodipicolinate synthase gene (dapA* gene) that is not subject to feedback inhibition by L-lysine and will be shown below.

To increase the expression of the dapA* gene and the lysC* gene, DNA fragments of the gene are ligated to a vector that is replicational in bacteria of the genus *Methylophilus,* desirably a multicopy vector, to prepare recombinant DNA. This is then transformed into a host Methylophilus bacterium. Since the number of copies of these genes is increased, the activity of dihydrodipicolinate synthase and aspartokinase in the cell is increased. And below, dihydrodipicolinate synthase will be abbreviated to "DDPS," aspartokinase to "AK," and aspartokinase III to "AKIII"

Any microorganism of the genus *Methylophilus* having DNA capable of expressing DDPS activity and AK activity can use as the microorganism to which both the gene encoding DDPS and the gene encoding AK are provided. The microorganism may be a wild strain or a mutant strain derived wild type strain. E. coli (Escherichia coli) K-12 strain and *Methylophilus methylotrophus* ASI strain (NCIMB 10515) include as the host strain. The DNA sequence of the gene encoding DDPS derived from genus *Escherichia* (Richaud, F, et al. J. Bacteriol., 297 (1986)) and that of the gene encoding AKIII (Cassan, M., Pasrot, C., Cohen, G.N. and Patte, J.C., J. Biol. Chem., 261, 1052 (1986)) have both been determined. Thus, these genes can be obtained by synthesized primer based on the sequences of the genes and using PCR with chromosomal DNA of E. coli K-12 or the like as tempelate. The examples of dapA and lysC derived from E. coli will be shown below, but the genes employed in the present invention are not limited thereto.

The DDPS and AK used in the present invention are not subject to feedback inhibition by L-lysine. Wild-type DDPS derived from E. coli is known to be subject to feedback inhibition by L-lysine. Wild-type AKIII derived from E. coli is also known to be inhibited by L-lysine and to be subject to feedback inhibition by L-lysine. Accordingly, it is desirable to introduce mutations that remove this feedback inhibition by L-lysine for introduction into bacteria of the genus *Methylophilus.*

However, for example, since DDPS derived from bacteria of the genus *Corynebacterium* is not subject to feedback inhibition by L-lysine, the DDPS gene and the AK gene employed in the present invention are not necessarily variant genes.

It is possible to obtain dapA* encoding DDPS and lysC* encoding AK in which feedback inhibition by L-lysine has been removed by PCR with two oligonucleotide primers prepared using known base sequences employing plasmid containing these genes as template.

The broad-host-range plasmid RSFD80 is known as a plasmid containing dapA* and lysC* (WO95/16042). An E. coli JM109 strain that has been transformed with this plasmid has been named "AJ12396." This strain was deposited as repository number FERM P-13936 with the National Institute of Bioscience and Human-Technology, Agency of Industrial Science and Technology, MITI (now the International Patent Organism Depositary of the National Institute of Advanced Industrial Science and Technology, an Independent Administrative Institution) on October 28, 1993, and transferred to international depositary authority under the Budapest Treaty on November 1, 1994, with the designation FERM BP-4859. RSFD80 can be obtained by known methods from the AJ12396 strain.

The dapA* contained in RSFD80 has a sequence in which the C at base number 597 is changed to T in the base sequence of the wild-type dapA gene given by SEQ ID NO: 40. Thus, the variant form of DDPS that is encoded has a sequence in which the histidine residue at position 118 in the amino acid sequence given by SEQ ID NO: 41 is replaced with a tyrosine residue. The lysC* contained in RSFD80 has a sequence in which the C at base number 1638 is changed to T in the base sequence of the wild-type lysC given by SEQ ID NO: 46. Thus, the variant form of AKIII that is encoded has a sequence in which the threonine residue at position 352 in the amino acid sequence given by SEQ ID NO: 47 is replaced with an isoleucine residue.

The plasmid used to clone the gene can be be replicable in a microorganism such as a bacterium of genus *Escherichia;* examples are: pBR322, pTWV228, pMW119, and pUC19.

The vector functioning in a bacterium of the genus *Methylophilus* is, for example, a plasmid that is autonomously replicable in a bacterium of the genus *Methylophilus.* Examples are the broad-host-range vector RSF1010 and its derivatives, such as pAYC32 (Chistorerdov, A. Y., Tsygankov, Y. D. Plasmid, 1986, 16, 161-167) and pMFY42 (Gene, 44, 53 (1990)); pRP301; and pTB70 (Nature, 287, 396 (1980)). pBBR1, another broad-host-range vector that is incompatible with RSF1010, and its derivatives, such as pBHR1 (Antoine, R. and Locht, C., Molecular Microbiology, 6,1785-99 (1992)) are further examples.

In ligating DNA encoding dapA*, lysC*, and other proteins to a vector functioning in a bacterium of the genus Methylophilus to prepare recombinant DNA, the vector is cut with restriction enzyme corresponding to the terminals of the DNA fragments containing these genes. The ligation is normally conducted with a ligase such as T4 DNA ligase. These genes can be carried into separate vectors, or carried into a single vector.

The usual methods, well known to those of ordinary skill in the art, can be employed to cut and ligate DNA; prepare chromosomal DNA; conduct PCR; prepare plasmid DNA; conduct transformation; identify oligonucleotides for use as primers; and the like. These methods are described in Sambrook, J., Fritsch, E. F., and Maniatis, T., "Molecular Cloning, A Laboratory Manual, Second Edition," Cold Spring Harbor Laboratory Press (1989) and the like.

Any method that affords adequate transformation efficiency can be employed to introduce the recombinant thus prepared into a bacterium of the genus *Methylophilus.* One example is electroporation (Canadian Journal of Microbiology, 43. 197 (1997)).

DDPS activity and AK activity can be increased by introducing multiple copies of dapA* and lysC* into the chromosomal DNA of a bacterium of the genus *Methylophilus.* The introduction of multiple copies of dapA* and lysC* onto the chromosomal DNA of a bacterium of the genus *Methylophilus* can be accomplished by using a sequence that is present in multiple copies on the chromosomal DNA as a target and conducting homologous recombination. Repetitive DNA and the inverted repeats present on the ends of transposons can be employed as sequences present in multiple copies on chromosomal DNA. Alternatively, as is disclosed in JP2-109985A, dapA* and/or lysC* can be carried into a transposon and transposed to introduce multiple copies into chromosomal DNA. With any method employed, the increase in the number of copies of dapA* and lysC* in the transformant amplifies DDPS activity and AK activity.

In addition to the above-described gene amplification, DDPS activity and AK activity can also be increased by substituting strong promoter for dapA* and lysC* promoters (see Japanese Patent Application Publication No. Heisei 1-215280). Examples of known strong promoters are lac promoter, trp promoter, trc promoter, tac promoter, lambda phage PR promoter, PL promoter, tet promoter, amyE promoter, and spac promoter. The substitution of these promoters strengthens the expression of dapA* and lysC*, thereby amplifying DDPS activity and AK activity. The enhancement of expression regulation sequences can be combined with increasing the number of copies of dapA* and lysC*.

Enhancing the expression of the dapA* gene and lysC* gene to increase the activity of DDPS and AK within the cell has been described. Strengthening of the expression of the lysE24 gene, dapB gene, lysA gene, ddh gene, and asd gene is also similarly possible.

In addition to enhancing the expression of the above-described six or seven genes, other enzymes involved in the biosynthesis of L-lysine can also be strengthened in the methanol-assimilating bacterium of the present invention. Examples of such enzymes are phosphenol pyruvate carboxylase (JP 60-87788A), aspartate aminotransferase (JP6-102028A), enzymes in the diaminopimelic acid pathway, such as diaminopimelate epimerase (JP2003-135066A), and enzymes in the aminoadipic acid pathway, such as homoaconitate hydratase.

Examples of enzymes catalyzing reactions branching away from L-lysine synthesis pathways to produce compounds other than L-lysine are homoserine dehydrogenase (see WO 95/23864) and L-lysine decarboxylase (Japanese Patent Application Publication No. 2004-254544). Modifications that reduce enzymatic activity can be effected by methods such as inactivation of the gene by homologous recombination.

The methanol-assimilating bacterium of the present invention can also be modified to require L-methionine for growth (JP2004-248669A). Modification to require L-methionine can be accomplished by subjecting the methanol-assimilating bacterium of the present invention to natural mutation or a mutagenic treatment so that it cannot grow in medium that does not contain L-methionine, or by disruption of the metA gene (JP2004-248669A) or metF gene (SEQ ID NO: 58).

Examples of bacteria producing L-amino acids other than L-lysine that can be employed in the present invention will be described below. The ability to produce an L-amino acid can be imparted by obtaining a variant with a auxotrophic mutant an analog-mutant, or a metabolically controlledmutant; creating a gene recombinant strain with increased expression of an enzyme involved in the biosynthesis of the L-amino acid; or applying a method that has conventionally been used to breed bacteria of the genus Escherichia, coryneform bacteria, or the like (see Amino Acid Fermentation, Gakkai Shuppan Center, 1st ed. May 30, 1986, pp. 77-100).

Further, an L-amino acid-producing bacteria can also be constructed by amplified genes related to the biosynthesis of the L-amino acid and attenuation of the genes that degradate the L-amino acid.

### <2-2 Imparting the ability to produce L-glutamic acid>

The ability to produce L-glutamic acid can be imparted to a bacterium of the genus *Methylophilus,* for example, by introducing DNA encoding enzymes such as: glutamate dehydrogenase (JP61-268185A); glutamine synthetase, glutamate synthase, and isocitrate dehydrogenase (JP62-166890 and 63-214189); aconitate hydratase (JP62-294086A); citrate synthase (JP62-201585A and Showa 63-119688A); phosphenol pyruvate carboxylase (JP60-87788A and JP62-55089A); pyruvate dehydrogenase, pyruvate kinase, phosphenol pyruvate synthase, enolase, phosphoglyceromutase, phosphoglycerate kinase, glyceryl aldehyde-3-phosphate dehydrogenase, triosephosphate isomerase, fructose bisphosphate aldolase, and phosphofructokinase (JP63-102692A); and glucose phosphate isomerase and glutamine oxoglutarate aminotransferase (WO 99/07853).

In the microorganism of the present invention, the activity of enzymes catalyzing reactions degradating L-glutamic acid biosynthesis pathways to produce compounds other than L-glutamic acid can be decreased or deleted. Examples of enzymes catalyzing reactions degradating L-glutamic acid biosynthesis pathways to produce compounds other than L-glutamic acid are α-ketoglutarate dehydrogenase (αKGDH), isocitrate lyase, phosphate acetyl transferase, acetate kinase, acetohydroxylate synthase, acetolactate synthase, formate acetyltransferase, lactate dehydrogenase, glutamate decarboxylase, and 1-pyrroline dehydrogenase.

### <2-3 Imparting the ability to produce L-threonine>

The ability to produce L-threonine can be imparted or strengthened by, for example, increasing the activity of aspartokinase, homoserine dehydrogenase, homoserine kinase, and threonine synthase. The activity of these enzymes can be increased, for example, by transforming a bacterium of the genus *Methylophilus* with a recombinant plasmid containing a threonine operon (see JP 55-131397A,JP59-31691A, and JP56-15696;A and JP3-501682A).

Further, production ability can be imparted and increased by amplifying or introducing a threonine operon having a gene encoding aspartokinase from which feedback inhibition by L-threonine has been removed (JP1-29559A), a gene encoding homoserine dehydrogenase (JP60-012995A), or genes encoding homoserine kinase and homoserine dehydrogenase (JP61-195695).

The ability to produce L-threonine can also be enhanced by introducing DNA encoding variant phosphenol pyruvate carboxylase having a mutation that removes feedback inhibition by aspartic acid.

### <2-4 Imparting the ability to produce L-valine>

The ability to produce L-valine can be imparted by, for example, introducing genes related to the biosynthesis of L-valine from which the control mechanisms have essentially been removed into a bacterium of the genus *Methylophilus.* Mutations that essentially remove the control mechanisms from genes related to the biosynthesis of L-valine that are present in microorganisms belonging to the genus *Methylophilus* can also be introduced.

An example of a gene related to the biosynthesis of L-valine is the ilvGMEDA operon of E. coli. The threonine deaminase encoded by the ilvA gene catalyzes the deamination reaction from L-threonine to 2-ketobutyric acid, which is a rate-limiting step in the biosynthesis of L-isoleucine. Accordingly, to make the L-valine synthesis reaction progress efficiently, it is desirable to employ an operon that does not express threonine deaminase activity. The ilvGMEDA operon, in which either ilvA has been damaged or a mutation that causes the loss of threonine deaminase activity has been introduced into ilvA, and the ilvGMED operon, in which ilvA has been deleted, are examples of such ilvGMEDA operons that do not express threonine deaminase activity.

Further, expression of the ilvGMEDA operon is regulated (attenuated) by L-valine and/or L-isoleucine and/or L-leucine. Thus, it is desirable to remove or bring about mutation of the region required for attenuation to remove expression inhibition by the L-valine that is produced.

An ilvGMEDA operon, such as the above, that does not express threonine deaminase activity and that is not attenuated can be obtained by subjecting the wild-type ilvGMEDA operon to a mutagenic treatment or through changes effected by genetic recombination techniques (see WO 96/06926).

### <2-5 Imparting the ability to produce L-leucine>

The ability to produce L-leucine can be imparted or increased by, for example, introducing genes relating to the biosynthesis of L-leucine from which the control mechanisms have been essentially removed into a microorganism belonging to the genus Methylophilus in addition to the above-described properties required for the production of L-valine. It is also possible to introduce mutations that essentially remove the control mechanisms of the genes related to the biosynthesis of L-leucine that are present in microorganisms belonging to the genus Methylophilus. An example of such a gene is the leuA gene, from which inhibition by L-leucine has been essentially removed.

### <2-6 Imparting the ability to produce L-isoleucine>

The ability to produce L-isoleucine can be imparted by introducing the thrABC operon comprising the thrA gene encoding aspartokinase I-homoserine dehydrogenase I from which inhibition by L-threonine has been essentially removed, derived from E. coli, and the ilvGMEDA operon, from which the regions required for attenuation have been removed, that comprises the ilvA gene encoding threonine deaminase from which inhibition by L-isoleucine has been essentially removed (see JP8-47397A).

### <2-7 Imparting the ability to produce other amino acids>

The biosynthesis of L-tryptophan, L-phenylalanine, L-tyrosine, L-threonine, and L-isoleucine can be increased by enhancing the ability to produce phosphenol pyruvate of bacteria belonging to the genus Methylophilus (WO 97/08333).

The ability to produce L-phenylalanine and L-tyrosine can be enhanced by amplifying or introducing the desensitizable chorismate mutase-prephenate dehydratase (CM-PDT) gene (see JP62-130693A) or the desensitizable 3-deoxy-D-arabino-hepturonic acid-7-phosphate synthase (DS) gene (see JP5-236947A and JP61-124375A).

Further, the ability to produce L-tryptophan can be enhanced by amplifying or introducing a tryptophan operon containing a gene encoding desensitizable anthranylate synthase (JP57-71397A and JP62-244382A, and US Patent 4,371,614).

In the present Description, the phrase "enzymatic activity is increased "normally means that the activity of the enzyme n the cell is higher than in the wild strain. When a bacterial strain with increased enzymatic activity is obtained through modification made by gene recombination techniques, it means that the activity of the enzyme in the cell is greater than before the modification. The phrase "enzymatic activity is decreased" normally means that the activity of the enzyme within the cell is lower than in the wild strain. When a bacterial strain with lowered enzymatic activity is obtained through changes made by gene recombination techniques, it means that the activity of the enzyme within the cell is lower than before the modification.

### [Example]

The present invention is described in detail below through embodiments. However, the present invention is not limited to the embodiments given below.

### [Reference Example 1]

Unless specifically stated otherwise, the reagents employed were obtained from Wako Pure Chemical Industries, Ltd. or Nacalai Tesque, Inc. The compositions of the media employed in the various embodiments were as indicated below. The pH of each medium was adjusted with NaOH or HCl.

| | |
|---|---|
| (LB medium) | |
| Tryptone peptone (made by Difco) | 10 g/L |
| Yeast extract (made by Difco) | 5 g/L |
| NaCl | 10 g/L |
| pH 7.0 | |
| [Steam sterilization was conducted for 20 minutes at 120°C] | |
| (LB agar medium) | |
| Bacto agar | 15 g/L |
| [Steam sterilization was conducted for 20 minutes at 120°C] | |
| (SEII medium) | |
| K₂HPO₄ | 1.9 g/L |
| NaH₂PO₄ | 1.56 g/L |
| MgSO₄·7H₂O | 0.2 g/L |
| (NH₄)₂SO₄ | 5 g/L |
| CuSO₄·5H₂O | 5 µg/L |
| MnSO₄·5H₂O | 25 µg/L |
| ZnSO₄·7H₂O | 23 µg/L |
| CaCl₂·2H₂O | 72 mg/L |
| FeCl₃·6H₂O | 9.7 mg/L |
| Methanol | 0.5 percent (vol/vol) |
| pH 7.0 | |
| [Steam sterilization of everything but methanol was conducted for 15 minutes at 121°C. The methanol was added after the components had suitably cooled.] | |
| (SEII production medium) | |
| K₂HPO₄ | 1.9 g/L |
| NaH₂PO₄ | 1.56 g/L |
| MgSO₄·7H₂O | 0.2 g/L |
| (NH₄)₂SO₄ | 5 g/L |
| CuSO₄·5H₂O | 5 µg/L |
| MnSO₄·5H₂O | 25 µg/L |
| ZnSO₄·7H₂O | 23 µg/L |
| CaCl₂·2H₂O | 72 mg/L |
| FeCl₃·6H₂O | 9.7 mg/L |
| Sodium pyruvate | 2.5 g/L |
| CaCO₃ (made by Kanto Chemical Co., Inc.) | 30 g/L |
| Methanol | 2 percent (vol/vol) |
| pH 7.0 | |
| [Steam sterilization of everything but methanol was conducted for 15 minutes at 121°C. The methanol was added after the component had suitably cooled.] | |
| (SEII agar medium) | |
| K₂HPO₄ | 1.9 g/L |
| NaH₂PO₄ | 1.56 g/L |
| MgSO₄·7H₂O | 0.2 g/L |
| (NH₄)₂SO₄ | 5 g/L |
| CuSO₄·5H₂O | 5 µg/L |
| MnSO₄·5H₂O | 25 µg/L |
| ZnSO₄·7H₂O | 23 µg/L |
| CaCl₂·2H₂O | 72 mg/L |
| FeCl₃·6H₂O | 9.7 mg/L |
| Sodium pyruvate | 1.0 g/L |
| Methanol | 1 percent (vol/vol) |
| pH 7.0 | |
| Bacto agar (made by Difco) | 15 g/L |
| [Steam sterilization of everything but methanol was conducted for 15 minutes at 121°C. The methanol and, as needed, L-methionine solution adjusted to 20 g/L and sterilized by filtration, were added after the components had suitably cooled.] | |

### [Reference Example 1]

### <Construction of a mini-Mu system, pMIV-Km, pMTV-Km-EA, pAET7>

To increase the number of copies of the lysE24 and variant dapA genes on the chromosome, a gene recombination system employing the functions of Mu-phage, a bacteriophage discovered in Escherichia coli, was employed.

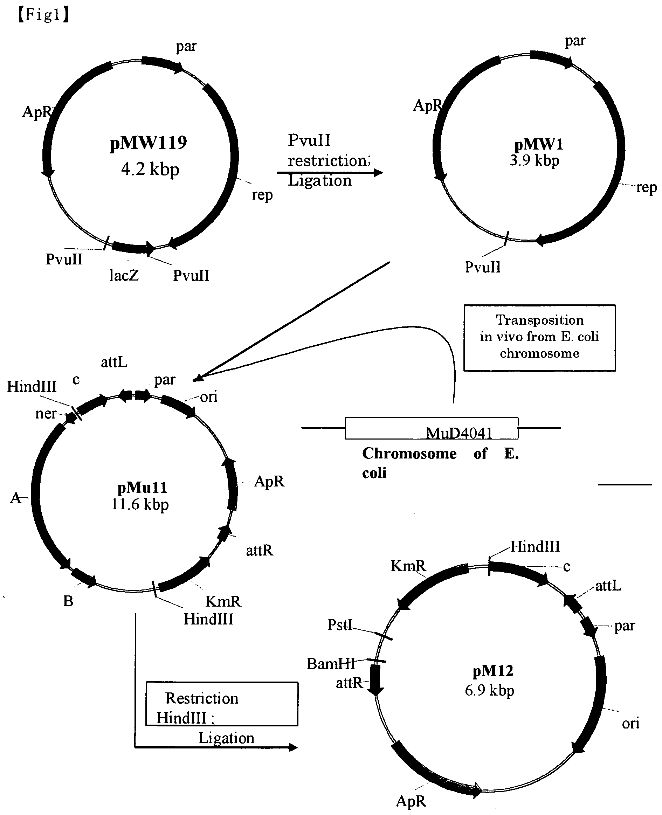

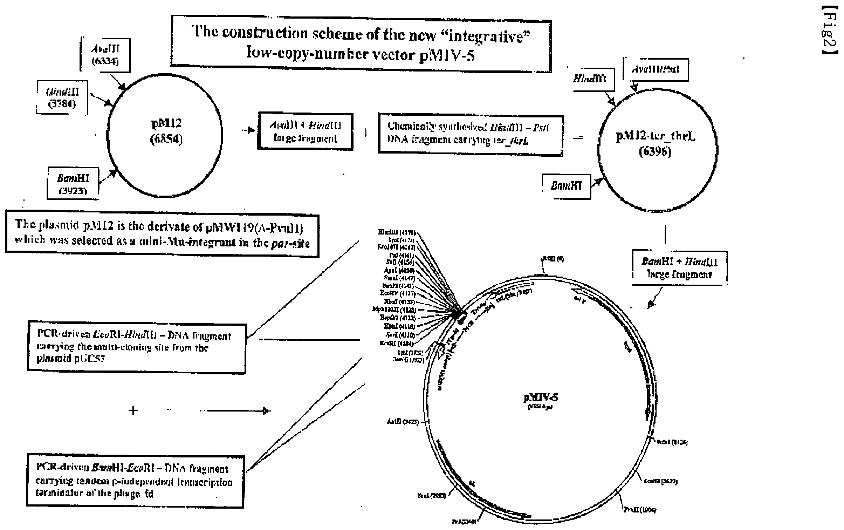

### Construction of pMIV5 (Figs. 1 and 2)

In order to incorporate Mu-phage into the chromosome of Escherichia coli, a drug resistance gene located between the recognition sequences attL and attR, and a transferase (Mu transposase) are required. It is not always necessary that both of them are incorporated into the same vector, and first, the pMIV5 plasmid was contructed, which contains the recognition sequences attL and attR and a kanamycin resistance gene, while the pAET7 plasmid with the Mu transposase was constructed separately. Both of the plasmids can function when they are present in the same bacterium, resulting in the transfer of the region located between attL and attR to the chromosomal DNA of the strain.

The pMIV5 plasmid was constructed as follows. First, pMW119 (available from TOYOBO Co., Ltd.) is digested with the PvuII restriction enzyme and separated by agarose gel electrophoresis to collect a fragment of about 3.9 kbp. This fragment was ligated with the DNA Ligation Kit (Takara Bio Inc.), to obtain the pMW1 plasmid. Subsequently, mini-Mu-phage was transferred to the pMW1 plasmid in E.coli cells. Specifically, pMD4041 (Journal of Bacteriology 158, 488-495 (1984)) was introduced into the Escherichia coli K12 strain, and a strain resistant to kanamycin and sensitive to ampicillin was selected to obtain a strain in which the plasmid pMD4041 was eliminated and mini-Mu 4041 was transferred to the chromosome. A factor that represses Mu transfer of mini-Mu4041, for example, c repressor, has a temperature-sensitive mutation. Therefore, when the strain is cultured at 42°C, c repressor of mini-Mu on the chromosome is deactivated, and transfer of mini-Mu4041 is significantly activated to cause effective transfer of mini-Mu to the chromosome, resulting in cell death. The strain with lysogenized mini-Mu4041 on the chromosome was transformed with pMW1 at 30°C. This strain with the plasmid pMW1 was cultured in LB medium until the number of cells reached 2 × 108 cells/ml and treated at 42°C for 1 hour. In order to obtain a plasmid with mini-Mu4041 transferred to the pMW1 plasmid, plasmid DNA was prepared from the cells and used to transform an Escherichia coli strain. Plasmids were prepared from 50 strains of transformants having kanamycin resistance and ampicillin resistance, and the structures of the plasmids were determined by treating with a restriction enzyme, to thereby select the plasmid of interest. This plasmid was designated as pMu11. In the plasmid pMu11, mini-Mu4041 was transferred to the par region in the pMW1. More specifically, in pMW119 containing fragments of the known plasmids pBR322 and pSC101, mini-Mu4041 was inserted into the position 259, where the boundary position between the plasmids was defined as position 0. The plasmid was digested with the HindIII restriction enzyme and separated by agarose gel electrophoresis to collect a fragment of about 6.9 kbp, and this fragment was ligated with the DNA Ligation Kit (Takara Bio Inc.), to construct the pM12 plasmid (Fig. 1). pM12 was digested with HindIII-AvaIII (EcoT22I) and used as a vector into which the terminator region of the PCR-amplified thr operon of E.coli was inserted. PCR amplification was performed using the chromosomal DNA of E.coli as the template and p-ter-thrL-f (SEQ ID NO: 1) and p-ter-thrL-r (SEQ ID NO: 2) as primers, under the following conditions for 25 cycles: denaturation at 94°C for 20 seconds, annealing at 55°C for 30 seconds, and extension at 72°C for 60 seconds. The resulting plasmid was digested with the EcoRI-HindIII restriction enzyme, and the PCR-amplified multi-cloning site region and the PCR-amplified ρ-factor independent transcription termination factor fragment from a bacteriophage fd were inserted into the plasmid. The multi-cloning site region was amplified by PCR using the pUC57 plasmid (Fermentus AB, available from LITHUANIA) as the template and pUC57-MCS-f (SEQ ID NO: 3:) and pUC57-MCS-r (SEQ ID NO: 4) as primers, and PCR was performed under the following conditions for 25 cycles: denaturation at 94°C for 20 seconds, annealing at 55°C for 30 seconds, and extension at 72°C for 30 seconds. The amplified fragment was digested with EcoRI-BamHI, the recognition sites of which had been added into the primer. The ρ-factor independent transcription termination factor fragment was amplified by PCR using the genomic DNA of the bacteriophage fd as the template and ter-fd-f (SEQ ID NO: 5) and ter-fd-r (SEQ ID NO: 6:) as primers, and PCR was performed under the following conditions for 25 cycles: denaturation at 94°C for 20 seconds, annealing at 55°C for 30 seconds, and extension at 72°C for 30 seconds. The amplified fragment was digested with EcoRI-HindIII, recognition sites of which had been added into the primer. The three fragments were ligated with the DNA Ligation kit (Takada Bio Inc.) to construct the pMIV5 plasmid (Fig. 2). Then, the plasmid was digested with EcoRV, and a kanamycin resistance gene fragment obtained by cleaving a commercially-available plasmid (pUC4K) with HincII was inserted into the plasmid to construct the pMIV5-Km plasmid.

### <pMN-Km-lysE24dapA>

A gene of interest was inserted into the pMIV5-Km plasmid and used to incorporate the gene with mini-Mu, and the plasmid was used to transfer the gene fragment of interest to the chromosome. Specifically, the plasmid pMIV5-Km was digested with SmaI, followed by a dephosphorylation treatment. On the other hand, the fragment lysE24+dapA* was obtained by amplification through PCR using a known plasmid containing the pRSlysEdapA gene (JP 2003-61687 A), as the template and pRS-1s (SEQ ID NO: 8:) and dapA-r (SEQ ID NO: 7:) as primers. PCR was performed under the following conditions for 25 cycles: denaturation at 94°C for 20 seconds, annealing at 55°C for 30 seconds, and extension at 72°C for 120 seconds. PCR-amplified fragments were ligated with the TaKaRa BKL kit (Takara Bio Inc.) to construct the pMIV-Km-lysE24dapA plasmid.

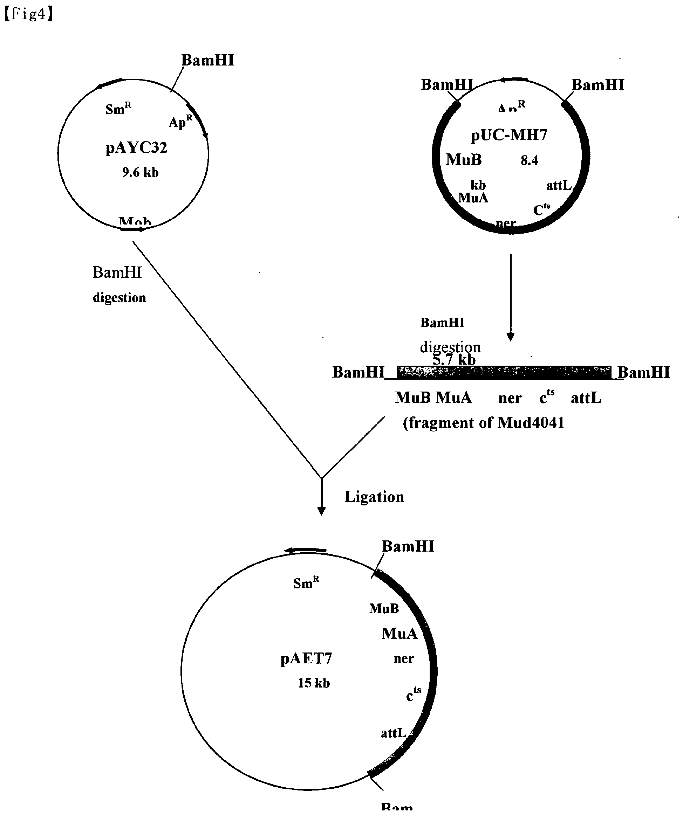

### <Construction of pAET7 >(Fig. 4)

pUC1918 (Gene, (1993) 134, 89-91) was digested with the EcoRI restriction enzyme, and the resulting fragment was blunt-ended. This fragment was used to insert the product obtained by blunt-ending a DNA fragment encoding Mu transposase, and a DNA fragment was obtained by digesting pMu4041 (Journal of Bacteriology, (1984), 158, 488-495) with ScaI-Eco47III. This plasmid was designated as pUC-MH7. pUC-MH7 was digested with BamHI, and the resulting DNA fragment encoding Mu transposase was inserted into the BamHI site of pAYC32 (Journal of General Microbiology 137, 169-178 (1991)) to obtain the pAET7 plasmid.

### [Reference Example 2]

### <Incorporation of the lysE24 and mutant dapA genes into the chromosome of Methylophilus methylotrophus, and the acquisition of VAE#1>

First, pAET7 was introduced into the M.methylotrophus AS strain by electroporation, and the bacterium was inoculated onto an SEII plate containing 50 mg/l streptomycin. Then, the pMIV-Km-lysE24dapA plasmid was introduced into the resulting transformant to obtain strains that formed colonies on the SEII plate containing 20 mg/l kanamycin and 50 mg/l streptomycin. The mini-Mu cassette includes a kanamycin resistance gene, and the pMIV-Km-lysE24dapA plasmid, which cannot replicate in M.methylotrophus. Therefore, the kanamycin-resistant strain has the mini-Mu cassette inserted into the chromosome. Accordingly, 200 strains were selected randomly from these strains and spread onto an SEII plate containing 50 mg/l streptomycin and 20 mg/L kanamycin, followed by culturing at 37°C overnight. Then, bacterial cells present on the medium surface of about 0.3 square centimeters were scraped off and inoculated into an SEII production medium (5 ml) containing 50 mg/l streptomycin and 20 mg/L kanamycin, and the cells were cultured with shaking at 37°C for 34 hours. After completion of the culture, the bacterial cells were removed by centrifugation, and the concentration of L-lysine in each culture supernatant was measured using Biotech-analyzer AS-210 (manufactured by Sakura Seiki Co., Ltd.). The strain containing the highest concentration L-lysine was selected and designated as VAE#1.

### [Reference Example 3]

Acquisition of a strain where lysE24 and mutant dapA genes are incorporated at higher copy numbers (VAE#8)

The VAE#1 strain was shown to have one or two copies of the mini-Mu cassette inserted into the chromosome. Therefore, in order to improve productivity of L-lysine, the mini-Mu cassette was amplified on the chromosome. A gene encoding the MuC protein, which is capable of suppressing the Mu transposase activity, is on the pAET7 plasmid carrying the Mu transposase. The MuC protein is temperature sensitive, and therefore, when the strain is cultured at 42°C, the Mu transposase activity is promoted, resulting in amplification of the mini-Mu cassette on the chromosome. Specifically, the VAE#1 strain was suspended in SEII liquid medium to an appropriate concentration, and the suspension was incubated at 42°C for 1 hour and diluted to appropriate concentrations. The bacterial solutions were inoculated onto SEII plates containing 50 mg/L streptomycin and 20 mg/L kanamycin to form single colonies. From the single colonies, 200 colonies were selected randomly and spread on SEII plates containing 50 mg/L streptomycin and 20 mg/L kanamycin, followed by culturing at 37°C overnight. Then, bacterial cells present on the medium surface of about 0.3 square centimeters were scraped off and inoculated into an SEII production medium (5 ml) containing 50 mg/L streptomycin and 20 mg/L kanamycin, and the cells were cultured with shaking at 37°C for 34 hours. After completion of the culture, the bacterial cells were removed by centrifugation, and the concentration of L-lysine in each culture supernatant was measured using a Biotech-analyzer AS-210 (manufactured by Sakura Seiki Co., Ltd.) to select the strain with the highest concentration of L-lysine. The strain was designated as VAE#2. The procedure was repeated 8 times to obtain the VAE#8 strain. The amount of Lys produced by VAE#1 was defined as 100, and the relative value of the amount of Lys produced by the VAE#8 strain was calculated and is shown in Table 1.

**Table 1**

| Name of strain Relative amount of produced Lys (%) | |
|---|---|
| VAE#1 | 100 |
| VAE#8 | 800 |

### [Reference Example 4]

### <Determination of transfer site in VAE#8>

Next, the site to which the mini-Mu cassette was transferred onto the chromosome of the VAE#8 strain was determined. The chromosomal DNA of the VAE#8 strain was prepared and completely digested with the SalI restriction enzyme. The resulting fragment was ligated to the pHSG398 vector, and selection was performed in an LB agar medium containing 12.5 mg/L chloramphenicol and 25 mg/L kanamycin to prepare plasmid DNA from the colonies. There is a kanamycin resistance gene on the mini-Mu cassette and chromosomal DNA around the transfer site in the plasmid. The nucleotide sequence of the plasmid was determined using a sequencing primer (SEQ ID NO: 9), which was designed outwardly in the inside of attR present on the right hand edge of the mini-Mu cassette, to thereby determine the transfer site of the mini-Mu cassette which was transferred to the chromosome. It is also possible to construct a strain identical to VAE#8 based on the information of the transfer region determined by the above-described method.

### [Reference Example 5]

### <Imparting Met-auxotrophy to VAE#8 (#403)>

Next, methionine auxotrophy was imparted to the VAE#8 strain. Imparting amino acid auxotrophy to an amino acid-producing bacterium is effective to control the number of bacterial cells during the culture. The VAE#8 strain was subjected to NTG-mutation treatment by a known method (WO 00/61723, US 7,223,572) and appropriately diluted to a cell density to form a single colony, and the bacterium was inoculated into an SEII agar medium containing 0.5 g/L L-methionine. The cells were replicated on an SEII agar medium containing no L-methionine to obtain a strain that could not grow on the plate, that is, a strain auxotrophic for L-methionine. The strain was designated as #403. A plurality of genes from the #403 strain, which was known to be involved in biosynthesis of L-methionine, were cloned by a method well-known to a person skilled in the art based on the homology to another microorganism to determine the nucleotide sequences. As a result, it was found that part of the metF gene encoding 5,10-methylenetetrahydrofolate reductase was deleted. Specifically, it was found that the region between the 92nd nucleotide and the 344th nucleotide, as counted from the initiation codon of the metF gene, was deleted. It was possible to impart L-methionine auxotrophy by disrupting the metF gene of VAE#8 by a known method (JP 2004-229662 A, Homologous recombination method using linear DNA). Details are described in Example 19. The strain with an artificially disrupted metF was found to have the same properties as a strain auxotrophic for L-methionine obtained by the above-described NTG-mutation treatment, #403. A DNA fragment for gene disruption was prepared by Over-lap-PCR (Ho, S. N., Hunt, H. D., Horton, R. M., Pullen, J. K. and Pease, L. R., Gene, 77, 51-9. (1989)). When the #403 strain and the control strain VAE#8 were cultured in SEII production medium containing 0.075 g/L L-methionine and 2.5 g/L sodium pyruvate, the amount of Lys was increased. The amount of Lys produced by the VAE#8 strain was defined as 100, and the relative value of the amount of Lys produced by the #403 strain was calculated and is shown in Table 2.

**[Table 2]**

| Name of strainRelative amount of produced Lys (%) | |
|---|---|
| VAE8 | 100 |
| #403 | 156 |

### [Reference Example 6]

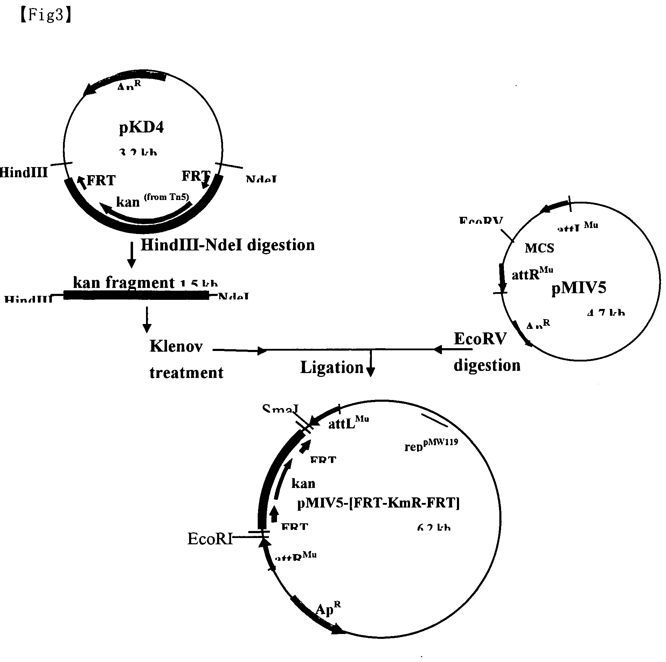

### <Construction of the pMIV-FRTGmFRT and pMIV-FRTGmFRT-EA plasmids(Fig. 3)>

In order to further incorporate a mini-Mu cassette into the #403 strain, an insertion cassette having a gene resistant to an antibiotic other than kanamycin was constructed. Specifically, the pMIV5 plasmid was digested with EcoRV and used as a vector. Then, pKD4 (Proceedings of the National Academy of Sciences of the United States of America, (2000) 97, 6640-6645) was digested with HindIII-NdeI, and the resulting fragment was blunt-ended, followed by insertion of a fragment having the kanamycin resistance gene region. This plasmid was designated as pMIV-FRTKmFRT. The plasmid was digested with the BglII restriction enzyme, and the resulting fragment was blunt-ended, followed by insertion of the PCR-amplified gentamicin resistance gene fragment. PCR was performed using pML122 (Gene, 89, 37-46. (1990)) as the template and pGm-f(SEQ ID NO: 10) and pGm-r (SEQ ID NO: 11) as primers, under the following conditions for 25 cycles: denaturation at 94°C for 20 seconds, annealing at 55°C for 30 seconds, and extension at 72°C for 60 seconds. The plasmid was designated as pMIV-FRTGmFRT. A gene of interest was inserted into the region between attL and attR to construct a mini-Mu cassette to amplify the gene of interest on the chromosome of M.methylotrophus (Fig. 3). Specifically, the pMIV-FRTGmFRT plasmid was digested with the SmaI restriction enzyme, followed by dephosphorylation. The lysE24+dapA* fragment was obtained by amplification with PCR using a known plasmid having the pRSlysEdapA gene (JP 2003-61687 A, US 7,169,587) as the template and pRS-1s and dapA-r as primers. PCR was performed under the following conditions for 25 cycles: denaturation at 94°C for 20 seconds, annealing at 55°C for 30 seconds, and extension at 72°C for 120 seconds. PCR-amplified fragments were phosphorylated using a TaKaRa BKL kit (Takara Bio Inc.) and ligated to a vector, to thereby construct the pMIV-FRTGmFRT-EA plasmid. The pKD4 and pCP20 plasmids were registered at the E.coli Genetic Stock Center as CGSC strains #7632 and #7629, respectively, and are available from the Center.

### [Reference Example 7]

Acquisition of a strain in which the lysE24 and mutant dapA genes are incorporated at higher copy numbers using pMIV-Gm-EA (#403-11-Gm)

First, pAET7 was introduced into the #403 strain, and the cells were inoculated onto an SEII plate containing 50 mg/l streptomycin. Then, the pMIV-FRTGmFRT-EA plasmid was introduced into the resulting pAET7 transformant by electroporation to obtain strains that formed colonies on an SEII plate containing 50 mg/l streptomycin. The mini-Mu cassette includes a gentamicin resistance gene, and the pMIV-FRTGmFRT-EA plasmid cannot replicate in M.methylotrophus. Therefore, the gentamicin-resistant strain has the mini-Mu cassette inserted into the chromosome. Accordingly, 200 strains were selected randomly from these strains and spread on an SEII plate containing 50 mg/L streptomycin and 20 mg/L gentamicin, followed by culturing at 37°C overnight. Then, bacterial cells present on the medium surface of about 0.3 square centimeters were scraped off and inoculated into an SEII production medium (5 ml) containing 50 mg/l streptomycin and 20 mg/L gentamicin, and the cells were cultured with shaking at 37°C for 48 hours. After completion of the culture, the bacterial cells were removed by centrifugation, and the concentration of L-lysine in each culture supernatant was measured using a Biotech-analyzer AS-210 (manufactured by Sakura Seiki Co., Ltd.) to select the strain with the highest concentration of L-lysine. The strain was designated as #403-11Gm.

### [Reference Example 8]

### <Determination of the transfer site in #403-11-Gm>

Next, the site to which the mini-Mu cassette was transferred into the chromosome of the #403-11-Gm strain was determined. The chromosomal DNA of the #403-11 strain was prepared and completely digested with the SalI restriction enzyme. The resulting fragment was ligated to the pHSG398 vector, and selection was performed in an LB agar medium containing 12.5 mg/L chloramphenicol and 25 mg/L gentamicin to prepare a plasmid DNA from the resulting colonies. There is a cloned kanamycin resistance gene on the mini-Mu cassette, as well as the chromosomal DNA around the transfer site in the plasmid. The nucleotide sequence of the plasmid was determined using a sequencing primer (SEQ ID NO: 12), which was designed in the attR region present on the right hand edge of the mini-Mu cassette, to thereby determine the transfer site of the mini-Mu cassette which had been transferred to the chromosome. It is also possible to construct a strain identical to #403-11Gm based on the information of the transfer region determined by the above-described method.

### [Reference Example 9]

### <Elimination of the antibiotic-resistant marker from #403-11-Gm (pFLP31), and acquisition of the #403-11 strain>

### <Construction of pAYCTER3>

Synthetic DNAs of SEQ ID NO: 13 and SEQ ID NO: 14, designed to contain the pUC19 multi-cloning site, were annealed by a well-known method to produce a polylinker. The polylinker was designed to have the same terminal as that of the fragment cleaved with restriction enzymes EcoRI and BglII. Moreover, the primers of SEQ ID NO: 15 and SEQ ID NO: 16 were synthesized, and the region encoding the rrnB terminator sequence was amplified by PCR from the chromosomal DNA of the Escherichia coli K-12 strain prepared by a conventional method (Saito and Miura [Biochim. Biophys. Acta, 72, 619 (1963)]). The primers of SEQ ID NO: 13 and SEQ ID NO: 14 were designed to have the recognition sequence of the restriction enzyme BglII and the recognition sequence of the restriction enzyme BclI, respectively. PCR was performed using Pyrobest DNA polymerase (manufactured by Takara Bio Inc.) under reaction conditions recommended by the manufacturer. The resulting PCR fragment was digested with restriction enzymes BglII and BclI, and the PCR fragment was ligated to the above-mentioned polylinker, to produce a DNA fragment of about 400 bp. The ligation reaction was performed with a DNA Ligation Kit Ver.2.1 (manufactured by Takara Bio Inc.) under reaction conditions recommended by the manufacturer. Then, a fragment of about 9.2 kbp, obtained by cleaving pAYC32 (J. Gen. Microbiol., 137, 169-178 (1991)) with restriction enzymes EcoRI and BamHI, was collected, and the above-mentioned DNA fragment was inserted to construct the pAYCTER3 expression plasmid , which is capable of functioning in M.methylotrophus NCIMB 10515. pAYCTER3 lacks a sequence upstream on the 5' side of the strA gene, and alternatively has a pUC19 multi-cloning site and an rrnB terminator.

### <Construction of pFLP31 >

The gentamicin resistance gene of the #403-11 Gm strain constructed in Example 7 was designed so that it is located between two FRT sequences, so the drug resistance gene can be eliminated from the chromosome by a reaction with FLP recombinase. pAYCTER3 constructed by the above-mentioned method was digested with BamHI-SmaI, and a 3.3-kbp fragment obtained by cleaving pCP20 (Proceedings of the National Academy of Sciences of the United States of America, (2000) 97, 6640-6645) with SmaI-BamHI containing an FLP recombinase was inserted. The resulting plasmid was designated as pFLP31. The plasmid pCP20 was registered at the E.coli Genetic Stock Center as CGSC strain #7629, and it is available from the Center.

### <Elimination of the antibiotic-resistance marker>

pAET7 was eliminated from #403-11 Gm by a known method to obtain a streptomycin-sensitive strain. The above-mentioned plasmid pFLP31 was introduced into the strain by electroporation, and the cells were inoculated onto an SEII agar medium containing 50 mg/L streptomycin and 0.5 g/L L-methionine. The resulting strain was suspended to an appropriate concentration in an SEII agar medium containing 50 mg/L streptomycin and 0.5 g/L L-methionine. The suspension was heated to 42°C for 1 hour and diluted to form single colonies, and the cells were inoculated onto an SEII agar medium containing 50 mg/L streptomycin and 0.5 g/L L-methionine. From the colonies, strains sensitive to gentamicin were selected. Then, pAET7 was eliminated from the strain to obtain a streptomycin-sensitive strain, which was designated as #403-11. When the #403-11 strain and a control strain (#403 strain) were cultured in an SEII production medium containing 0.075 g/L L-methionine and 2.5 g/L sodium pyruvate, the amount of Lys that was produced increased. The amount of Lys produced by the #403 strain was defined as 100, and the relative value of the amount of Lys produced by the #403-11 strain was calculated and shown in Table 3.

**Table 3**

| Name of strain | Relative amount of produced Lys (%) |
|---|---|
| #403 | 100 |
| #403-11 | 108 |

### [Reference Example 10]

### <Confirmation of increased DDPS activity>

In #403-11 obtained as described above, 8 copies of the lysE24dapA cassette was transferred to the chromosome. Therefore, an increase in the activity of dihydrodipicolinate synthase encoded by dapA was confirmed for the #403-11 strain. The dihydrodipicolinate synthase activity was measured by modifying a known method (Journal of Biological Chemistry, 240,4710-4716 (1965)). Specifically, a reaction solution was prepared so as to contain 50 mM imidazole-HCl (pH 7.4), 2 mM L-aspartate-β-semialdehyde, 2 mM sodium pyruvate, and a cell extract, and the final volume of the solution was adjusted to 1 ml. The results are shown in Table 4.

**[Table 4]**

| Name of bacterial strain (milliunit/milligram protein) | Specific activity |
|---|---|
| AS1 | 12 |
| #403-11 | 129 |

The amount of enzyme that produces 1 micromol of a product per minute was defined as 1 unit.

### [Reference Example 11]

### <Construction of the pBGEA plasmid , and construction of #403-11/pBGEA>

### (1) Construction of the pBGEA plasmid carrying an L-lysine biosynthetic enzyme gene (dapA*) and a gene having L-lysine export activity (lysE24)

In order to introduce the dapA* and LysE24 genes into a bacterium belonging to the genus Methylophilus, pBHR1 (Antoine, R. and Locht, C., Molecular Microbiology, 6, 1785-99. (1992)) was used to construct pBGEA for expressing dapA* and LysE24. First, pBHR1 was digested with the DraI restriction enzyme, and the resulting fragment was added to a phenol/chloroform solution and mixed to terminate the reaction. After the reaction mixture was centrifuged, the upper layer was collected, and DNA was collected by ethanol precipitation. The resulting DNA fragment was blunt-ended using a DNA Blunting kit (manufactured by Takara Shuzo).

On the other hand, the dapA* and LysE24 genes were obtained from pRSlysEdapA (JP 2003-61687 A, US 7,169,587). The E.coli JM109 strain transformed with the pRSlysEdapA plasmid was designated as AJ13832 and deposited at National Institute of Advanced Industrial Science and Technology, International Patent Organism Depositary on June 4, 2001 under the accession number FERM P-18371. Then, the deposit was converted to an international deposit under the provisions of the Budapest Treaty on May 13, 2002 under the accession number FERM BP-8042. First, pRSlysEdapA was digested with restriction enzymes EcoRI and BglII, and the resulting fragment was added to a phenol/chloroform solution and mixed to terminate the reaction. After the reaction mixture was centrifuged, the upper layer was collected, and DNA was collected by ethanol precipitation. Then, the DNA fragment of interest was separated by electrophoresis on an 0.8% agarose gel, and the DNA fragment of about 2.0 Kbp was collected by using EASY TRAP ver. 2 (DNA collection kit, manufactured by Takara Shuzo). The resulting DNA fragment was blunt-ended with a BKL kit (manufactured by Takara Shuzo) and phosphorylated.

The digestion product of pBHRI, and the dapA* and LysE24 gene region fragments prepared as described above were ligated by using a DNA Ligation Kit Ver. 2 (manufactured by Takara Shuzo). This ligation product was used to transform Escherichia coli (E.coli JM109 competent cells, manufactured by Takara Shuzo), and the cells were inoculated onto an LB agar medium containing 20 mg/L of kanamycin and incubated overnight at 37°C. The colonies that appeared on the agar medium were inoculated into an LB liquid medium containing 20 mg/L of kanamycin and cultured at 37°C for 8 hours with shaking. Plasmid DNA was extracted from each culture medium by the alkali-SDS method, and the structure of each plasmid was confirmed by digestion with restriction enzymes and determination of the nucleotide sequence. A plasmid with identical transcription directions of the chloramphenicol resistance gene and the dapA*and lysE24 genes was selected as pBHR-EA.

A gentamicin resistant marker was introduced into pBHR-EA obtained as described above to construct the pBGEA plasmid. First, pBHR-EA was digested with the restriction enzyme NcoI, and the resulting fragment was added to a phenol/chloroform solution and mixed to terminate the reaction. After the reaction mixture was centrifuged, the upper layer was collected, and DNA was collected by ethanol precipitation. The resulting DNA fragment was blunt-ended by using a DNA Blunting kit (manufactured by Takara Shuzo). On the other hand, the gentamicin resistance gene region was amplified by PCR using pML122 (Monika Labes, Alfred Puhler, and Reinhard Simon, Gene, 89, (1990), 37-46) as the template DNA and pGm-f(SEQ ID NO: 17) and pGm-r (SEQ ID NO: 18) as primers, and PCR was performed under the following conditions: denaturation at 94°C for 10 seconds, annealing at 60°C for 30 seconds, and extension at 72°C for 90 seconds. The PCR was performed using Pyrobest DNA polymerase (manufactured by Takara Shuzo). The resulting gentamicin resistance gene fragment was blunt-ended by using a BKL kit (manufactured by Takara Shuzo) and phosphorylated.

The digestion product of pBHR-EA and the gentamicin resistance gene region fragment prepared as described above were ligated by using a DNA Ligation Kit Ver. 2 (manufactured by Takara Shuzo). This ligation product was used to transform Escherichia coli (E.coli JM109 competent cells, manufactured by Takara Shuzo), and the cells were inoculated onto an LB agar medium containing 20 mg/L of gentamicin and incubated overnight at 37°C. The colonies that appeared on the agar medium were inoculated into an LB liquid medium containing 20 mg/L of gentamicin and cultured at 37°C for 8 hours with shaking. Plasmid DNA was extracted from each culture medium by the alkali-SDS method, and the structure of each plasmid was confirmed by digestion with restriction enzymes and determination of the nucleotide sequence to obtain pBGEA. pBGEA was then introduced into the #403-11 strain prepared in Example 9, in which the lysE24 and dapA* genes had been incorporated into the chromosome, to enhance the lysE24 and dapA* genes. The strain was designated as #403-11/pBGEA. When #403-11/pBGEA and a control strain #403-11 were cultured in an SEII production medium containing 20 mg/L kanamycin, 50 mg/L gentamicin (the medium for the control strain contains no gentamicin), 0.075 g/L L-methionine, and 2.5 g/L sodium pyruvate, the amount of Lys was increased. The amount of Lys produced by the #403-11 strain was defined as 100, and the relative value of the amount of Lys produced by the #403-11/pBGEA strain was calculated and shown in Table 5. It was found that the introduction of the plasmid increased the amount of Lys produced by the #403-11 strain.

**Table 5**

| Name of strain | Relative amount of produced Lys (%) |
|---|---|
| #403-11 | 100 |
| #403-11/pBGEA | 111 |

### [Reference Example 12]

### <Construction of the pRSlysA, pRSddh, pRSdapB, pRSasd, and pRSask plasmids, introduction of the plasmids into the #403-11/pBGEA strain, and evaluation of L-lysine productivity>

Next, expression plasmids carrying each Lys biosynthetic gene were constructed, and the plasmids were introduced into the #403-11/pBGEA strain, and the effects on L-lysine productivity were investigated.

### <1> Construction of the pRSlysA plasmid

The diaminopimelate decarboxylase gene (lysA) from Methylophilus methylotrophus was obtained by PCR using two oligonucleotide primers prepared based on a known sequence (SEQ ID NO: 13 in WO2000/061723) and using the chromosomal DNA of Methylophilus methylotrophus as the template. PCR was performed using plysA-f (SEQ ID NO: 19:) and plysA-r (SEQ ID NO: 20) as primers, under the following conditions for 25 cycles: denaturation at 94°C for 20 seconds, annealing at 55°C for 30 seconds, and extension at 72°C for 60 seconds. The amplified lysA gene fragment from M. methylotrophus was digested with Sse8387I-XbaI, recognition sites of which had been added into the primer sequence, and the resulting fragment was ligated to pRStac (JP 2003-61687 A, US 7,169,587) which had been digested with Sse8387I-XbaI. The plasmid was designated as pRSlysA.

### <2> pRSddh

The diaminopimelate hydrogenase gene (ddh) fromBrevibacterium lactofermentum was obtained by amplification with PCR using two kinds of oligonucleotide primers prepared based on the known nucleotide sequence of ddh based on Corynebacterium glutamicum (Ishino, S. et al. Nucleic acid res. 15, 3917 (1987)) and using the chromosomal DNA of Brevibacterium lactofermentum 2256 strain (ATCC 13869 strain) as the template. PCR was performed using pddh-f (SEQ ID NO: 21) and pddh-r (SEQ ID NO: 22) as primers, under the following conditions for 25 cycles: denaturation at 94°C for 20 seconds, annealing at 55°C for 30 seconds, and extension at 72°C for 60 seconds. The amplified ddh gene fragment was digested with Sse8387I-XbaI, recognition sites of which had been added into the primer sequence, and the resulting fragment was ligated to pRStac (JP 2003-61687 A, US 7,169,587) digested with Sse8387I-XbaI. The plasmid was designated as pRSddh.

### <3> pRSdapB

The dihydrodipicolinate reductase gene (dapB) from E.coli was amplified by PCR using two oligonucleotide primers prepared based on the known nucleotide sequence and using the chromosomal DNA of E.coli as the template. PCR was performed using pdapB-f (SEQ ID NO: 23:) and pdapB-r (SEQ ID NO: 24) as primers, under the following conditions for 25 cycles: denaturation at 94°C for 20 seconds, annealing at 55°C for 30 seconds, and extension at 72°C for 60 seconds. The amplified dapB gene fragment was digested with Sse8387I-XbaI, recognition sites of which had been added into the primer sequence, and the resulting fragment was ligated to pRStac (JP 2003-61687 A, US 7,169,587) which had been digested with Sse8387I-XbaI. The plasmid was designated as pRSdapB.

### <4> pRSlasd

The aspartate-semialdehyde dehydrogenase gene (asd) from E.coli was amplified by PCR using two oligonucleotide primers prepared based on the known nucleotide sequence using the chromosomal DNA of E.coli as the template. PCR was performed using pasd-f (SEQ ID NO: 25) and pasd-r (SEQ ID NO: 26) as primers, under the following conditions for 25 cycles: denaturation at 94°C for 20 seconds, annealing at 55°C for 30 seconds, and extension at 72°C for 60 seconds. The amplified ddh gene fragment was digested with Sse8387I-XbaI, reconition sites of which had been added into the primer sequence, and the resulting fragment was ligated to pRStac (JP 2003-61687 A, US 7,169,587) which had been digested with Sse8387I-XbaI. The plasmid was designated as pRSasd.

### <5> pRSask

The aspartokinase gene (ask) from Methylophilus methylotrophus was obtained by PCR using two oligonucleotide primers prepared based on a known sequence (SEQ ID NO: 5 in WO2000/061723) and using a chromosomal DNA of Methylophilus methylotrophus as a template. PCR was performed using pask-f (SEQ ID NO: 27:) and pask-r (SEQ ID NO: 28:) as primers. The following cycle was repeated 25 times: denaturation at 94°C for 20 seconds, annealing at 55°C for 30 seconds, and extension at 72°C for 90 seconds. The amplified ask gene fragment from M.methylotrophus was digested with Sse8387I-XbaI, recognition sites of which had been added into the primer and blunt-ended, and the resulting fragment was ligated to pRStac (JP 2003-61687 A, US 7,169,587) which had been digested with Sse8387I and blunt-ended. The plasmid was designated as pRSask.

### <6> Introduction and evaluation of the plasmids

The five plasmids prepared as described above were separately introduced into the #403-11/pBGEA strain prepared in Example 11, and the resulting strains were cultured in an SEII production medium containing 0.075 g/L L-methionine and 2.5 g/L sodium pyruvate. However, Lys productivity was not improved in all the strains. In the strain with ddh, Lys productivity was reduced.

### [Reference Example 13]

### <Enhancement of ddh+lysA in combination, construction of the pDA plasmid , and evaluation of productivity of L-lysine>

In order to determine the next limiting factor in Lys production using the #403-11 strain which was modified so that expression of the lysE24 and dapA* genes effective for improving Lys production in M.methylotrophus was sufficiently enhanced by gene incorporation and plasmid introduction, the genes were separately enhanced as shown in Example 12 to investigate the effects, but an effective gene was not determined. Therefore, various plasmids were constructed to enhance the genes in combination, each carrying two genes, and introduced into the #403-11 strain. As a result, it was found that enhancing lysA and ddh in combination improved productivity of L-lysine.

Diaminopimelate dehydrogenase encoded by ddh was thought to reversibly catalyze both the production and degradation reactions of diaminopimelic acid, and that enhancing ddh alone promoted not only production but also degradation of diaminopimelic acid. On the other hand, diaminopimelate decarboxylase which catalyzes the reaction subsequent to that of the diaminopimelate dehydrogenase is an irreversible enzyme that causes a decarboxylation reaction prior to L-lysine production. It was thought that enhancing both diaminopimelate dehydrogenase and diaminopimelate decarboxylase prevented degradation of diaminopimelic acid by diaminopimelate dehydrogenase and promoted synthesis of diaminopimelic acid.

Specifically, the pRSddh plasmid was digested with SapI, and the resulting fragment was blunt-ended and dephosphorylated to produce a vector, into which a PCR-amplified DNA fragment including the lysA gene region containing the tac promoter region. PCR amplification was performed using the pRSlysA plasmid as the template and ptac-f (SEQ ID NO: 29:) and plysA-r (SEQ ID NO: 20:) as primers. The following cycle was repeated 25 times: denaturation at 94°C for 20 seconds, annealing at 55°C for 30 seconds, and extension at 72°C for 60 seconds. The resulting PCR-amplified fragment was digested with XbaI, and the resulting fragment was blunt-ended and ligated to the above-mentioned vector. In the resulting plasmid, the transcription directions of the ddh and lysA genes were identical, and the plasmid was designated as pDA. When a strain into which the pDA was introduced (#403-11/pBGEA/pDA) and the control strain #403-11/pBGEA were cultured in an SEII production medium containing 20 mg/L kanamycin, 50 mg/L gentamicin, and 50 mg/L streptomycin (the medium for the control strain contains no streptomycin), 0.075 g/L L-methionine, and 2.5 g/L sodium pyruvate, the amount of Lys increased. The amount of Lys produced by the #403-11/pBGEA strain was defined as 100, and the relative value of the amount of Lys produced by the #403-11/pBGEA/pDA strain was calculated and shown in Table 6.

**Table 6**

| Name of strain | Relative amount of produced Lys (%) |
|---|---|
| #403-11/pBGEA | 100 |
| #403-11/pBGEA/pDA | 120 |

### [Reference Example 14]

### <Enhancement of further L-lysine biosynthetic genes in combination, construction of pBDAS (lysA+ddh+dapB+asd), and evaluation of L-lysine productivity>

It was found that the L-lysine productivity was improved by enhancing the ddh and lysA genes in combination, since these genes are capable of catalyzing two sequential reactions in the biosynthesis pathway. The effect of enhancing other enzyme genes in combination with ddh+lysA was investigated and it was found that the use of dapB and asd in combination with ddh and lysA improved L-lysine production.

Specifically, the pRSdapB plasmid was digested with EcoRI, and the resulting fragment was blunt-ended and dephosphorylated to prepare a vector. The plasmid pDA was digested with HpaI-SapI, and the resulting DNA fragment of 2.5 kbp, which includes lysA and ddh which each have a tac promoter upstream of the genes, was collected and blunt-ended to be ligated to the vector, to thereby construct pBDA. The plasmid was found to have the ddh and lysA genes inserted upstream of dapB so that the direction of ddh and lysA and the direction of dapB were identical. The pBDA plasmid was further digested with SapI, and the resulting fragment was blunt-ended and dephosphorylated to prepare a vector. A PCR-amplified DNA fragment including the asd gene region containing a tac promoter region was then inserted into the vector. PCR amplification was performed using the pRSasd plasmid as the template and ptac-f and pasd-r as primers. The following cycle was repeated 25 times: denaturation at 94°C for 20 seconds, annealing at 55°C for 30 seconds, and extension at 72°C for 90 seconds. The resulting PCR-amplified fragment was blunt-ended and phosphorylated, and the fragment was ligated to the above-mentioned vector. The resulting plasmid was designated as pBDAS. When a strain into which the pBDAS plasmid was introduced (#403-11/pBGEA/pBDAS) and a control strain #403-11/pBGEA were cultured in an SEII production medium containing 20 mg/L kanamycin, 50 mg/L gentamicin, and 50 mg/L streptomycin (the medium for the control strain contains no streptomycin), 0.075 g/L L-methionine, and 2.5 g/L sodium pyruvate, the amount of Lys increased. The amount of Lys produced by the #403-11/pBGEA strain was defined as 100, and the relative value of the amount ofLys produced by the #403-11/pBGEA/pBDAS strain was calculated and is shown in Table 7.

**Table 7**

| Name of strain | Relative amount of produced Lys (%) |
|---|---|
| #403-11/pBGEA | 100 |
| #403-11/pBGEA/pDA | 120 |
| #403-11/pBGEA/pBDAS | 143 |

### [Example:1]

### <The effect on fermentation results of the rate of increase in ionic concentration>

The effect on L-lysine fermentation results of the rate of increase in ionic concentration was determined with the #403-11/pBGEA/pBDAS strain.

First, the entire bacterial cell obtained by culturing the #403-11/pBGEA/pBDAS strain in SEII agar medium for 24 hours at 37°C was scraped off a single plate, the entire quantity of this suspension solution was inoculated into a 1-liter jar fermenter containing 300 mL of main culture medium, and culturing was conducted at 34°C at pH 6.6. The composition of the main culture medium is indicated below.

### <Main culture medium>

| | |
|---|---|
| K₂HPO₄ | 1.9 g/L |
| NaH₂PO₄ | 1.56 g/L |
| Sodium pyruvate | 2.5 g/L |
| MgSO₄·7H₂O | 0.2 g/L |
| (NH₄)₂SO₄ | 4.97 g/L |
| L-methionine | 0.65 g/L |
| Citric acid | 1 g/L |
| MgSO₄·7H₂O [sic] | 1 g/L |
| CuSO₄·5H₂O | 25 µg/L |
| MnSO₄·5H₂O | 125 µg/L |
| ZnSO₄·7H₂O | 115 µg/L |
| CaCl₂·2H₂O | 0.36 g/L |
| FeCl₃·6H₂O | 48.5 mg/L |
| Methanol | 2 percent (vol/vol) |
| Streptomycin sulfate | 50 mg/L |
| Gentamicin sulfate | 50 mg/L |
| Kanamycin sulfate | 25 mg/L |

### [Components other than methanol and antibiotics were mixed, sterilized by filtration, and added. Subsequently, methanol and filter-sterilized antibiotics were added.]

During culturing, the pH was adjusted to 6.6 by the addition of ammonia gas. When the methanol in the medium feeded to 0.2 percent or below, a 100 percent methanol solution was fed to conduct fed-batch culturing.

In Lys fermentation, a nitrogen source was added to the medium for Lys production and ammonium sulfate was added to the medium to supply counter ions for the targeted amino acid. In the course of adding ammonium sulfate to the medium, the effect of feeding at rates resulting in rates of increase in total ionic strength in the medium of 0.03 mol/m³/hour, 0.02 mol/m³/hour, and 0.012 mol/m³/hour were examined.

The results are given in Table 8. The production is given as a relative value where production under conditions of a rate of increase in ionic strength of 0.03 mol/m³/hour was adopted as 100 percent. As shown in Table 1, as the rate of increase in ionic strength dropped, production was found to rise. As a result, it was determined that L-lysine production was enhanced by conducting feeding so that the rate of increase in ionic strength in the medium was 0.02 mol/m³/hour, desirably 0.012 mol/m³/hour or less.

**[Table 8]**

| Rate of increase in ionic strength (mol/m³/hour) | Production Relative value (%) |
|---|---|
| 0.03 | 100 |
| 0.02 | 106 |
| 0.012 | 133 |

### [Example: 2]

To examine the effect of a reduced rate of increase in ionic strength on fermentation results, ammonium chloride and glutamic acid was employed as a monovalent counter ion was employed as a nitrogen source. The glutamic acid was employed in the form of a solution that had been neutralized with ammonia.

First, in the same manner as in Example 1, the entire bacterial cell obtained by culturing the #403-11/pBGEA/pBDAS strain in SEII agar medium for 24 hours at 37°C was scraped up, the entire quantity of this suspension solution was inoculated into a 1-liter jar fermenter containing 300 mL of main culture to 6.6 medium, and culturing was conducted at pH 6.6 and 34°C. The pH was adjusted during culturing by the addition of ammonia gas. When the methanol in the medium feeded to 0.2 percent or below, a 100 percent methanol solution was fed to conduct fed-batch culturing.

In the course of adding nitrogen sources in the form of ammonium chloride and glutamic acid to the medium, these comppounds were fed so as to achieve rates of increase in ionic strength of 0.008 mol/m³/hour and 0.006 mol/m³/hour.

The results are given in Table 9. The production is given as a relative value where production under conditions of a rate of increase in ionic strength of 0.03 mol/m³/hour with the use of ammonium sulfate was adopted as 100 percent. As a result of the test, it was confirmed that an increase in the rate of ionic strength of 0.012 mol/m³/hour or less further increased production.

**[Table 9]**

| Nitrogen source | Rate of increase in ionic strength (mol/m³/hour) | Production Relative value (%) |
|---|---|---|
| Ammonium sulfate | 0.03 | 100 |
| Ammonium sulfate | 0.02 | 106 |
| Ammonium sulfate | 0.012 | 133 |
| Ammonium chloride | 0.008 | 148 |
| Glutamic acid | 0.006 | 147 |

### Industrial Applicability

The present invention enhances the production of L-amino acid by methanol-assimilating bacteria.

## Claims

1. A method for producing an L-amino acid by a fermentation process, comprising
culturing a methanol-assimilating bacterium that has an ability to produce the L-amino acid in a liquid medium containing methanol and a substance comprising a counter ion and causing the L-amino acid to be produced and accumulated in the medium,
wherein the feeding of a substance comprising methanol and a substance comprising a counter ion to the medium is carried out by fed-batch culturing to control the total ionic strength in fermentation medium,
wherein the ionic strength is controlled to achieve a rate of increase in total ionic strength in the medium of 0.02 mol/m³/hour or less,
wherein said substance comprising a counter ion is one or more substances selected from the group consisting of ammonium sulfate, ammonium chloride, ammonium glutamate, ammonium succinate, ammonium fumarate, and ammonium aspartate, and
wherein said methanol-assimilating bacterium is a microorganism selected from the group consisting of bacteria of the genus *Acromobacter, Pseudomonas, Protaminobacter, Methanomonas, Microcyclus, Methylobacillus, Bacillus,* and *Methylophilus.*

2. The method according to claim 1, wherein the ionic strength is controlled during the proliferation period of the methanol-assimilating bacterium.

3. The method according to claim 1, wherein said L-amino acid is L-lysine.

4. The method according to claim 1, wherein said methanol-assimilating bacterium has been modified to increase the activity of one or more enzymes selected from the group consisting of diaminopimelate dehydrogenase, diaminopimelate decarboxylase, and aspartate semialdehyde dehydrogenase.

5. The method according to claim 4, wherein said methanol-assimilating bacterium comprises DNA encoding dihydrodipicolinate synthase and/or aspartokinase that has been modified so as not to subject to feedback inhibition by L-lysine.

6. The method according to claim 4, wherein said methanol-assimilating bacterium comprises DNA encoding a mutant lysE protein promoting the export of L-lysine to the outside of the cell when introduced into a methanol-assimilating bacterium.

## Patentansprüche

1. Verfahren zum Produzieren einer L-Aminosäure durch ein Fermentationsverfahren, welches das Kultivieren eines Methanol assimilierenden Bakteriums mit der Fähigkeit zum Produzieren der L-Aminosäure in einem Medium, das Methanol und eine ein Gegenion enthaltende Substanz umfasst, und das Bewirken der Produktion und Anhäufung der L-Aminosäure in dem Medium umfasst,
wobei das Einspeisen einer Substanz, die Methanol und eine ein Gegenion enthaltende Substanz umfasst, in das Medium durch ein Chargenkultivierungsverfahren durchgeführt wird, um die Gesamtionenstärke in dem Medium zu kontrollieren,
wobei die Ionenstärke so kontrolliert wird, dass die Erhöhung der Gesamtionenstärke in dem Medium 0,02 Mol/m³/Stunde oder weniger ist,
wobei die ein Gegenion enthaltende Substanz mindestens eine Substanz ist, die aus der Gruppe ausgewählt ist, die aus Ammoniumsulfat, Ammoniumchlorid, Ammoniumglutamat, Ammoniumsuccinat, Ammoniumfumarat und Ammoniumaspartat besteht, und
wobei das Methanol assmilierende Bakterium ein Mikroorganismus ist, der aus der Gruppe ausgewählt ist, die aus Bakterien der Gattung Acromobacter, Pseudomonas, Protaminobacter, Methanomonas, Microcyclus, Methylobacillus, Bacillus und Methylophilus besteht.

2. Verfahren nach Anspruch 1, wobei die Ionenstärke während der Proliferationsperiode des Methanol assimilierenden Bakteriums kontrolliert wird.

3. Verfahren nach Anspruch 1, wobei die L-Aminosäure L-Lysin ist.

4. Verfahren nach Anspruch 1, wobei das Methanol assimilierende Bakterium so modifiziert worden ist, dass die Aktivität mindestens eines Enzyms erhöht ist, das aus der Gruppe ausgewählt ist, die aus Diaminopimelatdehydrogenase, Diaminopimelatdecarboxylase und Aspartatsemialdehyddehydrogenase besteht.

5. Verfahren nach Anspruch 4, wobei das Methanol assimilierende Bakterium DNA enthält, die für Dihydripicolinatsynthase und/oder Aspartokinase kodiert, die so modifiziert worden ist, dass sie einer Rückkopplungshemmung durch L-Lysin nicht unterliegt.

6. Verfahren nach Anspruch 4, wobei das Methanol assmilierende Bakterium DNA enthält, die für ein mutiertes lysE-Protein kodiert, welches den Export von L-Lysin zur Außenseite der Zelle fördert, wenn es in ein Methanol assimilierndes Bakterium eingeführt wird.

## Revendications

1. Procédé de production d'un acide L-aminé par un procédé de fermentation, comprenant
la culture d'une bactérie assimilant le méthanol qui a une capacité à produire l'acide L-aminé dans un milieu liquide contenant du méthanol et une substance comprenant un contre-ion et entraînant la production et l'accumulation d'acide L-aminé dans le milieu,
dans lequel l'introduction d'une substance comprenant du méthanol et d'une substance comprenant un contre-ion dans le milieu est réalisée par culture à alimentation discontinue pour réguler la force ionique totale dans un milieu de fermentation,
dans lequel la force ionique est régulée de manière à atteindre un taux d'augmentation de la force ionique totale dans le milieu de 0,02 mol/m³/heure ou moins,
dans lequel ladite substance comprenant un contre-ion est une ou plusieurs substances choisies dans le groupe constitué du sulfate d'ammonium, du chlorure d'ammonium, du glutamate d'ammonium, du succinate d'ammonium, du fumarate d'ammonium et de l'aspartate d'ammonium, et
dans lequel ladite bactérie assimilant le méthanol est un microorganisme choisi dans le groupe constitué des bactéries du genre *Acromobacter, Pseudomanas, Protaminobacter, Methanomonas, Microcyclus, Methylobacillus, Bacillus* et *Methylophilus.*

2. Procédé selon la revendication 1, dans lequel la force ionique est régulée au cours de la période de prolifération de la bactérie assimilant le méthanol.

3. Procédé selon la revendication 1, dans lequel ledit acide L-aminé est la L-lysine.

4. Procédé selon la revendication 1, dans lequel ladite bactérie assimilant le méthanol a été modifiée pour augmenter l'activité d'une ou plusieurs enzymes choisies dans le groupe constitué de la diaminopimélate déshydrogénase, de la diaminopimélate décarboxylase et de l'aspartate semi-aldéhyde déshydrogénase.

5. Procédé selon la revendication 4, dans lequel ladite bactérie assimilant le méthanol comprend un ADN codant pour la dihydrodipicolinate synthase et/ou l'aspartokinase qui a été modifié de manière à ne pas subir l'inhibition de rétroaction par la L-lysine.

6. Procédé selon la revendication 4, dans lequel ladite bactérie assimilant le méthanol comprend un ADN codant pour une protéine de lysE mutante favorisant l'exportation de L-lysine à l'extérieur de la cellule lorsqu'il est introduit dans une bactérie assimilant le méthanol.


### SEQUENCE LISTING

<110> Aj inomoto Co., Inc.
<120> 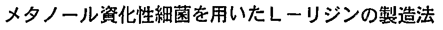
<130> C680-C6266
<150> JP2006-25617
   <151> 2006-02-02
<160> 59
<170> PatentIn version 3.1
<210> 1
   <211> 25
   <212> DNA
   <213> Artificial sequence
<220>
   <223> primer
<400> 1
   aaaaagctta acacagaaaa aagcc 25
<210> 2
   <211> 28
   <212> DNA
   <213> Artificial sequence
<220>
   <223> primer
<400> 2
   aaactgcagt ggtcgaaaaa aaaagccc 28
<210> 3
   <211> 23
   <212> DNA
   <213> Artificial sequence
<220>
   <223> primer
<400> 3
   aaagaattcg agctcggtac ctc 23
<210> 4
   <211> 23
   <212> DNA
   <213> Artificial sequence
<220>
   <223> primer
<400> 4
   aaaaagcttg catgcaggcc tct 23
<210> 5
   <211> 21
   <212> DNA
   <213> Artificial sequence
<220>
   <223> primer
<400> 5
   aaaggatccg catgccgttg a 21
<210> 6
   <211> 93
   <212> DNA
   <213> Artificial sequence
<220>
   <223> primer
<400> 6
   aaagaattcc gatacaatta aaggctcctt ttggagcctt ttttttggag attttcaacg 60
tgaaaaaatt attattcgca attccaagct aat 93
<210> 7
   <211> 36
   <212> DNA
   <213> Artificial sequence
<220>
   <223> primer
<400> 7
   cattctagat ccctaaactt tacagcaaac cggcat 36
<210> 8
   <211> 22
   <212> DNA
   <213> Artificial sequence
<220>
   <223> primer
<400> 8
   cacagagaca tattgcccgt tg 22
<210> 9
   <211> 29
   <212> DNA
   <213> Artificial sequence
<220>
   <223> primer
<400> 9
   catctgtttc atttgaagcg cgaaagcta 29
<210> 10
   <211> 21
   <212> DNA
   <213> Artificial sequence
<220>
   <223> primer
<400> 10
   cgccagccag gacagaaatg c 21
<210> 11
   <211> 22
   <212> DNA
   <213> Artificial sequence
<220>
   <223> primer
<400> 11
   gtccagcggt ttttcttggg ct 22
<210> 12
   <211> 29
   <212> DNA
   <213> Artificial sequence
<220>
   <223> primer
<400> 12
   catctgtttc atttgaagcg cgaaagcta 29
<210> 13
   <211> 57
   <212> DNA
   <213> Artificial sequence
<220>
   <223> primer
<400> 13
   aattcgagct cggtacccgg ggatcctcta gagtcgacct gcaggcatgc aagctta 57
<210> 14
   <211> 57
   <212> DNA
   <213> Artificial sequence
<220>
   <223> primer
<400> 14
   gatctaagct tgcatgcctg caggtcgact ctagaggatc cccgggtacc gagctcg 57
<210> 15
   <211> 30
   <212> DNA
   <213> Artificial sequence
<220>
   <223> primer
<400> 15
   ctatgatcat ttgcctggcg gcagtagcgc 30
<210> 16
   <211> 31
   <212> DNA
   <213> Artificial sequence
<220>
   <223> primer
<400> 16
   cttagatctc aaaaagagtt tgtagaaacg c 31
<210> 17
   <211> 21
   <212> DNA
   <213> Artificial sequence
<220>
   <223> primer
<400> 17
   cgccagccag gacagaaatg c 21
<210> 18
   <211> 22
   <212> DNA
   <213> Artificial sequence
<220>
   <223> primer
<400> 18
   gtccagcggt ttttcttggg ct 22
<210> 19
   <211> 39
   <212> DNA
   <213> Artificial sequence
<220>
   <223> primer
<400> 19
   aaacccgggg atcctgagcg ccaataccct caaacgcct 39
<210> 20
   <211> 43
   <212> DNA
   <213> Artificial sequence
<220>
   <223> primer
<400> 20
   tttcccgggc ttggcggctt cggttttttt attaggggtt gcc 43
<210> 21
   <211> 41
   <212> DNA
   <213> Artificial sequence
<220>
   <223> primer
<400> 21
   acccctgcag ggccaccaca attttggagg attacaagaa c 41
<210> 22
   <211> 33
   <212> DNA
   <213> Artificial sequence
<220>
   <223> primer
<400> 22
   tcctctagac tcgagctaaa ttagacgtcg cgt 33
<210> 23
   <211> 35
   <212> DNA
   <213> Artificial sequence
<220>
   <223> primer
<400> 23
   gcgcctgcag gcgctggtta ctctgaaaac ggtct 35
<210> 24
   <211> 46
   <212> DNA
   <213> Artificial sequence
<220>
   <223> primer
<400> 24
   gcatctagag acaatttaaa aacataacac caaaaataaa agggcc 46
<210> 25
   <211> 40
   <212> DNA
   <213> Artificial sequence
<220>
   <223> primer
<400> 25
   gcccctgcag gccggcacat ttatacagca cacatctttg 40
<210> 26
   <211> 35
   <212> DNA
   <213> Artificial sequence
<220>
   <223> primer
<400> 26
   taatctagaa agattacgcc agttgacgaa gcatc 35
<210> 27
   <211> 40
   <212> DNA
   <213> Artificial sequence
<220>
   <223> primer
<400> 27
   agggaattct aaaccggata tggcgatggc aggtggtact 40
<210> 28
   <211> 39
   <212> DNA
   <213> Artificial sequence
<220>
   <223> primer
<400> 28
   taactgcagg aagttttaat agtaccaaca cagcgcatg 39
<210> 29
   <211> 39
   <212> DNA
   <213> Artificial sequence
<220>
   <223> primer
<400> 29
   aaaagatctc ccgttctgga taatgttttt tgcgccgac 39
<210> 30
   <211> 20
   <212> DNA
   <213> Artificial sequence
<220>
   <223> primer
<400> 30
   tggactgacg gtggctactc 20
<210> 31
   <211> 18
   <212> DNA
   <213> Artificial sequence
<220>
   <223> primer
<400> 31
   gaccacgtca ttttccct 18
<210> 32
   <211> 58
   <212> DNA
   <213> Artificial sequence
<220>
   <223> primer
<400> 32
   ccagcctaca caatcgctca agacgtgtaa tgcacttccg gatgaaactc agggtaag 58
<210> 33
   <211> 19
   <212> DNA
   <213> Artificial sequence
<220>
   <223> primer
<400> 33
   tgccaaatac gggctactg 19
<210> 34
   <211> 18
   <212> DNA
   <213> Artificial sequence
<220>
   <223> primer
<400> 34
   tccgggctca attcactc 18
<210> 35
   <211> 53
   <212> DNA
   <213> Artificial sequence
<220>
   <223> primer
<400> 35
   gagaatagga acttcggaat aggaactaag gaggagctgg ttgcgtttac gtc 53
<210> 36
   <211> 30
   <212> DNA
   <213> Artificial sequence
<220>
   <223> primer
<400> 36
   gcattacacg tcttgagcga ttgtgtaggc 30
<210> 37
   <211> 34
   <212> DNA
   <213> Artificial sequence
<220>
   <223> primer
<400> 37
   cctccttagt tcctattccg aagttcctat tctc 34
<210> 38
   <211> 35
   <212> DNA
   <213> Artificial sequence
<220>
   <223> primer
<400> 38
   gaacctgcag gccctgacac gaggtagatt atgtc 35
<210> 39
   <211> 55
   <212> DNA
   <213> Artificial sequence
<220>
   <223> primer
<400> 39
   ctttcggcta gaagagcgag atgcagataa aaaaattaaa ggcaattatt ctccg 55
<210> 40
   <211> 879
   <212> DNA
   <213> Escherichia coli
<220>
   <221> CDS
   <222> (1)..(876)
<400> 40
   atg ttc acg gga agt att gtc gcg att gtt act ccg atg gat gaa aaa        48
   Met Phe Thr Gly Ser Ile Val Ala Ile Val Thr Pro Met Asp Glu Lys
   1               5                   10                  15
   ggt aat gtc tgt cgg gct agc ttg aaa aaa ctg att gat tat cat gtc        96
   Gly Asn Val Cys Arg Ala Ser Leu Lys Lys Leu Ile Asp Tyr His Val
               20                  25                  30
   gcc agc ggt act tcg gcg atc gtt tct gtt ggc acc act ggc gag tcc       144
   Ala Ser Gly Thr Ser Ala Ile Val Ser Val Gly Thr Thr Gly Glu Ser
           35                  40                  45
   gct acc tta aat cat gac gaa cat gct gat gtg gtg atg atg acg ctg       192
   Ala Thr Leu Asn His Asp Glu His Ala Asp Val Val Met Met Thr Leu
       50                  55                  60
   gat ctg gct gat ggg cgc att ccg gta att gcc ggg acc ggc gct aac       240
   Asp Leu Ala Asp Gly Arg Ile Pro Val Ile Ala Gly Thr Gly Ala Asn
   65                  70                  75                  80
   gct act gcg gaa gcc att agc ctg acg cag cgc ttc aat gac agt ggt       288
   Ala Thr Ala Glu Ala Ile Ser Leu Thr Gln Arg Phe Asn Asp Ser Gly
                   85                  90                  95
   atc gtc ggc tgc ctg acg gta acc cct tac tac aat cgt ccg tcg caa       336
   Ile Val Gly Cys Leu Thr Val Thr Pro Tyr Tyr Asn Arg Pro Ser Gln
               100                 105                 110
   gaa ggt ttg tat cag cat ttc aaa gcc atc gct gag cat act gac ctg       384
   Glu Gly Leu Tyr Gln His Phe Lys Ala Ile Ala Glu His Thr Asp Leu
           115                 120                 125
   ccg caa att ctg tat aat gtg ccg tcc cgt act ggc tgc gat ctg ctc       432
   Pro Gln Ile Leu Tyr Asn Val Pro Ser Arg Thr Gly Cys Asp Leu Leu
       130                 135                 140
   ccg gaa acg gtg ggc cgt ctg gcg aaa gta aaa aat att atc gga atc       480
   Pro Glu Thr Val Gly Arg Leu Ala Lys Val Lys Asn Ile Ile Gly Ile
   145                 150                 155                 160
   aaa gag gca aca ggg aac tta acg cgt gta aac cag atc aaa gag ctg       528
   Lys Glu Ala Thr Gly Asn Leu Thr Arg Val Asn Gln Ile Lys Glu Leu
                   165                 170                 175
   gtt tca gat gat ttt gtt ctg ctg agc ggc gat gat gcg agc gcg ctg       576
   Val Ser Asp Asp Phe Val Leu Leu Ser Gly Asp Asp Ala Ser Ala Leu
               180                 185                 190
   gac ttc atg caa ttg ggc ggt cat ggg gtt att tcc gtt acg gct aac       624
   Asp Phe Met Gln Leu Gly Gly His Gly Val Ile Ser Val Thr Ala Asn
           195                 200                 205
   gtc gca gcg cgt gat atg gcc cag atg tgc aaa ctg gca gca gaa ggg       672
   Val Ala Ala Arg Asp Met Ala Gln Met Cys Lys Leu Ala Ala Glu Gly
       210                 215                 220
   cat ttt gcc gag gca cgc gtt att aat cag cgt ctg atg cca tta cac       720
   His Phe Ala Glu Ala Arg Val Ile Asn Gln Arg Leu Met Pro Leu His
   225                 230                 235                 240
   aac aaa cta ttt gtc gaa ccc aat cca atc ccg gtg aaa tgg gca tgt       768
   Asn Lys Leu Phe Val Glu Pro Asn Pro Ile Pro Val Lys Trp Ala Cys
                   245                 250                 255
   aag gaa ctg ggt ctt gtg gcg acc gat acg ctg cgc ctg cca atg aca       816
   Lys Glu Leu Gly Leu Val Ala Thr Asp Thr Leu Arg Leu Pro Met Thr
               260                 265                 270
   cca atc acc gac agt ggt cgt gag acg gtc aga gcg gcg ctt aag cat       864
   Pro Ile Thr Asp Ser Gly Arg Glu Thr Val Arg Ala Ala Leu Lys His
           275                 280                 285
   gcc ggt ttg ctg taa                                                   879
   Ala Gly Leu Leu
       290
<210> 41
   <211> 292
   <212> PRT
   <213> Escherichia coli
<400> 41
   Met Phe Thr Gly Ser Ile Val Ala Ile Val Thr Pro Met Asp Glu Lys
   1               5                   10                  15
   Gly Asn Val Cys Arg Ala Ser Leu Lys Lys Leu Ile Asp Tyr His Val
               20                  25                  30
   Ala Ser Gly Thr Ser Ala Ile Val Ser Val Gly Thr Thr Gly Glu Ser
           35                  40                  45
   Ala Thr Leu Asn His Asp Glu His Ala Asp Val Val Met Met Thr Leu
       50                  55                  60
   Asp Leu Ala Asp Gly Arg Ile Pro Val Ile Ala Gly Thr Gly Ala Asn
   65                  70                  75                  80
   Ala Thr Ala Glu Ala Ile Ser Leu Thr Gln Arg Phe Asn Asp Ser Gly
                   85                  90                  95
   Ile Val Gly Cys Leu Thr Val Thr Pro Tyr Tyr Asn Arg Pro Ser Gln
               100                 105                 110
   Glu Gly Leu Tyr Gln His Phe Lys Ala Ile Ala Glu His Thr Asp Leu
           115                 120                 125
   Pro Gln Ile Leu Tyr Asn Val Pro Ser Arg Thr Gly Cys Asp Leu Leu
       130                 135                 140
   Pro Glu Thr Val Gly Arg Leu Ala Lys Val Lys Asn Ile Ile Gly Ile
   145                 150                 155                 160
   Lys Glu Ala Thr Gly Asn Leu Thr Arg Val Asn Gln Ile Lys Glu Leu
                   165                 170                 175
   Val Ser Asp Asp Phe Val Leu Leu Ser Gly Asp Asp Ala Ser Ala Leu
               180                 185                 190
   Asp Phe Met Gln Leu Gly Gly His Gly Val Ile Ser Val Thr Ala Asn
           195                 200                 205
   Val Ala Ala Arg Asp Met Ala Gln Met Cys Lys Leu Ala Ala Glu Gly
       210                 215                 220
   His Phe Ala Glu Ala Arg Val Ile Asn Gln Arg Leu Met Pro Leu His
   225                 230                 235                 240
   Asn Lys Leu Phe Val Glu Pro Asn Pro Ile Pro Val Lys Trp Ala Cys
                   245                 250                 255
   Lys Glu Leu Gly Leu Val Ala Thr Asp Thr Leu Arg Leu Pro Met Thr
               260                 265                 270
   Pro Ile Thr Asp Ser Gly Arg Glu Thr Val Arg Ala Ala Leu Lys His
           275                 280                 285
   Ala Gly Leu Leu
       290
<210> 42
   <211> 822
   <212> DNA
   <213> Escherichia coli
<220>
   <221> CDS
   <222> (1)..(822)
   <223>
<400> 42
   atg cat gat gca aac atc cgc gtt gcc atc gcg gga gcc ggg ggg cgt      48
   Met His Asp Ala Asn Ile Arg Val Ala Ile Ala Gly Ala Gly Gly Arg
   1               5                   10                  15
   atg ggc cgc cag ttg att cag gcg gcg ctg gca tta gag ggc gtg cag      96
   Met Gly Arg Gln Leu Ile Gln Ala Ala Leu Ala Leu Glu Gly Val Gln
               20                  25                  30
   ttg ggc gct gcg ctg gag cgt gaa gga tct tct tta ctg ggc agc gac      144
   Leu Gly Ala Ala Leu Glu Arg Glu Gly Ser Ser Leu Leu Gly Ser Asp
           35                  40                  45
   gcc ggt gag ctg gcc gga gcc ggg aaa aca ggc gtt acc gtg caa agc      192
   Ala Gly Glu Leu Ala Gly Ala Gly Lys Thr Gly Val Thr Val Gln Ser
       50                  55                  60
   agc ctc gat gcg gta aaa gat gat ttt gat gtg ttt atc gat ttt acc      240
   Ser Leu Asp Ala Val Lys Asp Asp Phe Asp Val Phe Ile Asp Phe Thr
   65                  70                  75                  80
   cgt ccg gaa ggt acg ctg aac cat ctc gct ttt tgt cgc cag cat ggc      288
   Arg Pro Glu Gly Thr Leu Asn His Leu Ala Phe Cys Arg Gln His Gly
                   85                  90                  95
   aaa ggg atg gtg atc ggc act acg ggg ttt gac gaa gcc ggt aaa caa      336
   Lys Gly Met Val Ile Gly Thr Thr Gly Phe Asp Glu Ala Gly Lys Gln
               100                 105                 110
   gca att cgt gac gcc gct gcc gat att gcg att gtc ttt gct gcc aat      384
   Ala Ile Arg Asp Ala Ala Ala Asp Ile Ala Ile Val Phe Ala Ala Asn
           115                 120                 125
   ttt agc gtt ggc gtt aac gtc atg ctt aag ctg ctg gag aaa gca gcc      432
   Phe Ser Val Gly Val Asn Val Met Leu Lys Leu Leu Glu Lys Ala Ala
       130                 135                 140
   aaa gtg atg ggt gac tac acc gat atc gaa att att gaa gca cat cat      480
   Lys Val Met Gly Asp Tyr Thr Asp Ile Glu Ile Ile Glu Ala His His
   145                 150                 155                 160
   aga cat aaa gtt gat gcg ccg tca ggc acc gca ctg gca atg gga gag      528
   Arg His Lys Val Asp Ala Pro Ser Gly Thr Ala Leu Ala Met Gly Glu
                   165                 170                 175
   gcg atc gcc cac gcc ctt gat aaa gat ctg aaa gat tgc gcg gtc tac      576
   Ala Ile Ala His Ala Leu Asp Lys Asp Leu Lys Asp Cys Ala Val Tyr
               180                 185                 190
   agt cgt gaa ggc cac acc ggt gaa cgt gtg cct ggc acc att ggt ttt      624
   Ser Arg Glu Gly His Thr Gly Glu Arg Val Pro Gly Thr Ile Gly Phe
           195                 200                 205
   gcc acc gtg cgt gca ggt gac atc gtt ggt gaa cat acc gcg atg ttt      672
   Ala Thr Val Arg Ala Gly Asp Ile Val Gly Glu His Thr Ala Met Phe
       210                 215                 220
   gcc gat att ggc gag cgt ctg gag atc acc cat aag gcg tcc agc cgt      720
   Ala Asp Ile Gly Glu Arg Leu Glu Ile Thr His Lys Ala Ser Ser Arg
   225                 230                 235                 240
   atg aca ttt gct aac ggc gcg gta aga tcg gct ttg tgg ttg agt ggt      768
   Met Thr Phe Ala Asn Gly Ala Val Arg Ser Ala Leu Trp Leu Ser Gly
                   245                 250                 255
   aag gaa agc ggt ctt ttt gat atg cga gat gta ctt gat ctc aat aat      816
   Lys Glu Ser Gly Leu Phe Asp Met Arg Asp Val Leu Asp Leu Asn Asn
               260                 265                 270
   ttg taa                                                              822
   Leu
<210> 43
   <211> 273
   <212> PRT
   <213> Escherichia coli
<400> 43
   Met His Asp Ala Asn Ile Arg Val Ala Ile Ala Gly Ala Gly Gly Arg
   1               5                   10                  15
   Met Gly Arg Gln Leu Ile Gln Ala Ala Leu Ala Leu Glu Gly Val Gln
               20                  25                  30
   Leu Gly Ala Ala Leu Glu Arg Glu Gly Ser Ser Leu Leu Gly Ser Asp
           35                  40                  45
   Ala Gly Glu Leu Ala Gly Ala Gly Lys Thr Gly Val Thr Val Gln Ser
       50                  55                  60
   Ser Leu Asp Ala Val Lys Asp Asp Phe Asp Val Phe Ile Asp Phe Thr
   65                  70                  75                  80
   Arg Pro Glu Gly Thr Leu Asn His Leu Ala Phe Cys Arg Gln His Gly
                   85                  90                  95
   Lys Gly Met Val Ile Gly Thr Thr Gly Phe Asp Glu Ala Gly Lys Gln
               100                 105                 110
   Ala Ile Arg Asp Ala Ala Ala Asp Ile Ala Ile Val Phe Ala Ala Asn
           115                 120                 125
   Phe Ser Val Gly Val Asn Val Met Leu Lys Leu Leu Glu Lys Ala Ala
       130                 135                 140
   Lys Val Met Gly Asp Tyr Thr Asp Ile Glu Ile Ile Glu Ala His His
   145                 150                 155                 160
   Arg His Lys Val Asp Ala Pro Ser Gly Thr Ala Leu Ala Met Gly Glu
                   165                 170                 175
   Ala Ile Ala His Ala Leu Asp Lys Asp Leu Lys Asp Cys Ala Val Tyr
               180                 185                 190
   Ser Arg Glu Gly His Thr Gly Glu Arg Val Pro Gly Thr Ile Gly Phe
           195                 200                 205
   Ala Thr Val Arg Ala Gly Asp Ile Val Gly Glu His Thr Ala Met Phe
       210                 215                 220
   Ala Asp Ile Gly Glu Arg Leu Glu Ile Thr His Lys Ala Ser Ser Arg
   225                 230                 235                 240
   Met Thr Phe Ala Asn Gly Ala Val Arg Ser Ala Leu Trp Leu Ser Gly
                   245                 250                 255
   Lys Glu Ser Gly Leu Phe Asp Met Arg Asp Val Leu Asp Leu Asn Asn
               260                 265                 270
   Leu
<210> 44
   <211> 1104
   <212> DNA
   <213> Escherichia coli
<220>
   <221> CDS
   <222> (1)..(1104)
   <223>
<400> 44
   atg aaa aat gtt ggt ttt atc ggc tgg cgc ggt atg gtc ggc tcc gtt      48
   Met Lys Asn Val Gly Phe Ile Gly Trp Arg Gly Met Val Gly Ser Val
   1               5                   10                  15
   ctc atg caa cgc atg gtt gaa gag cgc gac ttc gac gcc att cgc cct      96
   Leu Met Gln Arg Met Val Glu Glu Arg Asp Phe Asp Ala Ile Arg Pro
               20                  25                  30
   gtc ttc ttt tct act tct cag ctt ggc cag gct gcg ccg tct ttt ggc     144
   Val Phe Phe Ser Thr Ser Gln Leu Gly Gln Ala Ala Pro Ser Phe Gly
           35                  40                  45
   gga acc act ggc aca ctt cag gat gcc ttt gat ctg gag gcg cta aag     192
   Gly Thr Thr Gly Thr Leu Gln Asp Ala Phe Asp Leu Glu Ala Leu Lys
       50                  55                  60
   gcc ctc gat atc att gtg acc tgt cag ggc ggc gat tat acc aac gaa     240
   Ala Leu Asp Ile Ile Val Thr Cys Gln Gly Gly Asp Tyr Thr Asn Glu
   65                  70                  75                  80
   atc tat cca aag ctt cgt gaa agc gga tgg caa ggt tac tgg att gac     288
   Ile Tyr Pro Lys Leu Arg Glu Ser Gly Trp Gln Gly Tyr Trp Ile Asp
                   85                  90                  95
   gca gca tcg tct ctg cgc atg aaa gat gac gcc atc atc att ctt gac     336
   Ala Ala Ser Ser Leu Arg Met Lys Asp Asp Ala Ile Ile Ile Leu Asp
               100                 105                 110
   ccc gtc aat cag gac gtc att acc gac gga tta aat aat ggc atc agg     384
   Pro Val Asn Gln Asp Val Ile Thr Asp Gly Leu Asn Asn Gly Ile Arg
           115                 120                 125
   act ttt gtt ggc ggt aac tgt acc gta agc ctg atg ttg atg tcg ttg     432
   Thr Phe Val Gly Gly Asn Cys Thr Val Ser Leu Met Leu Met Ser Leu
       130                 135                 140
   ggt ggt tta ttc gcc aat gat ctt gtt gat tgg gtg tcc gtt gca acc     480
   Gly Gly Leu Phe Ala Asn Asp Leu Val Asp Trp Val Ser Val Ala Thr
   145                 150                 155                 160
   tac cag gcc gct tcc ggc ggt ggt gcg cga cat atg cgt gag tta tta     528
   Tyr Gln Ala Ala Ser Gly Gly Gly Ala Arg His Met Arg Glu Leu Leu
                   165                 170                 175
   acc cag atg ggc cat ctg tat ggc cat gtg gca gat gaa ctc gcg acc     576
   Thr Gln Met Gly His Leu Tyr Gly His Val Ala Asp Glu Leu Ala Thr
               180                 185                 190
   ccg tcc tct gct att ctc gat atc gaa cgc aaa gtc aca acc tta acc     624
   Pro Ser Ser Ala Ile Leu Asp Ile Glu Arg Lys Val Thr Thr Leu Thr
           195                 200                 205
   cgt agc ggt gag ctg ccg gtg gat aac ttt ggc gtg ccg ctg gcg ggt     672
   Arg Ser Gly Glu Leu Pro Val Asp Asn Phe Gly Val Pro Leu Ala Gly
       210                 215                 220
   agc ctg att ccg tgg atc gac aaa cag ctc gat aac ggt cag agc cgc     720
   Ser Leu Ile Pro Trp Ile Asp Lys Gln Leu Asp Asn Gly Gln Ser Arg
   225                 230                 235                 240
   gaa gag tgg aaa ggg cag gcg gaa acc aac aag atc ctc aac aca tct     768
   Glu Glu Trp Lys Gly Gln Ala Glu Thr Asn Lys Ile Leu Asn Thr Ser
                   245                 250                 255
   tcc gta att ccg gta gat ggt tta tgt gtg cgt gtc ggg gca ttg cgc     816
   Ser Val Ile Pro Val Asp Gly Leu Cys Val Arg Val Gly Ala Leu Arg
               260                 265                 270
   tgc cac agc cag gca ttc act att aaa ttg aaa aaa gat gtg tct att     864
   Cys His Ser Gln Ala Phe Thr Ile Lys Leu Lys Lys Asp Val Ser Ile
           275                 280                 285
   ccg acc gtg gaa gaa ctg ctg gct gcg cac aat ccg tgg gcg aaa gtc     912
   Pro Thr Val Glu Glu Leu Leu Ala Ala His Asn Pro Trp Ala Lys Val
       290                 295                 300
   gtt ccg aac gat cgg gaa atc act atg cgt gag cta acc cca gct gcc     960
   Val Pro Asn Asp Arg Glu Ile Thr Met Arg Glu Leu Thr Pro Ala Ala
   305                 310                 315                 320
   gtt acc ggc acg ctg acc acg ccg gta ggc cgc ctg cgt aag ctg aat    1008
   Val Thr Gly Thr Leu Thr Thr Pro Val Gly Arg Leu Arg Lys Leu Asn
                   325                 330                 335
   atg gga cca gag ttc ctg tca gcc ttt acc gtg ggc gac cag ctg ctg    1056
   Met Gly Pro Glu Phe Leu Ser Ala Phe Thr Val Gly Asp Gln Leu Leu
               340                 345                 350
   tgg ggg gcc gcg gag ccg ctg cgt cgg atg ctt cgt caa ctg gcg taa    1104
   Trp Gly Ala Ala Glu Pro Leu Arg Arg Met Leu Arg Gln Leu Ala
           355                 360                 365
<210> 45
   <211> 367
   <212> PRT
   <213> Escherichia coli
<400> 45
   Met Lys Asn Val Gly Phe Ile Gly Trp Arg Gly Met Val Gly Ser Val
   1               5                   10                  15
   Leu Met Gln Arg Met Val Glu Glu Arg Asp Phe Asp Ala Ile Arg Pro
               20                  25                  30
   Val Phe Phe Ser Thr Ser Gln Leu Gly Gln Ala Ala Pro Ser Phe Gly
           35                  40                  45
   Gly Thr Thr Gly Thr Leu Gln Asp Ala Phe Asp Leu Glu Ala Leu Lys
       50                  55                  60
   Ala Leu Asp Ile Ile Val Thr Cys Gln Gly Gly Asp Tyr Thr Asn Glu
   65                  70                  75                  80
   Ile Tyr Pro Lys Leu Arg Glu Ser Gly Trp Gln Gly Tyr Trp Ile Asp
                   85                  90                  95
   Ala Ala Ser Ser Leu Arg Met Lys Asp Asp Ala Ile Ile Ile Leu Asp
               100                 105                 110
   Pro Val Asn Gln Asp Val Ile Thr Asp Gly Leu Asn Asn Gly Ile Arg
           115                 120                 125
   Thr Phe Val Gly Gly Asn Cys Thr Val Ser Leu Met Leu Met Ser Leu
       130                 135                 140
   Gly Gly Leu Phe Ala Asn Asp Leu Val Asp Trp Val Ser Val Ala Thr
   145                 150                 155                 160
   Tyr Gln Ala Ala Ser Gly Gly Gly Ala Arg His Met Arg Glu Leu Leu
                   165                 170                 175
   Thr Gln Met Gly His Leu Tyr Gly His Val Ala Asp Glu Leu Ala Thr
               180                 185                 190
   Pro Ser Ser Ala Ile Leu Asp Ile Glu Arg Lys Val Thr Thr Leu Thr
           195                 200                 205
   Arg Ser Gly Glu Leu Pro Val Asp Asn Phe Gly Val Pro Leu Ala Gly
       210                 215                 220
   Ser Leu Ile Pro Trp Ile Asp Lys Gln Leu Asp Asn Gly Gln Ser Arg
   225                 230                 235                 240
   Glu Glu Trp Lys Gly Gln Ala Glu Thr Asn Lys Ile Leu Asn Thr Ser
                   245                 250                 255
   Ser Val Ile Pro Val Asp Gly Leu Cys Val Arg Val Gly Ala Leu Arg
               260                 265                 270
   Cys His Ser Gln Ala Phe Thr Ile Lys Leu Lys Lys Asp Val Ser Ile
           275                 280                 285
   Pro Thr Val Glu Glu Leu Leu Ala Ala His Asn Pro Trp Ala Lys Val
       290                 295                 300
   Val Pro Asn Asp Arg Glu Ile Thr Met Arg Glu Leu Thr Pro Ala Ala
   305                 310                 315                 320
   Val Thr Gly Thr Leu Thr Thr Pro Val Gly Arg Leu Arg Lys Leu Asn
                   325                 330                 335
   Met Gly Pro Glu Phe Leu Ser Ala Phe Thr Val Gly Asp Gln Leu Leu
               340                 345                 350
   Trp Gly Ala Ala Glu Pro Leu Arg Arg Met Leu Arg Gln Leu Ala
           355                 360                 365
<210> 46
   <211> 1350
   <212> DNA
   <213> Escherichia coli
<220>
   <221> CDS
   <222> (1)..(1350)
   <223>
<400> 46
   atg tct gaa att gtt gtc tcc aaa ttt ggc ggt acc agc gta gct gat 48
   Met Ser Glu Ile Val Val Ser Lys Phe Gly Gly Thr Ser Val Ala Asp
   1 5 10 15
   ttt gac gcc atg aac cgc agc gct gat att gtg ctt tct gat gcc aac 96
   Phe Asp Ala Met Asn Arg Ser Ala Asp Ile Val Leu Ser Asp Ala Asn
   20 25 30
   gtg cgt tta gtt gtc ctc tcg gct tct gct ggt atc act aat ctg ctg 144
   Val Arg Leu Val Val Leu Ser Ala Ser Ala Gly Ile Thr Asn Leu Leu
   35 40 45
   gtc gct tta gct gaa gga ctg gaa cct ggc gag cga ttc gaa aaa ctc 192
   Val Ala Leu Ala Glu Gly Leu Glu Pro Gly Glu Arg Phe Glu Lys Leu
   50 55 60
   gac gct atc cgc aac atc cag ttt gcc att ctg gaa cgt ctg cgt tac 240
   Asp Ala Ile Arg Asn Ile Gln Phe Ala Ile Leu Glu Arg Leu Arg Tyr
   65 70 75 80
   ccg aac gtt atc cgt gaa gag att gaa cgt ctg ctg gag aac att act 288
   Pro Asn Val Ile Arg Glu Glu Ile Glu Arg Leu Leu Glu Asn Ile Thr
   85 90 95
   gtt ctg gca gaa gcg gcg gcg ctg gca acg tct ccg gcg ctg aca gat 336
   Val Leu Ala Glu Ala Ala Ala Leu Ala Thr Ser Pro Ala Leu Thr Asp
   100 105 110
   gag ctg gtc agc cac ggc gag ctg atg tcg acc ctg ctg ttt gtt gag 384
   Glu Leu Val Ser His Gly Glu Leu Met Ser Thr Leu Leu Phe Val Glu
   115 120 125
   atc ctg cgc gaa cgc gat gtt cag gca cag tgg ttt gat gta cgt aaa 432
   Ile Leu Arg Glu Arg Asp Val Gln Ala Gln Trp Phe Asp Val Arg Lys
   130 135 140
   gtg atg cgt acc aac gac cga ttt ggt cgt gca gag cca gat ata gcc 480
   Val Met Arg Thr Asn Asp Arg Phe Gly Arg Ala Glu Pro Asp Ile Ala
   145 150 155 160
   gcg ctg gcg gaa ctg gcc gcg ctg cag ctg ctc cca cgt ctc aat gaa 528
   Ala Leu Ala Glu Leu Ala Ala Leu Gln Leu Leu Pro Arg Leu Asn Glu
   165 170 175
   ggc tta gtg atc acc cag gga ttt atc ggt agc gaa aat aaa ggt cgt 576
   Gly Leu Val Ile Thr Gln Gly Phe Ile Gly Ser Glu Asn Lys Gly Arg
   180 185 190
   aca acg acg ctt ggc cgt gga ggc agc gat tat acg gca gcc ttg ctg 624
   Thr Thr Thr Leu Gly Arg Gly Gly Ser Asp Tyr Thr Ala Ala Leu Leu
   195 200 205
   gcg gag gct tta cac gca tct cgt gtt gat atc tgg acc gac gtc ccg 672
   Ala Glu Ala Leu His Ala Ser Arg Val Asp Ile Trp Thr Asp Val Pro
   210 215 220
   ggc atc tac acc acc gat cca cgc gta gtt tcc gca gca aaa cgc att 720
   Gly Ile Tyr Thr Thr Asp Pro Arg Val Val Ser Ala Ala Lys Arg Ile
   225 230 235 240
   gat gaa atc gcg ttt gcc gaa gcg gca gag atg gca act ttt ggt gca 768
   Asp Glu Ile Ala Phe Ala Glu Ala Ala Glu Met Ala Thr Phe Gly Ala
   245 250 255
   aaa gta ctg cat ccg gca acg ttg cta ccc gca gta cgc agc gat atc 816
   Lys Val Leu His Pro Ala Thr Leu Leu Pro Ala Val Arg Ser Asp Ile
   260 265 270
   ccg gtc ttt gtc ggc tcc agc aaa gac cca cgc gca ggt ggt acg ctg 864
   Pro Val Phe Val Gly Ser Ser Lys Asp Pro Arg Ala Gly Gly Thr Leu
   275 280 285
   gtg tgc aat aaa act gaa aat ccg ccg ctg ttc cgc gct ctg gcg ctt 912
   Val Cys Asn Lys Thr Glu Asn Pro Pro Leu Phe Arg Ala Leu Ala Leu
   290 295 300
   cgt cgc aat cag act ctg ctc act ttg cac agc ctg aat atg ctg cat 960
   Arg Arg Asn Gln Thr Leu Leu Thr Leu His Ser Leu Asn Met Leu His
   305 310 315 320
   tct cgc ggt ttc ctc gcg gaa gtt ttc ggc atc ctc gcg cgg cat aat 1008
   Ser Arg Gly Phe Leu Ala Glu Val Phe Gly Ile Leu Ala Arg His Asn
   325 330 335
   att tcg gta gac tta atc acc acg tca gaa gtg agc gtg gca tta acc 1056
   Ile Ser Val Asp Leu Ile Thr Thr Ser Glu Val Ser Val Ala Leu Thr
                   340                 345                 350
   ctt gat acc acc ggt tca acc tcc act ggc gat acg ttg ctg acg caa      1104
   Leu Asp Thr Thr Gly Ser Thr Ser Thr Gly Asp Thr Leu Leu Thr Gln
           355                 360                 365
   tct ctg ctg atg gag ctt tcc gca ctg tgt cgg gtg gag gtg gaa gaa      1152
   Ser Leu Leu Met Glu Leu Ser Ala Leu Cys Arg Val Glu Val Glu Glu
       370                 375                 380
   ggt ctg gcg ctg gtc gcg ttg att ggc aat gac ctg tca aaa gcc tgc      1200
   Gly Leu Ala Leu Val Ala Leu Ile Gly Asn Asp Leu Ser Lys Ala Cys
   385                 390                 395                 400
   ggc gtt ggc aaa gag gta ttc ggc gta ctg gaa ccg ttc aac att cgc      1248
   Gly Val Gly Lys Glu Val Phe Gly Val Leu Glu Pro Phe Asn Ile Arg
                   405                 410                 415
   atg att tgt tat ggc gca tcc agc cat aac ctg tgc ttc ctg gtg ccc      1296
   Met Ile Cys Tyr Gly Ala Ser Ser His Asn Leu Cys Phe Leu Val Pro
               420                 425                 430
   ggc gaa gat gcc gag cag gtg gtg caa aaa ctg cat agt aat ttg ttt      1344
   Gly Glu Asp Ala Glu Gln Val Val Gln Lys Leu His Ser Asn Leu Phe
           435                 440                 445
   gag taa                                                              1350
   Glu
<210> 47
   <211> 449
   <212> PRT
   <213> Escherichia coli
<400> 47
   Met Ser Glu Ile Val Val Ser Lys Phe Gly Gly Thr Ser Val Ala Asp
   1               5                   10                  15
   Phe Asp Ala Met Asn Arg Ser Ala Asp Ile Val Leu Ser Asp Ala Asn
               20                  25                  30
   Val Arg Leu Val Val Leu Ser Ala Ser Ala Gly Ile Thr Asn Leu Leu
           35                  40                  45
   Val Ala Leu Ala Glu Gly Leu Glu Pro Gly Glu Arg Phe Glu Lys Leu
       50                  55                  60
   Asp Ala Ile Arg Asn Ile Gln Phe Ala Ile Leu Glu Arg Leu Arg Tyr
   65                  70                  75                  80
   Pro Asn Val Ile Arg Glu Glu Ile Glu Arg Leu Leu Glu Asn Ile Thr
                   85                  90                  95
   Val Leu Ala Glu Ala Ala Ala Leu Ala Thr Ser Pro Ala Leu Thr Asp
               100                 105                 110
   Glu Leu Val Ser His Gly Glu Leu Met Ser Thr Leu Leu Phe Val Glu
           115                 120                 125
   Ile Leu Arg Glu Arg Asp Val Gln Ala Gln Trp Phe Asp Val Arg Lys
       130                 135                 140
   Val Met Arg Thr Asn Asp Arg Phe Gly Arg Ala Glu Pro Asp Ile Ala
   145                 150                 155                 160
   Ala Leu Ala Glu Leu Ala Ala Leu Gln Leu Leu Pro Arg Leu Asn Glu
                   165                 170                 175
   Gly Leu Val Ile Thr Gln Gly Phe Ile Gly Ser Glu Asn Lys Gly Arg
               180                 185                 190
   Thr Thr Thr Leu Gly Arg Gly Gly Ser Asp Tyr Thr Ala Ala Leu Leu
           195                 200                 205
   Ala Glu Ala Leu His Ala Ser Arg Val Asp Ile Trp Thr Asp Val Pro
       210                 215                 220
   Gly Ile Tyr Thr Thr Asp Pro Arg Val Val Ser Ala Ala Lys Arg Ile
   225                 230                 235                 240
   Asp Glu Ile Ala Phe Ala Glu Ala Ala Glu Met Ala Thr Phe Gly Ala
                   245                 250                 255
   Lys Val Leu His Pro Ala Thr Leu Leu Pro Ala Val Arg Ser Asp Ile
               260                 265                 270
   Pro Val Phe Val Gly Ser Ser Lys Asp Pro Arg Ala Gly Gly Thr Leu
           275                 280                 285
   Val Cys Asn Lys Thr Glu Asn Pro Pro Leu Phe Arg Ala Leu Ala Leu
       290                 295                 300
   Arg Arg Asn Gln Thr Leu Leu Thr Leu His Ser Leu Asn Met Leu His
   305                 310                 315                 320
   Ser Arg Gly Phe Leu Ala Glu Val Phe Gly Ile Leu Ala Arg His Asn
                   325                 330                 335
   Ile Ser Val Asp Leu Ile Thr Thr Ser Glu Val Ser Val Ala Leu Thr
               340                 345                 350
   Leu Asp Thr Thr Gly Ser Thr Ser Thr Gly Asp Thr Leu Leu Thr Gln
           355                 360                 365
   Ser Leu Leu Met Glu Leu Ser Ala Leu Cys Arg Val Glu Val Glu Glu
       370                 375                 380
   Gly Leu Ala Leu Val Ala Leu Ile Gly Asn Asp Leu Ser Lys Ala Cys
   385                 390                 395                 400
   Gly Val Gly Lys Glu Val Phe Gly Val Leu Glu Pro Phe Asn Ile Arg
                   405                 410                 415
   Met Ile Cys Tyr Gly Ala Ser Ser His Asn Leu Cys Phe Leu Val Pro
               420                 425                 430
   Gly Glu Asp Ala Glu Gln Val Val Gln Lys Leu His Ser Asn Leu Phe
           435                 440                 445
   Glu
<210> 48
   <211> 1245
   <212> DNA
   <213> Methylophilus methylotrophus
<220>
   <221> CDS
   <222> (1)..(1245)
   <223>
<400> 48
   gtg acc gct ttt tca atc caa caa ggc cta cta cat gcc gag aat gta      48
   Val Thr Ala Phe Ser Ile Gln Gln Gly Leu Leu His Ala Glu Asn Val
   1               5                   10                  15
   gcc ctg cgt gac att gca caa acg cat caa acg ccc act tac gtc tat      96
   Ala Leu Arg Asp Ile Ala Gln Thr His Gln Thr Pro Thr Tyr Val Tyr
               20                  25                  30
   tca cgt gcc gcc ttg acg act gct ttc gag cgt ttt cag gca ggc ctg     144
   Ser Arg Ala Ala Leu Thr Thr Ala Phe Glu Arg Phe Gln Ala Gly Leu
           35                  40                  45
   act gga cat gac cat ttg atc tgc ttt gct gtc aaa gcc aac cca agc     192
   Thr Gly His Asp His Leu Ile Cys Phe Ala Val Lys Ala Asn Pro Ser
       50                  55                  60
   ctg gcc att ctc aac ctg ttt gcg cga atg gga gcg ggc ttt gat att     240
   Leu Ala Ile Leu Asn Leu Phe Ala Arg Met Gly Ala Gly Phe Asp Ile
   65                  70                  75                  80
   gtg tcc ggt ggt gag ctg gca cgc gtc ttg gcc gca ggt ggc gac ccg     288
   Val Ser Gly Gly Glu Leu Ala Arg Val Leu Ala Ala Gly Gly Asp Pro
                   85                  90                  95
   aaa aaa gtg gtg ttt tct ggt gtg ggc aaa tcc cat gcg gaa atc aaa     336
   Lys Lys Val Val Phe Ser Gly Val Gly Lys Ser His Ala Glu Ile Lys
               100                 105                 110
   gcc gcg ctt gaa gcg ggc att ctt tgc ttc aac gtg gaa tca gtg aat     384
   Ala Ala Leu Glu Ala Gly Ile Leu Cys Phe Asn Val Glu Ser Val Asn
           115                 120                 125
   gag cta gac cgc atc cag cag gtg gcg gcc agc ctg ggc aaa aaa gcg     432
   Glu Leu Asp Arg Ile Gln Gln Val Ala Ala Ser Leu Gly Lys Lys Ala
       130                 135                 140
   cct att tcc ctg cgc gtg aac ccc aat gtg gat gcc aaa acc cat ccc     480
   Pro Ile Ser Leu Arg Val Asn Pro Asn Val Asp Ala Lys Thr His Pro
   145                 150                 155                 160
   tat att tcc acc ggc ctc aaa aac aat aaa ttt ggt gtg gca ttt gaa     528
   Tyr Ile Ser Thr Gly Leu Lys Asn Asn Lys Phe Gly Val Ala Phe Glu
                   165                 170                 175
   gat gcc ttg ggc ctc tat gaa aaa gcg gcg caa ctg cca aac atc gag     576
   Asp Ala Leu Gly Leu Tyr Glu Lys Ala Ala Gln Leu Pro Asn Ile Glu
               180                 185                 190
   gta cac ggc gta gat tgc cat atc ggc tcg caa atc act gag ctg tca     624
   Val His Gly Val Asp Cys His Ile Gly Ser Gln Ile Thr Glu Leu Ser
           195                 200                 205
   cct ttc ctc gat gcc ttg gat aaa gta ttg ggc ctg gta gat gca ttg     672
   Pro Phe Leu Asp Ala Leu Asp Lys Val Leu Gly Leu Val Asp Ala Leu
       210                 215                 220
   gcc gcc aaa ggc att cat atc cag cat ata gac gtt ggc ggc ggt gtc     720
   Ala Ala Lys Gly Ile His Ile Gln His Ile Asp Val Gly Gly Gly Val
   225                 230                 235                 240
   ggt att act tac agc gac gaa acg cca cca gac ttt gca gcc tac act     768
   Gly Ile Thr Tyr Ser Asp Glu Thr Pro Pro Asp Phe Ala Ala Tyr Thr
                   245                 250                 255
   gca gcg att ctt aaa aag ctg gca ggc agg aat gta aaa gtg ttg ttt     816
   Ala Ala Ile Leu Lys Lys Leu Ala Gly Arg Asn Val Lys Val Leu Phe
               260                 265                 270
   gag ccc ggc cgt gcc ctg gtg ggt aac gcc ggt gtg ctg ctg acc aag     864
   Glu Pro Gly Arg Ala Leu Val Gly Asn Ala Gly Val Leu Leu Thr Lys
           275                 280                 285
   gtc gaa tac ctg aaa cct ggc gaa acc aaa aac ttt gcg att gtc gat      912
   Val Glu Tyr Leu Lys Pro Gly Glu Thr Lys Asn Phe Ala Ile Val Asp
       290                 295                 300
   gcc gcc atg aac gac ctc atg cgc ccg gct ttg tat gat gct ttc cac      960
   Ala Ala Met Asn Asp Leu Met Arg Pro Ala Leu Tyr Asp Ala Phe His
   305                 310                 315                 320
   aac att acg acc att gcc act tct gca gcc ccc gca caa atc tat gag     1008
   Asn Ile Thr Thr Ile Ala Thr Ser Ala Ala Pro Ala Gln Ile Tyr Glu
                   325                 330                 335
   atc gtt ggc ccg gtt tgc gag agt ggt gac ttt tta ggc cat gac cgt     1056
   Ile Val Gly Pro Val Cys Glu Ser Gly Asp Phe Leu Gly His Asp Arg
               340                 345                 350
   aca ctt gcg atc gaa gaa ggt gat tac ctg gcg att cac tcc gca ggc     1104
   Thr Leu Ala Ile Glu Glu Gly Asp Tyr Leu Ala Ile His Ser Ala Gly
           355                 360                 365
   gct tat ggc atg agc atg gcc agc aac tac aac acg cgc gcc cgt gcc     1152
   Ala Tyr Gly Met Ser Met Ala Ser Asn Tyr Asn Thr Arg Ala Arg Ala
       370                 375                 380
   gca gag gta ttg gtt gat ggt gac cag gtg cat gtg atc cgt gaa cgt     1200
   Ala Glu Val Leu Val Asp Gly Asp Gln Val His Val Ile Arg Glu Arg
   385                 390                 395                 400
   gaa caa att gcc gac ctg ttt aaa ctg gag cgt acg ctg cca taa         1245
   Glu Gln Ile Ala Asp Leu Phe Lys Leu Glu Arg Thr Leu Pro
                   405                 410
<210> 49
   <211> 414
   <212> PRT
   <213> Methylophilus methylotrophus
<400> 49
   Val Thr Ala Phe Ser Ile Gln Gln Gly Leu Leu His Ala Glu Asn Val
   1               5                   10                  15
   Ala Leu Arg Asp Ile Ala Gln Thr His Gln Thr Pro Thr Tyr Val Tyr
               20                  25                  30
   Ser Arg Ala Ala Leu Thr Thr Ala Phe Glu Arg Phe Gln Ala Gly Leu
           35                  40                  45
   Thr Gly His Asp His Leu Ile Cys Phe Ala Val Lys Ala Asn Pro Ser
       50                  55                  60
   Leu Ala Ile Leu Asn Leu Phe Ala Arg Met Gly Ala Gly Phe Asp Ile
   65                  70                  75                  80
   Val Ser Gly Gly Glu Leu Ala Arg Val Leu Ala Ala Gly Gly Asp Pro
                   85                  90                  95
   Lys Lys Val Val Phe Ser Gly Val Gly Lys Ser His Ala Glu Ile Lys
               100                 105                 110
   Ala Ala Leu Glu Ala Gly Ile Leu Cys Phe Asn Val Glu Ser Val Asn
           115                 120                 125
   Glu Leu Asp Arg Ile Gln Gln Val Ala Ala Ser Leu Gly Lys Lys Ala
       130                 135                 140
   Pro Ile Ser Leu Arg Val Asn Pro Asn Val Asp Ala Lys Thr His Pro
   145                 150                 155                 160
   Tyr Ile Ser Thr Gly Leu Lys Asn Asn Lys Phe Gly Val Ala Phe Glu
                   165                 170                 175
   Asp Ala Leu Gly Leu Tyr Glu Lys Ala Ala Gln Leu Pro Asn Ile Glu
               180                 185                 190
   Val His Gly Val Asp Cys His Ile Gly Ser Gln Ile Thr Glu Leu Ser
           195                 200                 205
   Pro Phe Leu Asp Ala Leu Asp Lys Val Leu Gly Leu Val Asp Ala Leu
       210                 215                 220
   Ala Ala Lys Gly Ile His Ile Gln His Ile Asp Val Gly Gly Gly Val
   225                 230                 235                 240
   Gly Ile Thr Tyr Ser Asp Glu Thr Pro Pro Asp Phe Ala Ala Tyr Thr
                   245                 250                 255
   Ala Ala Ile Leu Lys Lys Leu Ala Gly Arg Asn Val Lys Val Leu Phe
               260                 265                 270
   Glu Pro Gly Arg Ala Leu Val Gly Asn Ala Gly Val Leu Leu Thr Lys
           275                 280                 285
   Val Glu Tyr Leu Lys Pro Gly Glu Thr Lys Asn Phe Ala Ile Val Asp
       290                 295                 300
   Ala Ala Met Asn Asp Leu Met Arg Pro Ala Leu Tyr Asp Ala Phe His
   305                 310                 315                 320
   Asn Ile Thr Thr Ile Ala Thr Ser Ala Ala Pro Ala Gln Ile Tyr Glu
                   325                 330                 335
   Ile Val Gly Pro Val Cys Glu Ser Gly Asp Phe Leu Gly His Asp Arg
               340                 345                 350
   Thr Leu Ala Ile Glu Glu Gly Asp Tyr Leu Ala Ile His Ser Ala Gly
           355                 360                 365
   Ala Tyr Gly Met Ser Met Ala Ser Asn Tyr Asn Thr Arg Ala Arg Ala
       370                 375                 380
   Ala Glu Val Leu Val Asp Gly Asp Gln Val His Val Ile Arg Glu Arg
   385                 390                 395                 400
   Glu Gln Ile Ala Asp Leu Phe Lys Leu Glu Arg Thr Leu Pro
                   405                 410
<210> 50
   <211> 712
   <212> DNA
   <213> Brevibacterium lactofermentum
<220>
   <221> CDS
   <222> (1)..(375)
   <223>
<400> 50
   atg gtg atc atg gaa atc ttc att aca ggt ctg ctt ttg ggg gcc agt       48
   Met Val Ile Met Glu Ile Phe Ile Thr Gly Leu Leu Leu Gly Ala Ser
   1               5                   10                  15
   ctt ttg ctg tcc atc gga ccg cag aat gta ctg gtg att aaa caa gga       96
   Leu Leu Leu Ser Ile Gly Pro Gln Asn Val Leu Val Ile Lys Gln Gly
               20                  25                  30
   att aag cgc gaa gga ctc att gcg gtt ctt ctc gtg tgt tta att tct      144
   Ile Lys Arg Glu Gly Leu Ile Ala Val Leu Leu Val Cys Leu Ile Ser
           35                  40                  45
   gac gtc ttt ttg ttc atc gcc ggc acc ttg ggc gtt gat ctt ttg tcc      192
   Asp Val Phe Leu Phe Ile Ala Gly Thr Leu Gly Val Asp Leu Leu Ser
       50                  55                  60
   aat gcc gcg ccg atc gtg ctc gat att atg cgc tgg ggt ggc atc gct      240
   Asn Ala Ala Pro Ile Val Leu Asp Ile Met Arg Trp Gly Gly Ile Ala
   65                  70                  75                  80
   tac ctg tta tgg ttt gcc gtc atg gca gcg aaa gac gcc atg aca aac      288
   Tyr Leu Leu Trp Phe Ala Val Met Ala Ala Lys Asp Ala Met Thr Asn
                   85                  90                  95
   aag gtg gaa gcg cca cag atc att gaa gaa aca gaa cca acc gtg ccc      336
   Lys Val Glu Ala Pro Gln Ile Ile Glu Glu Thr Glu Pro Thr Val Pro
               100                 105                 110
   gat gac acg cct ttg ggc gtg ttc ggc ggt ggc cac tga cacgcgcaac       385
   Asp Asp Thr Pro Leu Gly Val Phe Gly Gly Gly His
           115                 120
   cgggtgcggg tggaggtgag cgtcgataag cagcgggttt gggtgaagcc catgttgatg    445
   gcaatcgtgc tgacctggtt gaacccgaat gcgtatttgg acgcgtttgt gtttatcggc    505
   ggcgtcggcg cgcaatacgg cgacaccgga cggtggattt tcgccgctgg cgcgttcgcg    565
   gcaagcctga tctggttccc gctggtgggt ttcggcgcag cagcattgtc acgcccgctg    625
   tccagcccca aggtgtggcg ctggatcaac gtcgtcgtgg cagttgtgat gaccgcattg    685
   gccatcaaac tgatgttgat gggttag                                        712
<210> 51
   <211> 124
   <212> PRT
   <213> Brevibacterium lactofermentum
<400> 51
   Met Val Ile Met Glu Ile Phe Ile Thr Gly Leu Leu Leu Gly Ala Ser
   1               5                   10                  15
   Leu Leu Leu Ser Ile Gly Pro Gln Asn Val Leu Val Ile Lys Gln Gly
               20                  25                  30
   Ile Lys Arg Glu Gly Leu Ile Ala Val Leu Leu Val Cys Leu Ile Ser
           35                  40                  45
   Asp Val Phe Leu Phe Ile Ala Gly Thr Leu Gly Val Asp Leu Leu Ser
       50                  55                  60
   Asn Ala Ala Pro Ile Val Leu Asp Ile Met Arg Trp Gly Gly Ile Ala
   65                  70                  75                  80
   Tyr Leu Leu Trp Phe Ala Val Met Ala Ala Lys Asp Ala Met Thr Asn
                   85                  90                  95
   Lys Val Glu Ala Pro Gln Ile Ile Glu Glu Thr Glu Pro Thr Val Pro
               100                 105                 110
   Asp Asp Thr Pro Leu Gly Val Phe Gly Gly Gly His
           115                 120
<210> 52
   <211> 963
   <212> DNA
   <213> Brevibacterium lactofermentum
<220>
   <221> CDS
   <222> (1)..(963)
   <223>
<400> 52
   atg acc aac atc cgc gta gct atc gtg ggc tac gga aac ctg gga cgc      48
   Met Thr Asn Ile Arg Val Ala Ile Val Gly Tyr Gly Asn Leu Gly Arg
   1               5                   10                  15
   agc gtc gaa aag ctt att gcc aag cag ccc gac atg gac ctt gta gga      96
   Ser Val Glu Lys Leu Ile Ala Lys Gln Pro Asp Met Asp Leu Val Gly
               20                  25                  30
   atc ttc tcg cgc cgg gcc acc ctc gac aca aag acg cca gtc ttt gat     144
   Ile Phe Ser Arg Arg Ala Thr Leu Asp Thr Lys Thr Pro Val Phe Asp
           35                  40                  45
   gtc gcc gac gtg gac aag cac gcc gac gac gtg gac gtg ctg ttc ctg     192
   Val Ala Asp Val Asp Lys His Ala Asp Asp Val Asp Val Leu Phe Leu
       50                  55                  60
   tgc atg ggc tcc gcc acc gac atc cct gag cag gca cca aag ttc gcg     240
   Cys Met Gly Ser Ala Thr Asp Ile Pro Glu Gln Ala Pro Lys Phe Ala
   65                  70                  75                  80
   cag ttc gcc tgc acc gta gac acc tac gac aac cac cgc gac atc cca     288
   Gln Phe Ala Cys Thr Val Asp Thr Tyr Asp Asn His Arg Asp Ile Pro
                   85                  90                  95
   cgc cac cgc cag gtc atg aac gaa gcc gcc acc gca gcc ggc aac gtt     336
   Arg His Arg Gln Val Met Asn Glu Ala Ala Thr Ala Ala Gly Asn Val
               100                 105                 110
   gca ctg gtc tct acc ggc tgg gat cca gga atg ttc tcc atc aac cgc     384
   Ala Leu Val Ser Thr Gly Trp Asp Pro Gly Met Phe Ser Ile Asn Arg
           115                 120                 125
   gtc tac gca gcg gca gtc tta gcc gag cac cag cag cac acc ttc tgg     432
   Val Tyr Ala Ala Ala Val Leu Ala Glu His Gln Gln His Thr Phe Trp
       130                 135                 140
   ggc cca ggt ttg tca cag ggc cac tcc gat gct ttg cga cgc atc cct     480
   Gly Pro Gly Leu Ser Gln Gly His Ser Asp Ala Leu Arg Arg Ile Pro
   145                 150                 155                 160
   ggc gtt caa aag gca gtc cag tac acc ctc cca tcc gaa gac gcc ctg     528
   Gly Val Gln Lys Ala Val Gln Tyr Thr Leu Pro Ser Glu Asp Ala Leu
                   165                 170                 175
   gaa aag gcc cgc cgc ggc gaa gcc ggc gac ctt acc gga aag caa acc     576
   Glu Lys Ala Arg Arg Gly Glu Ala Gly Asp Leu Thr Gly Lys Gln Thr
               180                 185                 190
   cac aag cgc caa tgc ttc gtg gtt gcc gac gcg gcc gat cac gag cgc     624
   His Lys Arg Gln Cys Phe Val Val Ala Asp Ala Ala Asp His Glu Arg
           195                 200                 205
   atc gaa aac gac atc cgc acc atg cct gat tac ttc gtt ggc tac gaa     672
   Ile Glu Asn Asp Ile Arg Thr Met Pro Asp Tyr Phe Val Gly Tyr Glu
       210                 215                 220
   gtc gaa gtc aac ttc atc gac gaa gca acc ttc gac tcc gag cac acc     720
   Val Glu Val Asn Phe Ile Asp Glu Ala Thr Phe Asp Ser Glu His Thr
   225                 230                 235                 240
   ggc atg cca cac ggt ggc cac gtg att acc acc ggc gac acc ggt ggc     768
   Gly Met Pro His Gly Gly His Val Ile Thr Thr Gly Asp Thr Gly Gly
                   245                 250                 255
   ttc aac cac acc gtg gaa tac atc ctc aag ctg gac cga aac cca gat     816
   Phe Asn His Thr Val Glu Tyr Ile Leu Lys Leu Asp Arg Asn Pro Asp
               260                 265                 270
   ttc acc gct tcc tca cag atc gct ttc ggt cgc gca gct cac cgc atg     864
   Phe Thr Ala Ser Ser Gln Ile Ala Phe Gly Arg Ala Ala His Arg Met
           275                 280                 285
   aag cag cag ggc caa agc gga gct ttc acc gtc ctc gaa gtt gct cca     912
   Lys Gln Gln Gly Gln Ser Gly Ala Phe Thr Val Leu Glu Val Ala Pro
       290                 295                 300
   tac ctg ctc tcc cca gag aac ttg gac gat ctg atc gca cgc gac gtc     960
   Tyr Leu Leu Ser Pro Glu Asn Leu Asp Asp Leu Ile Ala Arg Asp Val
   305                 310                 315                 320
   taa                                                                 963
<210> 53
   <211> 320
   <212> PRT
   <213> Brevibacterium lactofermentum
<400> 53
   Met Thr Asn Ile Arg Val Ala Ile Val Gly Tyr Gly Asn Leu Gly Arg
   1 5 10 15
   Ser Val Glu Lys Leu Ile Ala Lys Gln Pro Asp Met Asp Leu Val Gly
   20 25 30
   Ile Phe Ser Arg Arg Ala Thr Leu Asp Thr Lys Thr Pro Val Phe Asp
   35 40 45
   Val Ala Asp Val Asp Lys His Ala Asp Asp Val Asp Val Leu Phe Leu
   50 55 60
   Cys Met Gly Ser Ala Thr Asp Ile Pro Glu Gln Ala Pro Lys Phe Ala
   65 70 75 80
   Gln Phe Ala Cys Thr Val Asp Thr Tyr Asp Asn His Arg Asp Ile Pro
   85 90 95
   Arg His Arg Gln Val Met Asn Glu Ala Ala Thr Ala Ala Gly Asn Val
   100 105 110
   Ala Leu Val Ser Thr Gly Trp Asp Pro Gly Met Phe Ser Ile Asn Arg
   115 120 125
   Val Tyr Ala Ala Ala Val Leu Ala Glu His Gln Gln His Thr Phe Trp
   130 135 140
   Gly Pro Gly Leu Ser Gln Gly His Ser Asp Ala Leu Arg Arg Ile Pro
   145 150 155 160
   Gly Val Gln Lys Ala Val Gln Tyr Thr Leu Pro Ser Glu Asp Ala Leu
   165 170 175
   Glu Lys Ala Arg Arg Gly Glu Ala Gly Asp Leu Thr Gly Lys Gln Thr
   180 185 190
   His Lys Arg Gln Cys Phe Val Val Ala Asp Ala Ala Asp His Glu Arg
   195 200 205
   Ile Glu Asn Asp Ile Arg Thr Met Pro Asp Tyr Phe Val Gly Tyr Glu
   210 215 220
   Val Glu Val Asn Phe Ile Asp Glu Ala Thr Phe Asp Ser Glu His Thr
   225 230 235 240
   Gly Met Pro His Gly Gly His Val Ile Thr Thr Gly Asp Thr Gly Gly
   245 250 255
   Phe Asn His Thr Val Glu Tyr Ile Leu Lys Leu Asp Arg Asn Pro Asp
   260 265 270
   Phe Thr Ala Ser Ser Gln Ile Ala Phe Gly Arg Ala Ala His Arg Met
   275 280 285
   Lys Gln Gln Gly Gln Ser Gly Ala Phe Thr Val Leu Glu Val Ala Pro
   290 295 300
   Tyr Leu Leu Ser Pro Glu Asn Leu Asp Asp Leu Ile Ala Arg Asp Val
   305 310 315 320
<210> 54
   <211> 711
   <212> DNA
   <213> Brevibacterium lactofermentum
<220>
   <221> CDS
   <222> (1).. (711)
<400> 54
   atg gtg atc atg gaa atc ttc att aca ggt ctg ctt ttg ggg gcc agt      48
   Met Val Ile Met Glu Ile Phe Ile Thr Gly Leu Leu Leu Gly Ala Ser
   1               5                   10                  15
   ctt tta ctg tcc atc gga ccg cag aat gta ctg gtg att aaa caa gga      96
   Leu Leu Leu Ser Ile Gly Pro Gln Asn Val Leu Val Ile Lys Gln Gly
               20                  25                  30
   att aag cgc gaa gga ctc att gcg gtt ctt ctc gtg tgt tta att tct      144
   Ile Lys Arg Glu Gly Leu Ile Ala Val Leu Leu Val Cys Leu Ile Ser
           35                  40                  45
   gac gtc ttt ttg ttc atc gcc ggc acc ttg ggc gtt gat ctt ttg tcc      192
   Asp Val Phe Leu Phe Ile Ala Gly Thr Leu Gly Val Asp Leu Leu Ser
       50                  55                  60
   aat gcc gcg ccg atc gtg ctc gat att atg cgc tgg ggt ggc atc gct      240
   Asn Ala Ala Pro Ile Val Leu Asp Ile Met Arg Trp Gly Gly Ile Ala
   65                  70                  75                  80
   tac ctg tta tgg ttt gcc gtc atg gca gcg aaa gac gcc atg aca aac      288
   Tyr Leu Leu Trp Phe Ala Val Met Ala Ala Lys Asp Ala Met Thr Asn
                   85                  90                  95
   aag gtg gaa gcg cca cag atc att gaa gaa aca gaa cca acc gtg ccc      336
   Lys Val Glu Ala Pro Gln Ile Ile Glu Glu Thr Glu Pro Thr Val Pro
               100                 105                 110
   gat gac acg cct ttg ggc ggt tcg gcg gtg gcc act gac acg cgc aac      384
   Asp Asp Thr Pro Leu Gly Gly Ser Ala Val Ala Thr Asp Thr Arg Asn
           115                 120                 125
   cgg gtg cgg gtg gag gtg agc gtc gat aag cag cgg gtt tgg gta aag      432
   Arg Val Arg Val Glu Val Ser Val Asp Lys Gln Arg Val Trp Val Lys
       130                 135                 140
   ccc atg ttg atg gca atc gtg ctg acc tgg ttg aac ccg aat gcg tat      480
   Pro Met Leu Met Ala Ile Val Leu Thr Trp Leu Asn Pro Asn Ala Tyr
   145                 150                 155                 160
   ttg gac gcg ttt gtg ttt atc ggc ggc gtc ggc gcg caa tac ggc gac      528
   Leu Asp Ala Phe Val Phe Ile Gly Gly Val Gly Ala Gln Tyr Gly Asp
                   165                 170                 175
   acc gga cgg tgg att ttc gcc gct ggc gcg ttc gcg gca agc ctg atc      576
   Thr Gly Arg Trp Ile Phe Ala Ala Gly Ala Phe Ala Ala Ser Leu Ile
               180                 185                 190
   tgg ttc ccg ctg gtg ggt ttc ggc gca gca gca ttg tca cgc ccg ctg      624
   Trp Phe Pro Leu Val Gly Phe Gly Ala Ala Ala Leu Ser Arg Pro Leu
           195                 200                 205
   tcc agc ccc aag gtg tgg cgc tgg atc aac gtc gtc gtg gca gtt gtg      672
   Ser Ser Pro Lys Val Trp Arg Trp Ile Asn Val Val Val Ala Val Val
       210                 215                 220
   atg acc gca ttg gcc atc aaa ctg atg ttg atg ggt tag                  711
   Met Thr Ala Leu Ala Ile Lys Leu Met Leu Met Gly
   225                 230                 235
<210> 55
   <211> 236
   <212> PRT
   <213> Brevibacterium lactofermentum
<400> 55
   Met Val Ile Met Glu Ile Phe Ile Thr Gly Leu Leu Leu Gly Ala Ser
   1 5 10 15
   Leu Leu Leu Ser Ile Gly Pro Gln Asn Val Leu Val Ile Lys Gln Gly
   20 25 30
   Ile Lys Arg Glu Gly Leu Ile Ala Val Leu Leu Val Cys Leu Ile Ser
   35 40 45
   Asp Val Phe Leu Phe Ile Ala Gly Thr Leu Gly Val Asp Leu Leu Ser
   50 55 60
   Asn Ala Ala Pro Ile Val Leu Asp Ile Met Arg Trp Gly Gly Ile Ala
   65 70 75 80
   Tyr Leu Leu Trp Phe Ala Val Met Ala Ala Lys Asp Ala Met Thr Asn
   85 90 95
   Lys Val Glu Ala Pro Gln Ile Ile Glu Glu Thr Glu Pro Thr Val Pro
   100 105 110
   Asp Asp Thr Pro Leu Gly Gly Ser Ala Val Ala Thr Asp Thr Arg Asn
   115 120 125
   Arg Val Arg Val Glu Val Ser Val Asp Lys Gln Arg Val Trp Val Lys
   130 135 140
   Pro Met Leu Met Ala Ile Val Leu Thr Trp Leu Asn Pro Asn Ala Tyr
   145 150 155 160
   Leu Asp Ala Phe Val Phe Ile Gly Gly Val Gly Ala Gln Tyr Gly Asp
   165 170 175
   Thr Gly Arg Trp Ile Phe Ala Ala Gly Ala Phe Ala Ala Ser Leu Ile
   180 185 190
   Trp Phe Pro Leu Val Gly Phe Gly Ala Ala Ala Leu Ser Arg Pro Leu
   195 200 205
   Ser Ser Pro Lys Val Trp Arg Trp Ile Asn Val Val Val Ala Val Val
   210 215 220
   Met Thr Ala Leu Ala Ile Lys Leu Met Leu Met Gly
   225 230 235
<210> 56
   <211> 761
   <212> DNA
   <213> Brevibacterium lactofermentum
<220>
   <221> CDS
   <222> (1)..(372)
   <223>
<220>
   <221> CDS
   <222> (401).. (712)
   <223>
<400> 56
   atg gtg atc atg gaa atc ttc att aca ggt ctg ctt ttg ggg gcc agt 48
   Met Val Ile Met Glu Ile Phe Ile Thr Gly Leu Leu Leu Gly Ala Ser
   1               5                   10                  15
   ctt ttg ctg tcc atc gga ccg cag aat gta ctg gtg att aaa caa gga      96
   Leu Leu Leu Ser Ile Gly Pro Gln Asn Val Leu Val Ile Lys Gln Gly
               20                  25                  30
   att aag cgc gaa gga ctc att gcg gtt ctt ctc gtg tgt tta att tct      144
   Ile Lys Arg Glu Gly Leu Ile Ala Val Leu Leu Val Cys Leu Ile Ser
           35                  40                  45
   gac gtc ttt ttg ttc atc gcc ggc acc ttg ggc gtt gat ctt ttg tcc      192
   Asp Val Phe Leu Phe Ile Ala Gly Thr Leu Gly Val Asp Leu Leu Ser
       50                  55                  60
   aat gcc gcg ccg atc gtg ctc gat att atg cgc tgg ggt ggc atc gct      240
   Asn Ala Ala Pro Ile Val Leu Asp Ile Met Arg Trp Gly Gly Ile Ala
   65                  70                  75                  80
   tac ctg tta tgg ttt gcc gtc atg gca gcg aaa gac gcc atg aca aac      288
   Tyr Leu Leu Trp Phe Ala Val Met Ala Ala Lys Asp Ala Met Thr Asn
                   85                  90                  95
   aag gtg gaa gcg cca cag atc att gaa gaa aca gaa cca acc gtg ccc      336
   Lys Val Glu Ala Pro Gln Ile Ile Glu Glu Thr Glu Pro Thr Val Pro
               100                 105                 110
   gat gac acg cct ttg ggc gtg ttc ggc ggt ggc cac tgactagcta           382
   Asp Asp Thr Pro Leu Gly Val Phe Gly Gly Gly His
           115                 120
   aaccgggtgc gggtggag gtg agc gtc gat aag cag cgg gtt tgg gtg aag      433
                       Val Ser Val Asp Lys Gln Arg Val Trp Val Lys
                       125                 130                 135
   ccc atg ttg atg gca atc gtg ctg acc tgg ttg aac ccg aat gcg tat      481
   Pro Met Leu Met Ala Ile Val Leu Thr Trp Leu Asn Pro Asn Ala Tyr
                   140                 145                 150
   ttg gac gcg ttt gtg ttt atc ggc ggc gtc ggc gcg caa tac ggc gac      529
   Leu Asp Ala Phe Val Phe Ile Gly Gly Val Gly Ala Gln Tyr Gly Asp
               155                 160                 165
   acc gga cgg tgg att ttc gcc gct ggc gcg ttc gcg gca agc ctg atc      577
   Thr Gly Arg Trp Ile Phe Ala Ala Gly Ala Phe Ala Ala Ser Leu Ile
           170                 175                 180
   tgg ttc ccg ctg gtg ggt ttc ggc gca gca gca ttg tca cgc ccg ctg      625
   Trp Phe Pro Leu Val Gly Phe Gly Ala Ala Ala Leu Ser Arg Pro Leu
       185                 190                 195
   tcc agc ccc aag gtg tgg cgc tgg atc aac gtc gtc gtg gca gtt gtg      673
   Ser Ser Pro Lys Val Trp Arg Trp Ile Asn Val Val Val Ala Val Val
   200                 205                 210                 215
   atg acc gca ttg gcc atc aaa ctg atg ttg atg ggt tag ttttcgcggg       722
   Met Thr Ala Leu Ala Ile Lys Leu Met Leu Met Gly
                   220                 225
   ttttggagct cttctagcag aagagcatac atctggaag                           761
<210> 57
   <211> 124
   <212> PRT
   <213> Brevibacterium lactofermentum
<400> 57
   Met Val Ile Met Glu Ile Phe Ile Thr Gly Leu Leu Leu Gly Ala Ser
   1               5                   10                  15
   Leu Leu Leu Ser Ile Gly Pro Gln Asn Val Leu Val Ile Lys Gln Gly
               20                  25                  30
   Ile Lys Arg Glu Gly Leu Ile Ala Val Leu Leu Val Cys Leu Ile Ser
           35                  40                  45
   Asp Val Phe Leu Phe Ile Ala Gly Thr Leu Gly Val Asp Leu Leu Ser
       50                  55                  60
   Asn Ala Ala Pro Ile Val Leu Asp Ile Met Arg Trp Gly Gly Ile Ala
   65                  70                  75                  80
   Tyr Leu Leu Trp Phe Ala Val Met Ala Ala Lys Asp Ala Met Thr Asn
                   85                  90                  95
   Lys Val Glu Ala Pro Gln Ile Ile Glu Glu Thr Glu Pro Thr Val Pro
               100                 105                 110
   Asp Asp Thr Pro Leu Gly Val Phe Gly Gly Gly His
           115                 120
<210> 58
   <211> 828
   <212> DNA
   <213> Methylophilus methylotrophus
<220>
   <221> CDS
   <222> (1).. (825)
<400> 58
   gtg aat aat aat atc tct tac agt ttt gaa ttc ttc ccg ccc aag acg      48
   Val Asn Asn Asn Ile Ser Tyr Ser Phe Glu Phe Phe Pro Pro Lys Thr
   1               5                   10                  15
   gta gaa ggc atg gcc aat ctg cgc aat gtg cgc aat gag ctg gcg gca      96
   Val Glu Gly Met Ala Asn Leu Arg Asn Val Arg Asn Glu Leu Ala Ala
               20                  25                  30
   ttt tca ccc gaa ttt ttc tcg gtc act ttt ggc gca ggt ggc tcc acg      144
   Phe Ser Pro Glu Phe Phe Ser Val Thr Phe Gly Ala Gly Gly Ser Thr
           35                  40                  45
   cgt gac cgt acc atg gaa agc gtg ctg gaa atc cag gcg gaa ggc cat      192
   Arg Asp Arg Thr Met Glu Ser Val Leu Glu Ile Gln Ala Glu Gly His
       50                  55                  60
   ggg gca gca cct cat att tcc tgt att tcc tct agt aaa gaa gaa att      240
   Gly Ala Ala Pro His Ile Ser Cys Ile Ser Ser Ser Lys Glu Glu Ile
   65                  70                  75                  80
   cgt gag tta tta cag gct tat caa gcc aaa ggc atc aag cga ctg gtc      288
   Arg Glu Leu Leu Gln Ala Tyr Gln Ala Lys Gly Ile Lys Arg Leu Val
                   85                  90                  95
   acc ttg cgc ggc gat atc cct tca ggc gaa gtg agt gct ggc gat ttt      336
   Thr Leu Arg Gly Asp Ile Pro Ser Gly Glu Val Ser Ala Gly Asp Phe
               100                 105                 110
   aaa tat gcc aat gag ctg gtg agt ttt atc cgt gct gaa acc ggt gac      384
   Lys Tyr Ala Asn Glu Leu Val Ser Phe Ile Arg Ala Glu Thr Gly Asp
           115                 120                 125
   tgg ttt cac ctc gaa gtg gcg gct tac cct gag ttt cat ccg gaa gca      432
   Trp Phe His Leu Glu Val Ala Ala Tyr Pro Glu Phe His Pro Glu Ala
       130                 135                 140
   ggt tct gca caa aaa gac ctg gaa aac ttc aaa cgt aaa atc gat gcc      480
   Gly Ser Ala Gln Lys Asp Leu Glu Asn Phe Lys Arg Lys Ile Asp Ala
   145                 150                 155                 160
   ggt gcc gat tct gcc att acg cag tac ttt tac aat atg gat gcg tat      528
   Gly Ala Asp Ser Ala Ile Thr Gln Tyr Phe Tyr Asn Met Asp Ala Tyr
                   165                 170                 175
   ttc cgt ttt gtg gaa gcg gcg caa aaa atg ggt gtt aca gcg cct atc      576
   Phe Arg Phe Val Glu Ala Ala Gln Lys Met Gly Val Thr Ala Pro Ile
               180                 185                 190
   att ccc ggc atc atg ccg atc tac aat tac acg cag ctg gcg cgt ttt      624
   Ile Pro Gly Ile Met Pro Ile Tyr Asn Tyr Thr Gln Leu Ala Arg Phe
           195                 200                 205
   tcc aat gta tgt ggt gca gag att cca cgc tgg ttg cgt tta cgt ctg      672
   Ser Asn Val Cys Gly Ala Glu Ile Pro Arg Trp Leu Arg Leu Arg Leu
       210                 215                 220
   gaa gct tat ggt gat gac ttg gct tca tta cgt gct ttt ggc gtg gat      720
   Glu Ala Tyr Gly Asp Asp Leu Ala Ser Leu Arg Ala Phe Gly Val Asp
   225                 230                 235                 240
   gta gtc acc gat att tgc gcc aag ctg att gcg tct ggc gtg gat aaa      768
   Val Val Thr Asp Ile Cys Ala Lys Leu Ile Ala Ser Gly Val Asp Lys
                   245                 250                 255
   atg cat ttc tat acg ctg aac cag gct ggc att att ggc cag att atc      816
   Met His Phe Tyr Thr Leu Asn Gln Ala Gly Ile Ile Gly Gln Ile Ile
               260                 265                 270
   cgg caa ctg taa                                                      828
   Arg Gln Leu
           275
<210> 59
   <211> 275
   <212> PRT
   <213> Methylophilus methylotrophus
<400> 59
   Val Asn Asn Asn Ile Ser Tyr Ser Phe Glu Phe Phe Pro Pro Lys Thr
   1               5                   10                  15
   Val Glu Gly Met Ala Asn Leu Arg Asn Val Arg Asn Glu Leu Ala Ala
               20                  25                  30
   Phe Ser Pro Glu Phe Phe Ser Val Thr Phe Gly Ala Gly Gly Ser Thr
           35                  40                  45
   Arg Asp Arg Thr Met Glu Ser Val Leu Glu Ile Gln Ala Glu Gly His
       50                  55                  60
   Gly Ala Ala Pro His Ile Ser Cys Ile Ser Ser Ser Lys Glu Glu Ile
   65                  70                  75                  80
   Arg Glu Leu Leu Gln Ala Tyr Gln Ala Lys Gly Ile Lys Arg Leu Val
                   85                  90                  95
   Thr Leu Arg Gly Asp Ile Pro Ser Gly Glu Val Ser Ala Gly Asp Phe
               100                 105                 110
   Lys Tyr Ala Asn Glu Leu Val Ser Phe Ile Arg Ala Glu Thr Gly Asp
           115                 120                 125
   Trp Phe His Leu Glu Val Ala Ala Tyr Pro Glu Phe His Pro Glu Ala
       130                 135                 140
   Gly Ser Ala Gln Lys Asp Leu Glu Asn Phe Lys Arg Lys Ile Asp Ala
   145                 150                 155                 160
   Gly Ala Asp Ser Ala Ile Thr Gln Tyr Phe Tyr Asn Met Asp Ala Tyr
                   165                 170                 175
   Phe Arg Phe Val Glu Ala Ala Gln Lys Met Gly Val Thr Ala Pro Ile
               180                 185                 190
   Ile Pro Gly Ile Met Pro Ile Tyr Asn Tyr Thr Gln Leu Ala Arg Phe
           195                 200                 205
   Ser Asn Val Cys Gly Ala Glu Ile Pro Arg Trp Leu Arg Leu Arg Leu
       210                 215                 220
   Glu Ala Tyr Gly Asp Asp Leu Ala Ser Leu Arg Ala Phe Gly Val Asp
   225                 230                 235                 240
   Val Val Thr Asp Ile Cys Ala Lys Leu Ile Ala Ser Gly Val Asp Lys
                   245                 250                 255
   Met His Phe Tyr Thr Leu Asn Gln Ala Gly Ile Ile Gly Gln Ile Ile
               260                 265                 270
   Arg Gln Leu
           275